# FASCICULE DE BREVET EUROPEEN

(11) **EP 0 628 046 B1**
(45) Date de publication et mention de la délivrance du brevet: **12.05.1999**
(21) Numéro de dépôt: 93905402.9
(22) Date de dépôt: 18.02.1993
(51) Int. Cl.: C07D 487/04, A61K 31/505

(54) **DERIVES DE TRIAZOLOPYRIMIDINES ANTAGONISTES DES RECEPTEURS A L'ANGIOTENSINE II**
TRIAZOLOPYRIMIDINDERIVATE ALS ANGIOTENSIN-II REZEPTOR ANTAGONISTEN
TRIAZOLOPYRIMIDIN DERIVATIVES AS ANTIOTENSIN II RECEPTOR ANTAGONISTS

(30) Priorité: 24.02.1992 FR 9202109; 30.04.1992 FR 9205417
(43) Date de publication de la demande: 14.12.1994
(73) Titulaire: LABORATOIRES UPSA, 47000 Agen (FR)
(72) Inventeur: BRU-MAGNIEZ, Nicole, F-75016 Paris (FR); GÜNGÖR, Timur, F-92500 Rueil-Malmaison (FR); TEULON, Jean-Marie, F-78170 La Celle-Saint-Cloud (FR)
(74) Mandataire: Hubert, Philippe
(86) Numéro de dépôt international: FR9300161
(87) Numéro de publication internationale: WO9317024

(56) Documents cités:
- EP-A- 0 521 768
- WO-A-91/15209

## Description

La présente invention concerne en tant que produits nouveaux, les dérivés de triazolopyrimidines de formule générale (I) ci-dessous ainsi que leurs formes tautomères et éventuellement leurs sels d'addition en particulier les sels d'addition pharmaceutiquement acceptables.

Les composés en question présentent un profil pharmacologique très intéressant dans la mesure où ils sont doués de propriétés antagonistes des récepteurs à l'angiotensine Il et de propriétés antiprolifératives. Ils sont donc particulièrement indiqués pour le traitement et la prévention des maladies cardiovasculaires, en particulier pour le traitement de l'hypertension, pour le traitement de l'insuffisance cardiaque et pour le traitement et la prévention des maladies de la paroi artérielle notamment de l'athérosclérose.

La présente invention concerne également le procédé de préparation des dits produits et leurs utilisations en thérapeutique.

Des composés doués de propriétés antagonistes des récepteurs à l'angiotensine Il ont, par exemple, été décrits dans les documents WO 91/15209 et EP 0.400.974. Il s'agit respectivement de dérivés de pyrimidine ou de pyridopyrimidine pour le premier document, et de dérivés d'hétérocycles fusionnés à un noyau imidazole pour le second. EP 0.521.768, publié le 07.01.93, décrit des dérivés triazolopyrimidine comme antagonistes des récepteurs de l'antagiotensine II.

Les dérivés conformes à la présente invention sont caractérisés en ce qu'ils répondent à la formule générale (I) 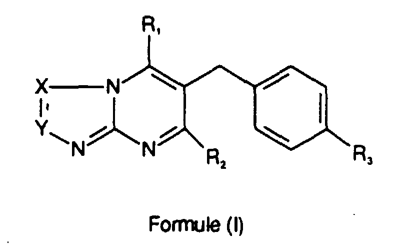 dans la formule (I) :
- l'un des radicaux R₁ et R₂ représente
   - un radical alkyle inférieur de 1 à 6 atomes de carbone ;
   - un radical éther de formule -(CH₂)ₚ OR dans laquelle p est un nombre entier de 1 à 6 et R représente un radical alkyle inférieur de 1 atomes de carbone ou un radical benzyle ;
   - un radical alcool de formule -(CH2)p OH dans laquelle p est tel que défini précédemment ;
   - l'autre des radicaux R₁ et R₂ représente
      - l'atome d'hydrogène,
      - un atome d'halogène,
      - un radical alkyle inférieur de 1 à 6 atomes de carbone,
      - un radical choisi dans le groupe comprenant les radicaux N₃, OR₄, SR₄,NR₅R₆, NH(CH₂)ₙ-NR₅R₆;
      dans lesquels :
      R₄ représente :
         - l'atome d'hydrogène,
         - un radical alkyle inférieur de 1 à 6 atomes de carbone ou un radical cycloalkyle en C₃-C₇,
         - un radical (CH₂)ₘ-COOR', m étant un nombre entier de 1 à 4, R' l'atome d'hydrogène ou un radical alkyle inférieur de 1 à 6 atomes de carbone,
         - un radical (CH₂)ₘ-O-R', m et R'étant défini comme ci-dessus ;
      R₅ et R₆, identiques ou différents, représentent :
         - l'atome d'hydrogène,
         - un radical alkyle inférieur de 1 à 6 atomes de carbone ou un radical cycloalkyle en C₃-C₇,
            ou
      R₅ et R₆, pris ensemble avec l'atome d'azote auquel ils sont rattachés, forment un hétérocycle choisi parmi la morpholine, la pyrrolidine ou la pipéridine ;
         - n est un nombre entier de 1 à 4,
- X et Y différents représentent :
   - l'un, l'atome d'azote,
   - l'autre un groupement C-R₇ dans lequel R₇ représente
      - l'atome d'hydrogène,
      - un radical alkyle inférieur de 1 à 6 atomes de carbone ou un radical cycloalkyle en C₃-C₇,
      - un radical (CH₂)_{n'} OH dans lequel n' est un nombre entier de 0 à 4,
      - un radical SR',R' étant tel que défini précédemment,
      - un radical NR₅R₆ dans lequel R₅ et R₆ identiques ou différents représentent l'atome d'hydrogène ou un radical alkyle inférieur de 1 à 6 atomes de carbone ou un radical cycloalkyle en C₃-C₇ ;
- R₃ représente un radical de formule : 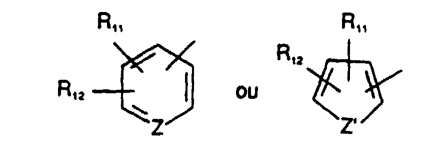 dans lesquelles
   - Z représente CH, N ou Z' représente S, O,
   - R₁₁ représente l'atome d'hydrogène ou un atome d'halogène,
   - R₁₂ représente un radical tétrazole, CN, COOH, CONH₂.

Les dérivés précités doivent être considérés également sous leur forme tautomère.

Dans le cas où R₂ représente un groupement azide, les composés pourront être considérés sous la forme tricyclique tétrazolo [1,5-c]-1,2,4-triazolo [1,5-a] pyrimidine selon l'équilibre bien connu de la littérature (cf. Temple and Montgomery J. Org. Chem. 30,826 (1965)).

Les dérivés précités peuvent se présenter sous la forme de sels d'addition en particulier des sels d'addition pharmaceutiquement acceptables.

Avantageusement, les dérivés de l'invention répondent à la formule générale (I) précitée, dans laquelle :
- l'un des radicaux R₁ et R₂ représente:
   - un radical alkyle inférieur de 1 à 6 atomes de carbone ;
   - un radical éther de formule -(CH₂)ₚ OR dans laquelle p est un nombre entier de 1 à 6 et R représente un radical alkyle inférieur de 1 à 6 atomes de carbone ou un radical benzyle ;
   - un radical alcool de formule ((CH₂)ₚ OH dans laquelle p est tel que défini précédemment ;
   - l'autre des radicaux R₁ et R₂ représente :
      - l'atome d'hydrogène,
      - un atome d'halogène,
      - un radical alkyle inférieur de 1 à 6 atomes de carbone,
      - un radical choisi dans le groupe comprenant les radicaux N₃, OR₄, SR₄, NR₅R₆, NH(CH₂)ₙ-NR₅R₆ ;
      dans lesquels :
      R₄ représente :
         - l'atome d'hydrogène,
         - un radical -(CH₂)ₘ-O-R' dans lequel, m est un nombre entier de 1 à 4 et R' représente un radical alkyle inférieur de 1 à 6 atomes de carbone,
      R₅ et R₆, identiques ou différents, représentent :
         - l'atome d'hydrogène,
         - un radical alkyle inférieur de 1 à 6 atomes de carbone, ou
      R₅ et R₆, pris ensemble avec l'atome d'azote auquel ils sont rattachés, forment un hétérocycle choisi parmi la morpholine, la pyrrolidine ou la pipéridine ;
         - n est un nombre entier de 1 à 4
- X et Y différents représentent :
   - l'un, l'atome d'azote,
   - l'autre un groupement C-R₇ dans lequel R₇ représente
      - l'atome d'hydrogène,
      - un radical alkyle inférieur de 1 à 6 atomes de carbone,
      - un radical (CH₂)_{n'} OH dans lequel n' est un nombre entier de 0 à 4,
      - un radical SR',R' étant tel que défini précédemment,
      - un radical NR₅R₆ dans lequel R₅ et R₆ identiques ou différents représentent l'atome d'hydrogène ou un radical alkyle inférieur de 1 à 6 atomes de carbone ;
- R₃ représente l'un des radicaux suivants :

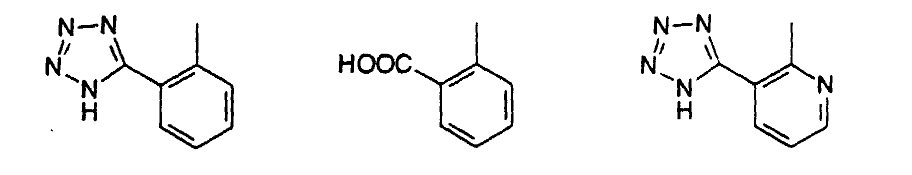

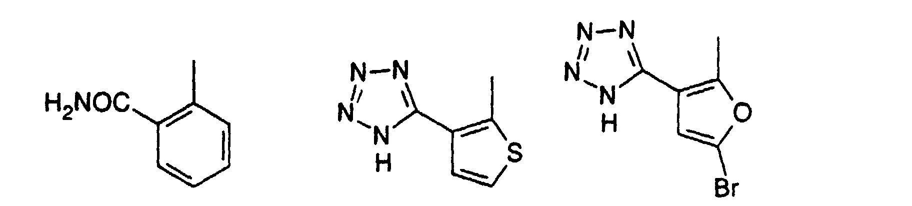

Dans la description et les revendications, on entend par radical alkyle inférieur une chaîne hydrocarbonée ayant de 1 à 6 atomes de carbone, linéaire ou ramifiée. Un radical alkyle inférieur est par exemple un radical méthyle, éthyle, propyle, isopropyle, butyle, isobutyle, tertiobutyle, pentyle, isopentyle, hexyle, isohexyle.

Par radical cycloalkyle en C₃-C₇, on entend un radical hydrocarboné cyclique saturé, il s'agit de préférence d'un radical cyclopropyle, cyclobutyle, cyclohexyle ou cycloheptyle.

On entend par halogène un atome de chlore, de brome, d'iode ou de fluor.
Selon une variante de réalisation, R₁ est un groupement n-propyl,
selon une autre variante de réalisation, R₁ est un groupement N-diéthylamino,
selon une autre variante de réalisation, R₁ est un groupement n-butyl,
selon une variante de réalisation, R₂ est un groupement hydroxy,
selon une autre variante de réalisation, R₂ est un groupement n-propyl,
selon une autre variante de réalisation, R₂ est un groupement N-diéthylamino,
selon une variante de réalisation, R₃ est un groupement 2-(1-H tétrazol-5-yl) phényl,
selon une variante de réalisation X est l'atome d'azote,
selon une variante de réalisation, Y représente le groupement CH,
selon une autre variante de réalisation, Y est le groupement C-CH₃,
selon une autre variante de réalisation, Y est le groupement C-NH₂,

Les composés de l'invention particulièrement préférés sont ceux qui sont choisis parmi les produits de formule :

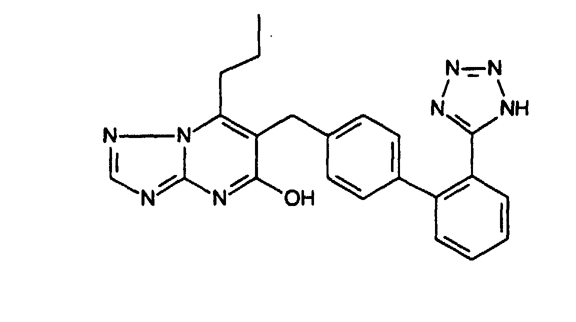

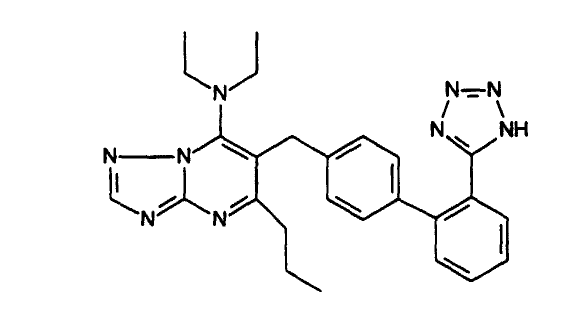

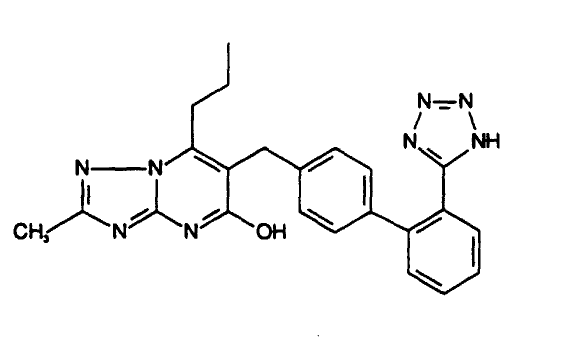

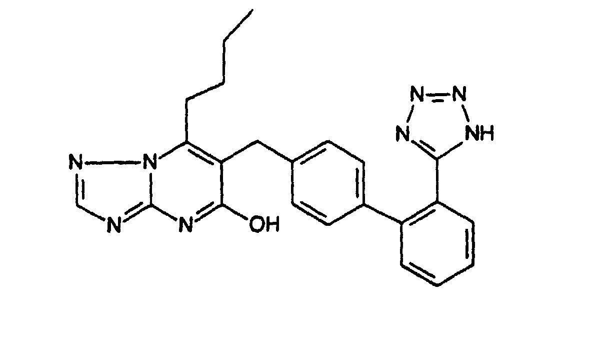

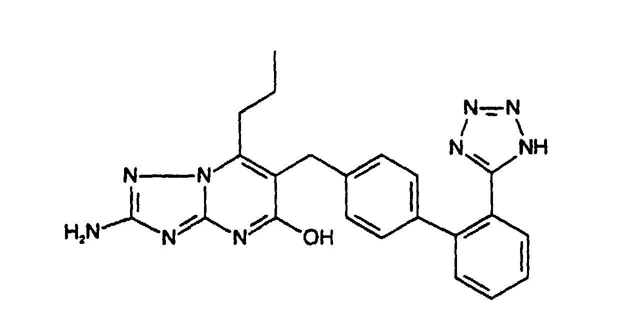

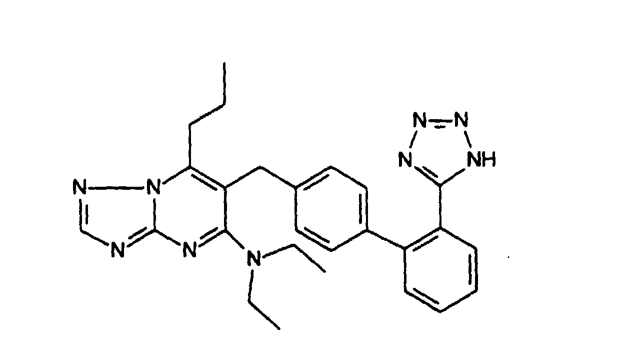

D'une façon générale, le procédé de préparation des composés de formule (I) est caractérisé en ce qu'il comprend :
a) la préparation d'un composé de formule (α) 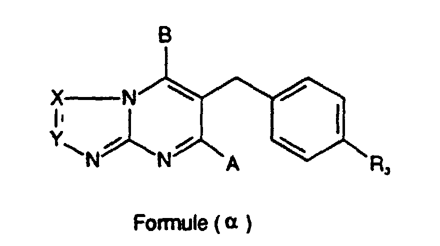 dans laquelle :
   - X, Y et R₃ sont tels que définis ci-dessus,
   - A et B représentent l'un un groupe hydroxy ou un radical alkyle inférieur de 1 à 6 atomes de carbone, l'autre un radical alkyle inférieur de 1 à 6 atomes de carbone ou un radical éther de formule -(CH₂)ₚ-OR dans laquelle p est un nombre entier de 1 à 6 et R représente un radical alkyle inférieur de 1 à 6 atomes de carbone ou un radical benzyle, par condensation d'un 3-amino-1,2,4-triazole de formule (Il) 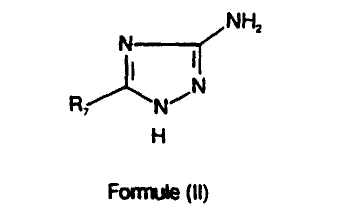 dans laquelle R₇ est défini comme ci-dessus sur un dérivé de formule (β) 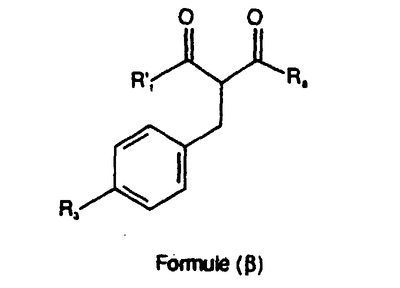 dans laquelle R'₁ est un radical alkyle inférieur de 1 à 6 atomes de carbone ou un radical éther de formule -(CH₂)ₚ-OR dans laquelle p est un nombre entier de 1 à 6 et R représente un radical alkyle inférieur de 1 à 6 atomes de carbone ou un radical benzyle, R₈ représente un radical alkyle inférieur de 1 à 6 atomes de carbone ou un groupement O-alkyle inférieur de 1 à 6 atomes de carbone de préférence méthyle ou éthyle et R₃ est tel que défini ci-dessus,
      dans un solvant aprotique comme le dichloro ou le trichlorobenzène ou dans un solvant acide comme l'acide acétique ou encore dans un alcool en présence de l'alcoolate de sodium ou de potassium correspondant ou encore dans la pyridine ou la 2-méthyl -5-éthyl pyridine en présence ou non de 4-diméthylamino pyridine à une température comprise entre 50 et 200°C ;
b) éventuellement, la protection de la fonction portée par R₃ selon une méthode connue en soi ;
c) le chauffage du dérivé ainsi obtenu à partir du dérivé de la formule (β) lorsque celui-ci est un céto ester dans un réactif approprié comme par exemple POCl₃, pour transformer le groupe hydroxy représenté par A ou B en un atome de chlore ;
d) le chauffage de ce dérivé chloré en présence d'un nucléophile azoté, oxygéné ou soufré dans un alcool au reflux ou à l'autoclave à 100°C, en présence ou non d'une base comme par exemple Na₂CO₃ pour obtenir un dérivé de formule (α) dans laquelle A et B ont la même signification que R₁ et R₂ respectivement ;
e) éventuellement, la déprotection de la fonction portée par R₃;
   e₁) la transformation de cette fonction en une fonction acide par exemple par hydrolyse dans le cas où cette fonction est un nitile ; ou
   e₂) la transformation de cette fonction en une fonction tétrazole, par exemple dans le cas où cette fonction est un nitrile par action de l'azoture d'un trialkylétain au chauffage du toluène ou du xylène suivie d'un traitement par l'acide chlorhydrique gazeux dans le tétrahydrofurane ; ou
   e₃) la transformation de cette fonction en fonction amide par exemple dans le cas où cette fonction est un nitrile par action de l'acide sulfurique ou par action de l'eau oxygénée ou encore par action de l'acide polyphosphorique ; et
f) éventuellement, la transformation du dérivé ainsi obtenu en un sel d'addition, de préférence pharmaceutiquement acceptable.

Selon l'invention, les composés de formule (I) pourront être synthétisés selon la suite de réactions suivantes :

On préparera les composés de formule (III): 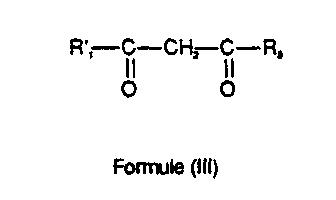 dans laquelle R'₁ est un radical alkyle inférieur de 1 à 6 atomes de carbone ou un radical éther de formule -(CH₂)ₚ-OR dans laquelle p est un nombre entier de 1 à 6 et R représente un radical alkyle inférieur de 1 à 6 atomes de carbone ou un radical benzyle et R₈ représente un radical alkyle inférieur de 1 à 6 atomes de carbone ou un groupement O-alkyle inférieur de 1 à 6 atomes de carbone, de préférence méthyle ou éthyle, par des méthodes connues en soi, telles que par exemple la réaction de Claisen ou la méthode à l'acide de Meldrum, ces méthodes pouvant être facilement retrouvées dans la littérature aux références :
- HAUSER C.R. ; SWAMER F.W. ; ADAMS J.T.; Org. Reaction, Vol. VIII 1954, 59-196.
- HENNE A.L. ; TEDDER J.M. ; J. Chem. Soc. 1953, 3628.
- BRESLOW D.S. ; BAUMGARTEN E. ; HAUSER C.R. ; J. Am. Chem. Soc. 1944, 66, 1286.
- OIKAWA.Y ; SUGANO.K ; YONEMITSU.O ; J.Org.Chem., 1978, 43(10), 2087-88.
- WIERENGA.W ; SKULNICK.H.I.; J.Org.Chem., 1979, 44, 310 .
- HOUGHTON.R ; LAPHAM.D ; SYNTHESIS, 1982, 6, 451-2 .
- BRAM.G ; VILKAS.M ; Bull.Soc.Chim.France, 1964(5), 945-51.
- BALYAKINA M.V. ; ZHDANOVICH E.S. ; PREOBRAZHENSKII N.A. ; Tr. Vses. Nauchn. Issled. Vitam in. Inst., 1961, 7, 8-16.
- RENARD M. ; MAQUINAY A. ; Bull. Soc. Chim. Belg., 1946, 55, 98-105.
- BRUCE F.W.; COOVER H.W.; J. Am. Chem. Soc., 1944, 66, 2092-94.
- EBY C.J. et HAUSER C.R. ; J. Am. Chem. Soc. 1957, 79, 723-5

Par benzylation des composés de formule (III),
par des composés de formule (IV) 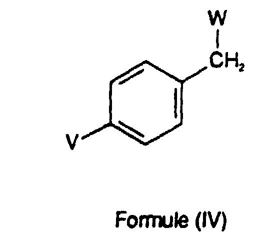 en présence d'une base telle qu'un carbonate de sodium ou de potassium dans l'acétone, un alcoolate de sodium ou de potassium dans un alcool, un hydrure de sodium ou de lithium dans des solvants comme le tétrahydrofurane, le dioxane ou le diméthyl formamide par exemple, à une température comprise entre 50 et 100°C ou encore, en présence d'un équivalent de chlorure ou de bromure de lithium et de deux équivalents de diisopropyl éthyl amine au reflux du tétrahydrofurane selon la référence :
SUNG-EUN YOO ; KYU YANG YI ; Bull. Korean. Chem. Soc. 1989, 10 (1), 112;
on obtiendra les composés de formule :

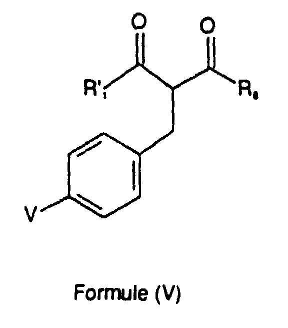

Ces composés de formule (V) peuvent être également obtenus par condensation d'un aldéhyde de formule (VI) 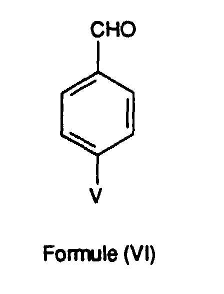 sur les composés de formule (III) suivie d'une hydrogénation en présence d'un catalyseur comme le Nickel de Raney, le palladium sur charbon ou l'oxyde de platine dans un solvant comme un alcool ou le tétrahydrofurane sous pression ou à pression ordinaire lorsque les substitutions présentes le permettent.

D'une manière plus générale, on trouvera des méthodes de préparation des composés de formule (V) dans les références suivantes :
- DURGESHWARI.P ; CHAUDHURY.N.D ; J.lnd.Chem.Soc, 1962, 39, 735-6
- HEINZ.P ; KREGLEWSKI.A ; J.Prakt.Chem, 1963, 21 (3-4), 186-197
- ZAUGG.H.E ; DUNNIGAN.D.A ; MICHAELS.R.J ; SWETT.LR ; J.Org.Chem., 1961, 26, 644-51.
- KAGAN.H.B ; HENG SUEN.Y; Bull.Soc.Chim.France, 1966 (6), 1819-22.
- RATHKE.M.W. ; DEITCH.J ; Tetrahedron Lett, 1971 (31), 2953-6.
- BORRIES KUBEL ; Liebigs.Ann.Chem, 1980, 1392-1401,.
- MARQUET. J ; MORENO-MANAS. M ; Chem. Lett, 1981, 2, 173-6.
- IOFFE .T ; POPOV.E.M ; VATSURO.K.V. ; TULIKOVA.E.K. ; KABACHNIK.M.I. ; Tetrahedron, 1962, 18, 923-940.
- SHEPHERD.T.M ; Chem. Ind. (London), 1970, 17, 567.

Dans la formule (IV), W représente un atome d'halogène préférentiellement le chlore ou le brome.

Dans la même formule :
- V peut être un groupement 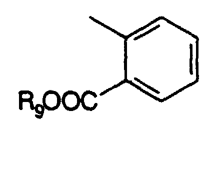 R₉ étant un radical alkyle inférieur ou benzyle ; les composés de formule (IV) sont alors préparés par réaction d'un magnésien du p-bromo toluène sur un composé de formule : 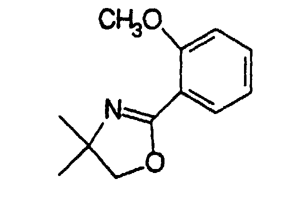 pour obtenir un composé de formule : 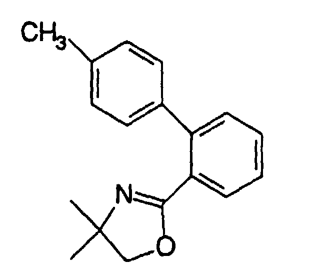 qui est ensuite hydrolysé pour conduire au composé de formule :

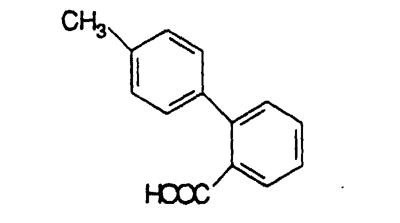

On trouvera des modes opératoires pour les trois étapes décrites ci-dessus dans la référence :
- MEYERS.A.I.; MIHELICH E.D., J.Am.Chem.Soc.,1975, 97, 7383 .

L' acide est ensuite estérifié par un alcool de formule R₉OH, R₉ étant défini comme ci-dessus.

Ces dérivés sont alors bromés ou chlorés par exemple par le N-bromo succinimide, le N-chloro succinimide ou le brome dans un solvant comme le tétrachlorure de carbone ou le dibromoéthane ou le dichloroéthane pour conduire aux composés de formule (IV) où V est le groupement 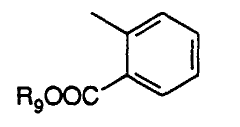
- V peut être le groupement 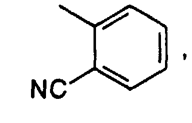 dans ce cas le composé : 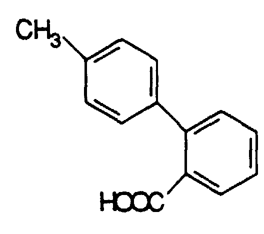 précédemment préparé sera transformé en amide primaire par action du chlorure d'acide, obtenu avec le chlorure de thionyle ou l'oxychlorure de phosphore, sur l'ammoniaque et cet amide sera transformé en nitrile par action de l'oxychlorure de phosphore dans le diméthylformamide ou du chlorure de thionyle. De même, on pourra transformer directement le composé 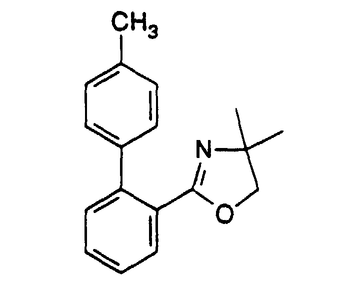 précédemment obtenu en dérivé carbonitrile par traitement dans la pyridine en présence de POCl₃. Le dérivé nitrile obtenu de formule : 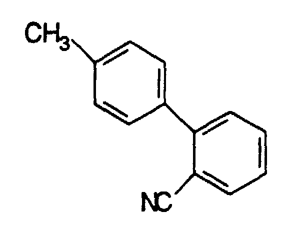 sera ensuite bromé ou chloré dans les mêmes conditions que l'ester précédent pour conduire aux composés de formule (IV) où V est le groupement 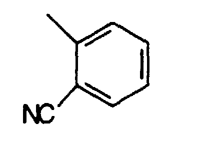
- V peut être un groupement 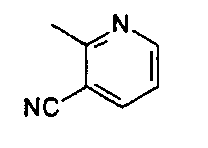 dans ce cas, les composés de formule (IV) correspondants sont synthétisés de la façon suivante :
   le magnésien : 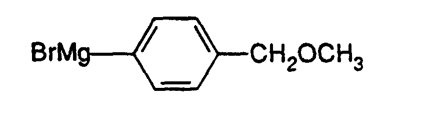 préparé selon une réaction classique de Grignard, est transformé en dérivé zincique par action du chlorure de zinc. Ce dérivé zincique est condensé sur le 2-chloronicotinonitrile en présence de [Ni(PΦ₃)]₂Cl₂ pour conduire au dérivé de formule : 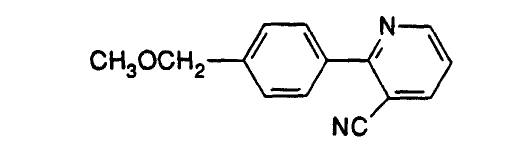 Ce composé traité par le tribromure de bore dans le chloroforme conduira aux composés de formule (IV) dans laquelle W est l'atome de brome et V le
   groupement 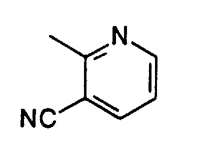
- V peut être un groupement 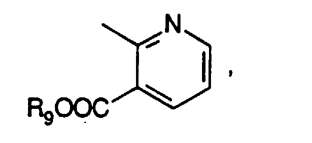 R₉ étant défini comme ci-dessus, dans ce cas les composés de formule (IV) correspondants pourront être préparés à partir du nitrile précédemment préparé de formule 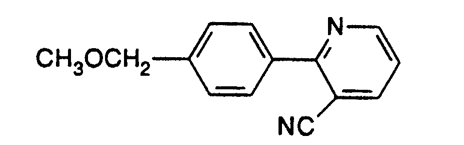 par hydrolyse classique de la fonction nitrile puis estérification de l'acide obtenu ou passage directement de la fonction nitrile à la fonction ester selon les méthodes connues de l'homme de l'art, suivi d'un traitement au tribromure de bore dans le chloroforme pour conduire aux composés de formule (IV) dans lequel W est l'atome de brome et V le groupement 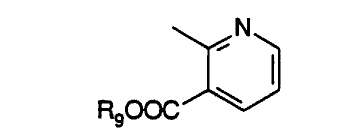
- V peut être un groupement 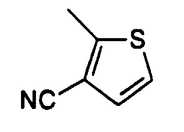 dans ce cas les composés de formule (IV) correspondants sont synthétisés de la façon suivante.

A partir de la chloro-4 méthyl-4' butyrophénone de formule : 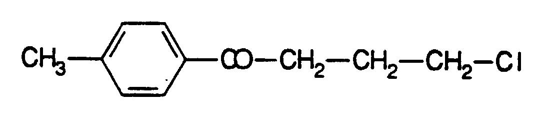 dont la préparation est décrite dans le brevet BE : 577,977 du 15 mai 1959, CA : 54, 4629 c,
on obtiendra par traitement par l'oxychlorure de phosphore et le diméthyl formamide selon les conditions décrites dans la référence :
- VOLODINA. M.A ; TENENT'EV. A.P. ; KUDRYASHOVA. V.A. ; KABOSHINA. L.N. ; Khim. Geterosikl. Soedim ; 1967, 5-8 ;
le composé de formule :

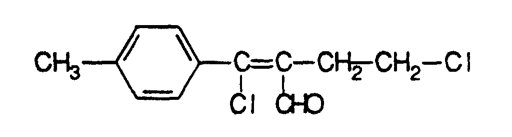

Ce composé est ensuite traité par le sulfure de sodium dans un solvant comme le tétrahydrofurane au reflux pour donner le dérivé 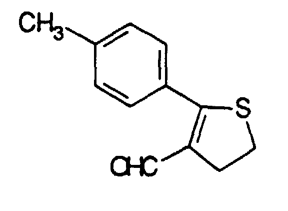 qui est ensuite transformé en deux étapes en dérivé nitrile par déshydratation de l'oxime formée à partir de l'aldéhyde et de l'hydroxylamine. Cette déshydratation pourra être effectuée par exemple à l'aide de l'anhydride acétique pour donner le composé nitrile : 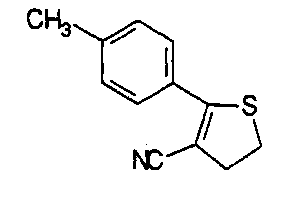 qui pourra être ensuite aromatisé par traitement avec du brome dans le tétrachlorure de carbone pour donner le composé :

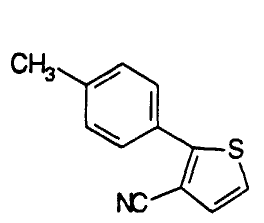

Ce composé peut être ensuite chloré ou bromé par des agents d'halogénation tels que le N-chloro succinimide ou le N-bromo succinimide dans un solvant comme le tétrachlorure de carbone ou le dibromoéthane pour donner les composés de formule (IV) dans laquelle V représente le groupement 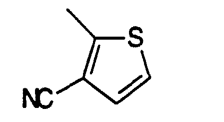
- V peut être un groupement 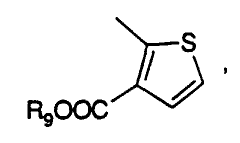 R₉ étant défini comme ci-dessus, dans ce cas les composés de formule (IV) correspondants pourront être préparés à partir du nitrile précédemment préparé de formule : 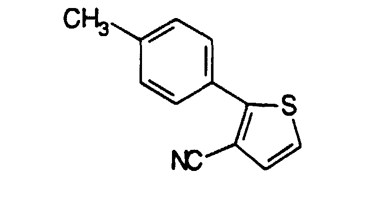 par hydrolyse classique de la fonction nitrile puis estérication de l'acide obtenu ou passage directement de la fonction nitrile à la fonction ester selon les méthodes connues de l'homme de l'art, suivie d'une chloration ou d'une bromation de l'ester par le N-chloro succinimide ou le N-bromo succinimide dans le tétrachlorure de carbone ou le dibromo éthane par exemple pour conduire aux composés de formule (IV) dans laquelle W est l'atome de brome ou l'atome de chlore et V le groupement 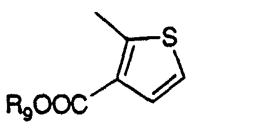
- V peut être un groupement 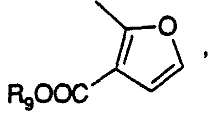 R₉ étant défini comme ci-dessus, dans ce cas les composés de formule (IV) correspondants pourront être préparés à partir du dérivé diazoté de la p-toluidine sur l'acide 3-furoïque pour conduire aux composés de formule 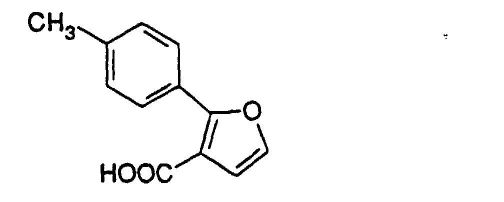 selon la méthode décrite dans la littérature à la référence :
   A. JURASEK et Coll. Collect. Czech. Chem. Commun. 1989, 54, 215.

Ce composé sera ensuite estérifié selon les méthodes classiques connues de l'homme de l'art pour conduire au composé : 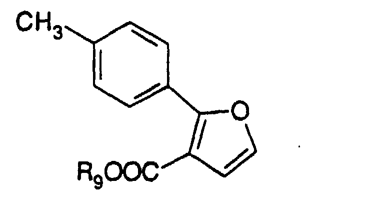 où R₉ est défini comme ci-dessus ;
ce dérivé étant ensuite bromé ou chloré par action du N-bromo succinimide ou du N-chloro succinimide dans le tétrachlorure de carbone ou le 1,2-dichloroéthane par exemple pour conduire au dérivé de formule (IV) dans laquelle W est l'atome de brome ou l'atome de chlore et V le groupement 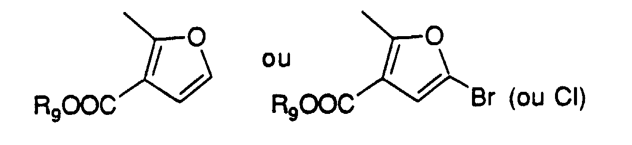
- V peut être un groupement 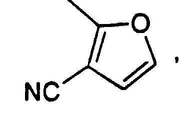 dans ce cas l'acide : 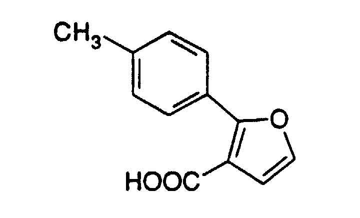 précédemment préparé sera transformé en chlorure d'acide par action du chlorure de thionyle puis en amide par action de l'ammoniac. L'amide obtenu sera transformé en nitrile par action de l'oxychlorure de phosphore dans le diméthyl formamide pour conduire aux composés de formule

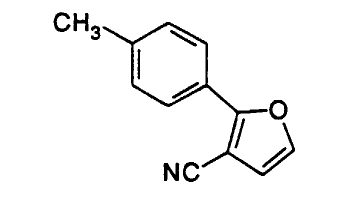

Ce dérivé sera ensuite bromé ou chloré par action du N-bromo succinimide ou du N-chlorosuccinimide dans le tétrachlorure de carbone ou le 1,2-dichloroéthane par exemple pour conduire aux composés de formule (IV) dans laquelle W est l'atome de brome ou l'atome de chlore et V le groupement

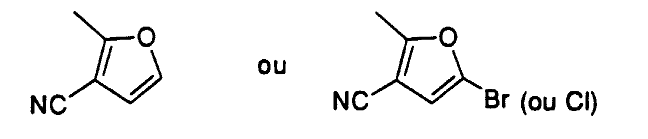

Dans la formule (V), R'₁ et R₈ sont définis comme précédemment et V a la même signification que dans la formule (IV).

Cependant, les composés de formule (V), dans laquelle V est un groupement 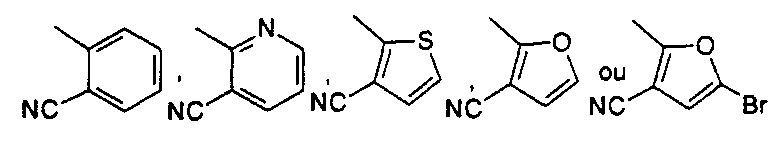 pourront réagir avec un équivalent d'azoture de sodium dans un solvant tel que le diméthyl formamide en présence d'un sel d'ammonium comme le chlorure d'ammonium ou par chauffage dans le toluène ou le xylène avec l'azoture d'un trialkyl étain pour conduire aux composés de formule (V) où V représente le groupement

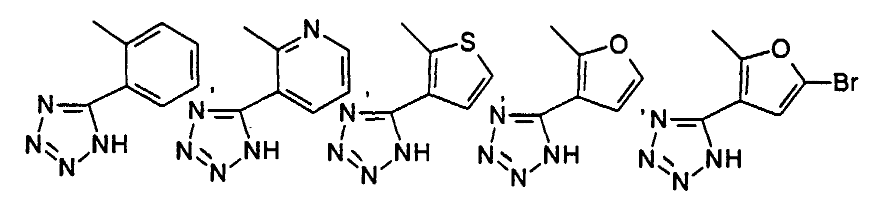

Dans la formule (VI), V a la même définition que dans la formule (IV), mais cette méthode de condensation ne sera utilisée que lorsque V possède une fonction que respecte l'hydrogénation.

Ainsi par action d'un 3-amino-1,2,4-triazole de formule (II) 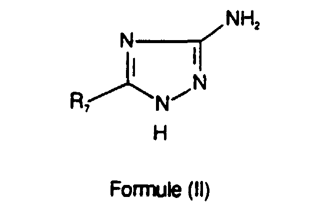 dans laquelle R₇ est défini comme ci-dessus,
(ces produits étant commerciaux ou pouvant être préparés selon les méthodes décrites dans la littérature aux références :
HUFFMANN et SCHAEFER J. of Org. Chem. 1963, 28, p 1812-1816 et p 1816-1821
ALLEN et Coll. J. of Org. Chem. 1959, 24, p 793-796
Organic Synthesis Collective volume 3 p 95).
sur les composés de formule (V) où R'₁ et R₈ sont définis comme ci-dessus et où V représente un des groupements suivants : 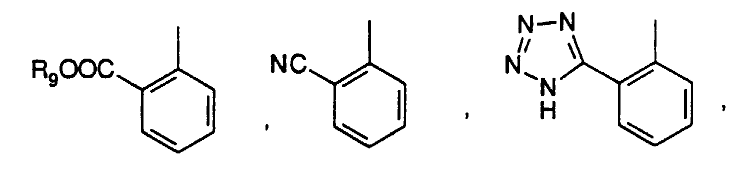 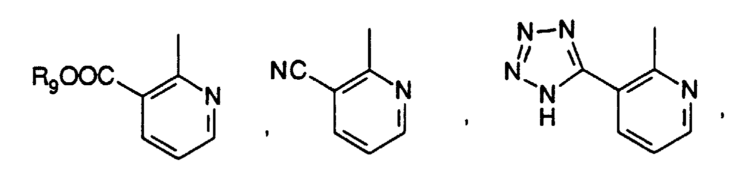 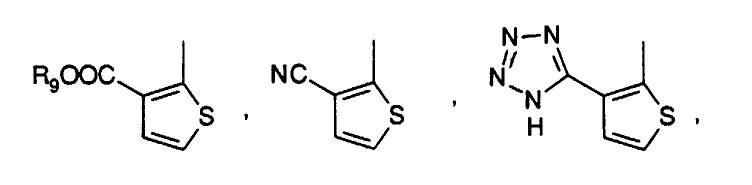 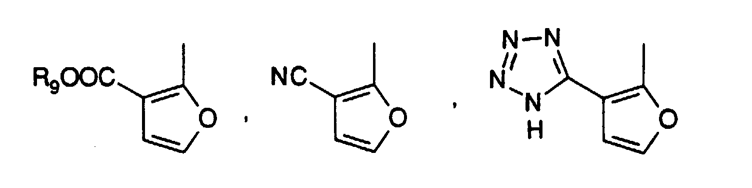 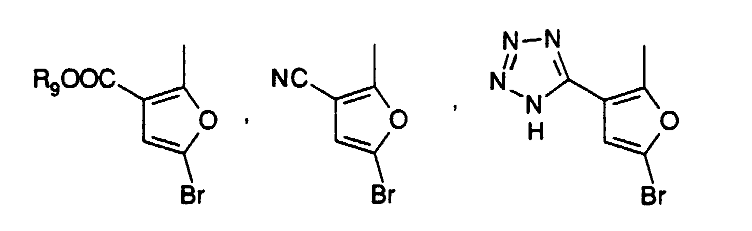 avec R₉ défini comme précédemment, on obtiendra par condensation dans un solvant aprotique comme le dichloro ou le trichlorobenzène ou dans un solvant acide comme l'acide acétique ou encore dans un alcool en présence de l'alcoolate de sodium ou de potassium correspondant ou encore dans la pyridine ou la 2-méthyl-5-éthyl pyridine en présence ou non de 4-diméthyl amino pyridine à une température comprise entre 50° à 200°C, les composés de formule (Vlla) et/ou (Vllb) ainsi que leurs formes tautomères dans lesquelles R'₁, X, Y et V sont définis comme ci-dessus et R₁₀ représente le groupement hydroxy lorsque les composés de formule (V) sont des β céto esters et un radical alkyle inférieur de 1 à 6 atomes de carbone dans le cas où ces mêmes composés de formule (V) sont des β di cétones :

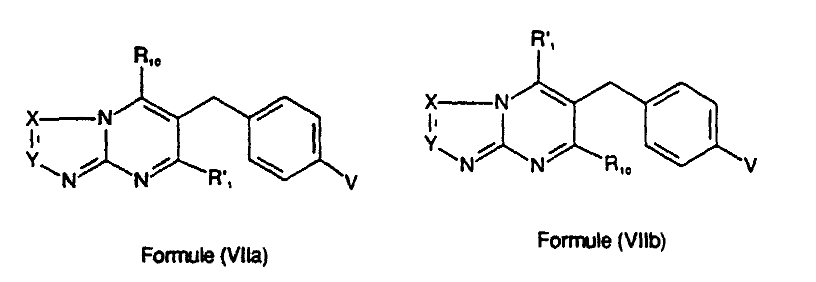

Dans le cas où V possède une fonction tétrazole, les températures de réaction ne devraient pas dépasser 140°C pour ne pas décomposer le tétrazole.

Les réactions d'aminotriazoles ou d'amines hétéroaromatiques similaires, sur les β céto-esters et les dérivés β dicétoniques sont bien décrites dans la littérature et selon les conditions opératoires les formes obtenues sont identifiées. On peut par exemple citer les travaux de
J.A. VAN ALLAN et al. J. Org. Chem. p. 779 à p. 801 (1959) et de
L.A. WILLIAMS J. Chem. Soc. p. 1829 (1960)
L.A. WILLIAMS J. Chem. Soc. p. 3046 (1961)

Ainsi on identifiera les composés Vlla et Vllb pour les traiter séparement.

La demanderesse a toutefois découvert que la 2-méthyl-5-éthyl pyridine en présence ou non de 4-diméthylamino pyridine était un solvant de choix pour orienter la réaction presque exclusivement vers la formation des dérivés de formule (Vllb); en effet ce solvant permet d'obtenir la température (170-180°C) et le pH nécessaires à cette orientation.

Par traitement des dérivés de formule (Vlla) et (Vllb) où R₁₀ est un groupement hydroxy à l'aide d'un réactif comme le P₂S₅ ou le réactif de Lawesson, on obtiendra les dérivés de formule (Vlla) et (Vllb) où le groupement R₁₀ est un thiol.

Par chauffage dans du POCl₃, par exemple, des dérivés de formule (Vlla) et (Vllb) où R₁₀ est un groupement hydroxy, on obtiendra les dérivés de formule (Villa) et (Vlllb) 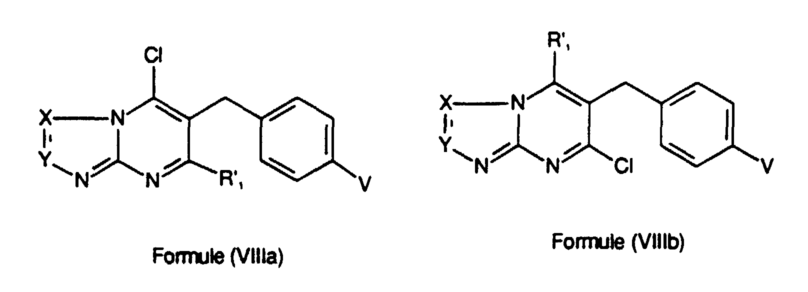 dans lesquelles R'₁, X, Y et V sont définis comme ci-dessus.

L'hydrogénation des dérivés de formule (Vllla) et (Vlllb) en présence d'un catalyseur comme le palladium sur charbon permettra le remplacement du chlore par un atome d'hydrogène et le chauffage des dérivés de formule (Villa) et (Vlllb) en présence d'un nucléophile azoté, oxygéné ou soufré dans un alcool au reflux en présence ou non d'une base comme le Na₂CO₃ ou à l'autoclave à 100°C permettra d'obtenir les dérivés de formule (IX). 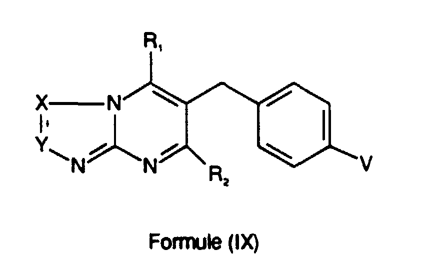 dans laquelle R₁, R₂, X, Y et V sont définis comme précédemment.

Les dérivés de formule (Vlla) et (Vllb) dans lesquels R'₁ représente le groupement (CH₂)ₚO-benzyl, p étant tel que défini précédemment, pourront être hydrogénés en présence de palladium sur charbon dans l'acide acétique pour conduire aux composés de formule (Vlla) et (Vllb) dans lesquels R'₁ représente un groupement alcool.

Une autre voie d'accès à ces triazolo pyrimidines de formule (Vlla) et (Vllb), pourra être réalisée par réaction des dérivés de formule (X) 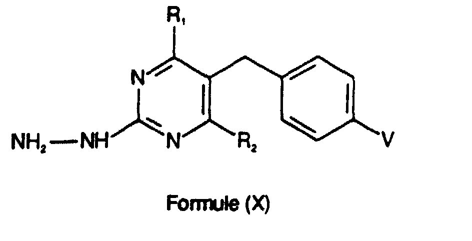 dans laquelle R₁, R₂ et V sont définis comme ci-dessus avec :
- les acides, chlorures d'acides ou esters carboxyliques
- les isocyanates ou isothiocyanates
- les orthoesters
- le carbonyl diimidazole
- l'urée ou le xanthogénate de potassium ou le disulfure de carbone ou un réactif analogue par chauffage sans solvant ou dans un solvant comme la N-méthyl pyrrolidone ou un alcool comme l'éthanol ou le méthoxy éthanol en présence ou non d'une base comme la triéthylamine, la pyridine ou la 2-méthyl-5-éthyl pyridine.

Selon les conditions opératoires notamment température et pH de la réaction on obtiendra des dérivés 1,2,4-triazolo [4,3-a] pyrimidine ou leur produit de réarrangement 1,2,4-triazolo [1,5-a] pyrimidine.

Les composés de formule (X) peuvent être obtenus par l'une quelconque des méthodes connues de la synthèse des hydrazino-2 pyrimidines (Cf. : The Pyrimidines ; The Chemistry of Heterocyclic Compounds ; D.J. BROWN ; Wiley Interscience 1970) notamment par la substitution des dérivés de formule (XI) où R₁, R₂ et V sont définis comme précédemment, par l'hydrate d'hydrazine par exemple. 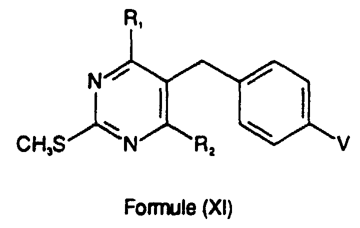 Les composés de formule (XI) sont obtenus par condensation de la S-méthyl thiourée sur les dérivés de formule (V) par exemple ou par l'une quelconque des méthodes de synthèse des 2-thiométhyl pyrimidines connues dans la littérature (Cf : The Pyrimidines, référence citée précédemment).

Les composés de formule (IX) dans lesquels :
- V possède une fonction ester : COOR₉ pourront être hydrolysés en milieu acide ou basique ou hydrogénés dans le cas où R₉ est un benzyl pour conduire aux composés de formule (I) où R₃ possède une fonction acide ;
- V possède une fonction nitrile pourront réagir avec un équivalent d'azoture de sodium dans un solvant tel que le diméthyl formamide en présence d'un sel d'ammonium comme le chlorure d'ammonium ou par chauffage dans le toluène ou le xylène avec un azoture d'un trialkyl étain, par exemple le triméthyl étain ou le tributyl étain, suivi d'un traitement acide par exemple l'acide chlorhydrique gazeux dans le tétrahydrofurane pour conduire aux composés de formule générale (I) où R₃ possède une fonction tétrazol-5-yl.
- Ces mêmes composés où V possède une fonction nitrile pourront être transformés par action de l'acide sulfurique ou par action de l'eau oxygénée ou encore par action de l'acide polyphosphorique en dérivés de formule générale (I) où R₃ possède une fonction amide.
- Les dérivés où V possède une fonction nitrile ou une fonction amide pourront également être transformés par hydrolyse basique ou acide en dérivés de formule générale (I) où R₃ possède une fonction acide.

Des sels d'addition de certains composés de formule (I) peuvent s'obtenir, en particulier des sels d'addition pharmaceutiquement acceptables. On peut citer en particulier lorsque les composés de la formule (I) présentent une fonction acide ou tétrazole : les sels de sodium, potassium, calcium, d'amine comme la dicyclohexylamine ou d'amino acide comme la lysine ; et lorsqu'ils présentent une fonction amine : un sel d'acide minéral ou organique comme par exemple chlorhydrate, méthane sulfonate, acétate, maléate, succinate, fumarate, sulfate, lactate, citrate.

Les nouveaux composés selon l'invention possèdent des propriétés pharmacologiques remarquables comme antagonistes des récepteurs à l'angiotensine Il et antiprolifératives et peuvent être utilisés en thérapeutique pour le traitement et la prévention des maladies cardiovasculaires en particulier pour traiter l'hypertension, l'insuffisance cardiaque et des maladies de la paroi artérielle notamment l'athérosclérose.

Ainsi, l'invention s'étend aux compositions pharmaceutiques renfermant comme principe actif, les médicaments constitués par une quantité pharmaceutiquement efficace d'au moins un composé de formule (I), tel que précédemment défini, ainsi qu'éventuellement l'un de ses sels d'addition pharmaceutiquement acceptables.

Ces compositions peuvent être administrées par voie buccale, rectale, par voie parentérale, par voie transdermique ou par voie oculaire.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les systèmes transdermiques et les collyres. Elles sont préparées selon les méthodes usuelles. Le principe actif, constitué par une quantité pharmaceutiquement efficace d'au moins un composé de formule (I) défini comme ci-dessus ou un de ses sels d'addition pharmaceutiquement acceptables, peut y être incorporé à des excipients habituellement employés dans ces compositions pharmaceutiques, tels que le talc, la gomme arabique, le lactose, l'amidon, le stéarate de magnésium, la polyvidone, les dérivés de la cellulose, le beurre de cacao, les glycérides semi-synthétiques, les véhicules aqueux ou non, les corps gras d'origine animale ou végérale, les glycols, les divers agents mouillants, dispersants ou émulsiants, les gels de silicone, certains polymères ou copolymères, les conservateurs, arômes et colorants.

L'invention couvre encore une composition pharmaceutique à activité antagoniste des récepteurs à l'angiotensine Il et ou antiproliférative permettant notamment de traiter favorablement ou de prémunir les maladies cardiovasculaires en particulier l'hypertension, l'insuffisance cardiaque et les maladies de la paroi artérielle notamment l'athérosclérose caractérisée en ce qu'elle comprend une quantité pharmaceutiquement efficace d'au moins un composé de formule (I) précitée ou un de ses sels d'addition pharmaceutiquement acceptables, pouvant être incorporé dans un excipient, véhicule ou support pharmaceutiquement acceptable.

La posologie varie notamment en fonction de la voie d'administration, de l'affection traitée et du sujet en cause.

Par exemple, chez l'adulte de poids moyen 60 à 70 kg, elle peut varier entre 1 et 400 mg de principe actif en une ou plusieurs doses journalières par voie orale, ou de 0,01 à 50 mg en une ou plusieurs doses journalières par voie parentérale.

L'invention couvre encore un procédé de préparation d'une composition pharmaceutique, caractérisé en ce qu'on incorpore une quantité pharmaceutiquement efficace d'au moins un composé de formule (I) tel que défini précédemment, ou un de ses sels d'addition pharmaceutiquement acceptables, dans un excipient, véhicule ou support pharmaceutiquement acceptable. Cette composition pharmaceutique peut être formulée sous forme de gélules, de comprimés dosés de 1 à 400 mg ou sous forme de préparations injectables dosées de 0,01 à 50 mg.

L'invention couvre encore un procédé de traitement thérapeutique des mammifères, caractérisé en ce que l'on administre à ce mammifère une quantité thérapeutiquement efficace d'au moins un composé de formule (I) tel que précédemment défini, ou un de ses sels d'addition pharmaceutiquement acceptables.

En thérapeutique animale, la dose journalière utilisable se situe habituellement de 1 à 100 mg par kg.

D'autres caractéristiques et avantages de l'invention seront mieux compris à la lecture qui va suivre de quelques exemples de préparation nullement limitatifs, mais donnés à titre d'illustration.

### Exemple 1 : 3-oxo hexanoate d'éthyle

**Formule (III)** : R'₁ = n-Propyl, R₈ = O-ethyl

176 g de 2,2-diméthyl-4,6-dioxo-1,3-dioxanne (acide de Meldrum) sont dissous dans 550 ml de dichlorométhane et 188 ml de pyridine ; le mélange est refroidi à 5°C par un bain d'eau et de glace et 133 ml de chlorure d'acide butanoïque sont ajoutés goutte à goutte. A la fin de l'addition le mélange est agité trois heures à température ambiante. La solution est lavée avec une solution diluée d'acide chlorhydrique, séchée sur sulfate de magnésium et évaporée sous vide pour donner une huile. Cette huile est dissoute dans 700 ml d'éthanol et le mélange est chauffé au reflux pendant six heures. L'éthanol est évaporé sous vide et le résidu obtenu est distillé pour donner 145,4 g de 3-oxo hexanoate d'éthyle sous forme d'une huile.
Température d'ébullition (20 mm de mercure) : 98°-100°C.

Selon le mode opératoire de l'exemple 1, le composé de l'exemple 2 a été préparé.

### Exemple 2 : 3-oxo heptanoate d'éthyle

**Formule (III)**: R'₁ = n-Butyl, R₈ = O-ethyl

Température d'ébullition (20 mm de mercure) : 115°-120°C.

### Exemple 3 : 4-benzyloxy-3-oxo butanoate d'éthyle

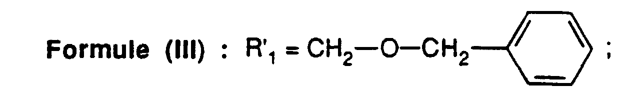

R₈ = O-éthyl

On ajoute par portions 80 g de NaH à 60 % dans 800 ml de THF anhydre. La température du milieu est ramenée et maintenue à 10°C. On introduit alors goutte à goutte 500 ml d'alcool benzylique. Rajouter ensuite 65.8 g de 4-chloro acétoacétate d'éthyle en solution dans 200 ml d'alcool benzylique. Le mélange est agité à température ambiante pendant 20 h. Neutraliser par addition lente d'acide acétique (120 ml) tout en refroidissant par un bain de glace. L'ensemble est ensuite versé dans un mélange d'eau et de glace et extrait à l'éther. La phase organique est lavée avec une solution de bicarbonate de sodium, séchée sur MgSO₄ puis concentrée pour obtenir une huile orangée. Le produit est purifié par deux distillations successives pour donner une huile jaune.
Point d'ébullition (sous 0.05 mm de mercure) : 126-132°C.

### Exemple 4 : 4'-Bromo méthyl 2-cyano biphényl

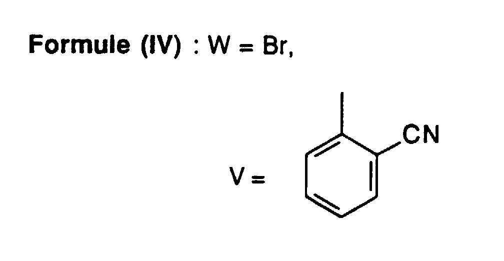

### a) Préparation de 2-cyano-4'-méthyl biphényl :

563,8 g d'acide (4'-méthyl biphényl-2-yl) carboxylique préparé selon MEYERS A.I. ; MIHELICH E.D. ; J. Am. Chem. Soc., 1975, 97 (25), 7383, sont rajoutés par petites portions dans 800 ml de chlorure de thionyle. Le mélange est porté au reflux pendant deux heures. Le chlorure de thionyle est concentré sous vide et le résidu est versé sur une solution à 28 % d'hydroxyde d'ammonium refroidie au préalable par un bain d'eau et de glace. Le mélange est agité pendant 30 minutes et les cristaux obtenus sont essorés et lavés à l'eau puis à l'éther puis séchés pour donner 554,8 g de 4'-méthyl biphényl 2-yl- carboxamide sous forme de cristaux de point de fusion 128°-132°C. Ces cristaux sont repris dans 1300 ml de chlorure de thionyle et le mélange est chauffé 3 heures au reflux puis concentré sous vide pour donner une huile orangée. Reprendre dans deux litres de chloroforme, laver à l'eau puis sécher la phase organique et concentrer pour obtenir 509.8 g d'huile qui cristallise dans le pentane pour donner 467.3 g de 2-cyano-4'-méthyl biphényl.

Point de fusion 46°-48°C.

### b) 4'-bromométhyl-2-cyano biphényl

467.3 g de 2-cyano-4'-méthyl biphényl préparés ci-dessus sont dissous dans 4,7 l de 1,2-dichloro éthane en présence de 467.3 g de N-bromo succinimide et de 9.3 g de péroxyde de benzoyle. Le mélange est chauffé très progressivement pour bien contrôler l'effet exothermique. Porter ensuite 4 h au reflux. Refroidir à 50°C puis laver 3 fois à l'eau chaude, sécher, concentrer la phase organique pour obtenir des cristaux crème.

Une recristallisation dans l'isopropanol conduit à 451 g de cristaux blancs de 4'-bromométhyl-2-cyano biphényl.

Point de fusion 128°C.

### Exemple 5: 2-[(2'-cyano biphényl-4-yl) méthyl] 3-oxo hexanoate d'éthyle

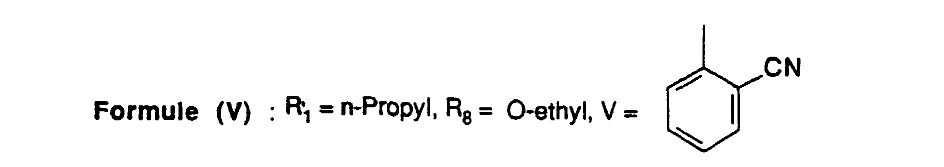

23 g de 3-oxo hexanoate d'éthyle préparés à l'exemple 1 sont dissous dans 120 ml de tétrahydrofuranne. 30.3 g de 4'-bromo méthyl-2-cyano biphényl préparés à l'exemple 4 et 4.7 g de chlorure de lithium sont ajoutés et le mélange est agité à température ambiante. On introduit alors goutte à goutte, 39 ml de diisopropyl éthyl amine, ce qui provoque un léger effet exothermique. Le mélange est ensuite agité trois heures à température ambiante, puis dix heures au reflux. Les solvants sont évaporés sous vide et le résidu est repris à l'eau puis extrait au chloroforme. La phase organique est décantée puis lavée avec une solution diluée d'acide chlorhydrique, séchée sur sulfate de magnésium et évaporée sous vide pour donner 38 g d'une huile orangée.

Une purification par chromatographie sur gel de silice (éluant CHCl₃) conduit à 32.3 g de 2-[(2'-cyano biphényl-4-yl) méthyl] 3-oxo hexanoate d'éthyle.

Selon le mode opératoire de l'exemple 5 mais en utilisant le β céto ester approprié, on obtient les composés des exemples 6 à 10.

### Exemple 6 : 2-[(2'-cyano biphényl-4-yl) méthyl)-3-oxo heptanoate d'éthyle

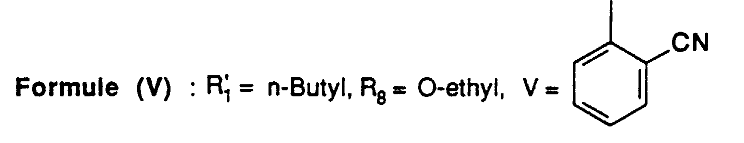

Huile utilisée telle quelle pour la suite.

### Exemple7 : 2-[(2'-cyano biphényl-4-yl) méthyl]-3-oxo butanoate d'éthyle

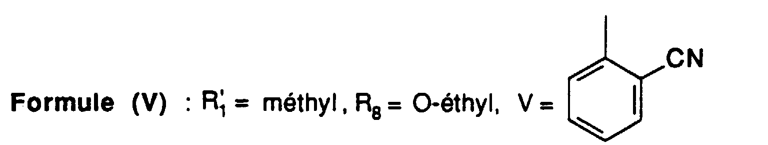

Huile jaune purifiée par chromatographie sur gel de silice (éluant chloroforme 95 % / éther 5 %).

### Exemple 8 : 2-[(2'-cyano biphényl-4-yl) méthyl)-3-oxo pentanoate d'éthyle

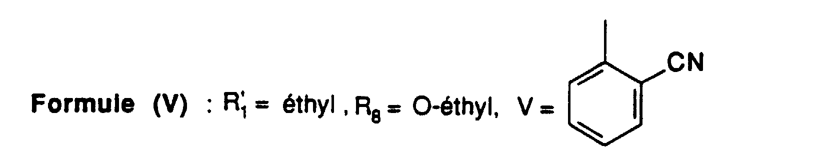

Huile purifiée par chromatographie sur gel de silice (éluant CHCl₃ 95 % / éther 5 %).

### Exemple 9 : 2-[(2'-cyano biphényl-4-yl) méthyl]-4-méthoxy-3-oxo butanoate d'éthyle

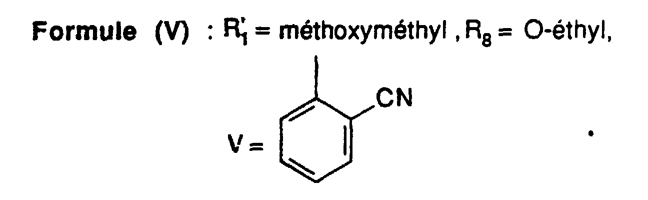

Huile jaune purifiée par chromatographie sur gel de silice (éluant CHCl₃ 95 % / éther 5 %).

### Exemple 10 : 4-benzyloxy 2-[(2'-cyano biphényl-4-yl) méthyl]3-oxo butanoate d'éthyle

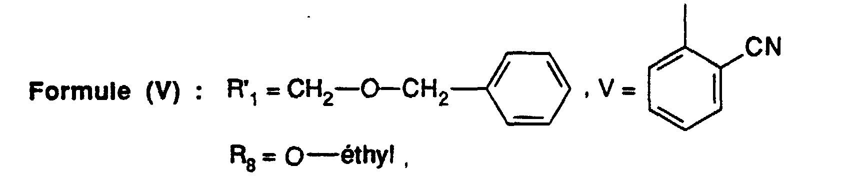

Huile purifiée par deux chromatographies successives (éluants chloroforme puis cyclohexane 80 %/acétate d'éthyle 20 %).

### Exemple 11 : 2-[4-(3-cyano-2-pyridyl) benzyl]-3-oxo hexanoate d'éthyle

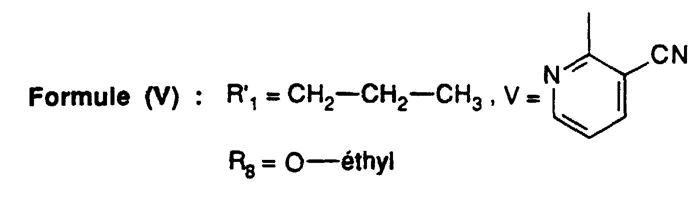

### a) Préparation du 4-bromobenzyl méthyl éther:

Une solution de méthanolate de sodium préparée à partir de 11.8 g de sodium et de 350 ml de méthanol est introduite goutte à goutte à une suspension de 117.7 g de bromure de 4-bromo benzyl dans 350 ml de méthanol. Agiter 2 h à température ambiante et laisser au repos une nuit. Evaporer le méthanol, reprendre à l'éther, laver à l'eau la phase organique puis sécher et concentrer pour obtenir une huile jaune que l'on purifie par distillation. Ainsi on obtient 102 g d'un liquide incolore de bromobenzyl méthyl éther. Température d'ébullition sous 17 mm de mercure : 112-114°C.

### b) Synthèse de la 3-cyano-2-(4-méthoxy méthyl phényl) pyridine

On ajoute, 2 g du composé 4-bromo benzyl méthyl éther, préparé précédemment, à une suspension de 18 g de magnésium dans 50 ml de THF anhydre. La formation du magnésien est amorcée avec quelques cristaux d'iode et éventuellement par un chauffage à l'aide d'un bain d'eau tiède. Introduire goutte à goutte 121.8 g de 4-bromo benzyl méthyl éther en solution dans 200 ml de THF anhydre de sorte que la température n'excède pas 40°C. Faire réagir pendant 1 h à température ambiante, puis introduire sous azote et par surpression d'azote 800 ml d'une solution de chlorure de zinc dans l'éther. Un précipité blanc se forme. Laisser réagir 1 h 30 à température ambiante. Ajouter 800 mg du catalyseur de couplage chlorure de bis (triphényl phosphine) nickel Il, [NiP(phényl)₃]₂Cl₂ puis introduire 76.9 g de 2-chloro nicotinonitrile en solution dans 300 ml de THF. Le mélange est agité pendant une nuit à température ambiante. Concentrer sous vide. Reprendre dans un mélange de 1 l de dichlorométhane, 1 l d'eau et 1 l de disel de sodium de l'EDTA. Essorer l'émulsion sur célite 545. Décanter, laver la phase organique à l'eau, sécher, concentrer pour obtenir 133.6 g d'une huile orangée que l'on purifie par deux chromatographies successives (éluant chloroforme 95 % / éther 5 %). On isole ainsi 69.4 g de 3-cyano-2-(4-méthoxy méthyl phényl) pyridine sous forme d'huile orangée qui cristallise.

Point de fusion 74°C.

### c) Préparation de 3-cyano-2-(4-bromo méthyl phényl) pyridine

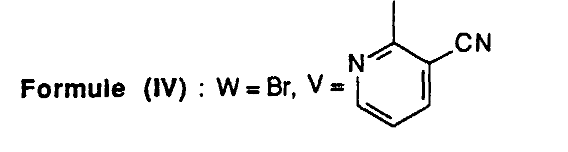

Dissoudre 69.4 g de 3-cyano-2-(4-méthoxy méthyl phényl) pyridine, préparé à l'étape précédente, dans 700 ml de chloroforme stabilisé à l'amylène. Refroidir à -10°C. Introduire goutte à goutte 66 ml de BBr₃ en solution dans 200 ml de chloroforme stabilisé à l'amylène de sorte que la température n'excède pas 5°C. Laisser le mélange 1 h 30 dans un bain de glace. Hydrolyser, avec de la glace puis par de l'eau. Filtrer, reprendre la suspension avec un mélange d'eau et de chloroforme. Décanter, réunir les phases organiques, sécher puis concentrer pour obtenir 78.2 g de cristaux crème de 3-cyano-2-(4-bromo méthyl phényl) pyridine.

Point de fusion : 118°C.

### d) Préparation de 2-[4-(3-cyano-2-pyridyl) benzyl]-3-oxo hexanoate d'éthyle

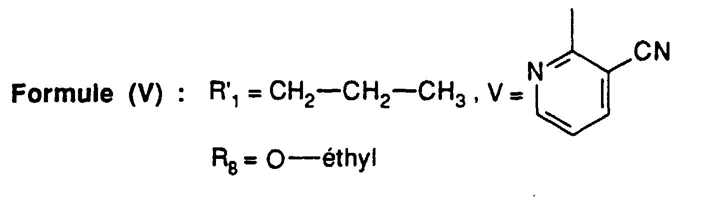

Selon le mode opératoire de l'exemple 5, on obtient le dérivé attendu sous forme d'une huile orangée utilisée telle quelle à l'étape suivante.

### Exemple 12 : 2-[4-(3-cyano-2-thiényl) benzyl]-3-oxo hexanoate d'éthyle

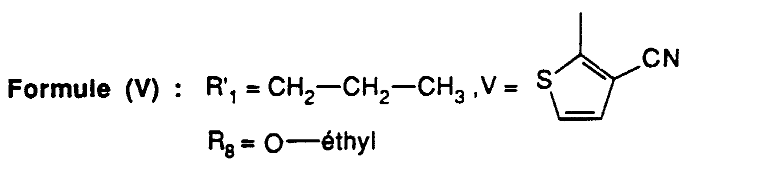

### a) Préparation de 4-chloro-1-(4-méthyl phényl) butanone

Un mélange de 560 ml de chlorure de l'acide 4-chloro butyrique et de 550 ml de toluène est ajouté goutte à goutte à une suspension de 740 g d'AlCl₃ dans 2 l de dichlorométhane en maintenant la température entre 10° et 15°C. Agiter 30 mn à température ambiante. Le mélange réactionnel est versé dans la glace. Décanter, séparer la phase organique, extraire au dichlorométhane la phase aqueuse. Réunir les phases organiques, laver à l'eau puis sécher et concentrer sous vide pour obtenir 994.5 g de 4-chloro-1-(4-méthyl phényl) butanone sous forme d'huile utilisée sans purification ultérieure à l'étape suivante.

### b) Préparation de 3-chloro-2-(2-chloroéthyl)-3-(4-méthyl phényl)-2-propen-1-al

On introduit, goutte à goutte, à une température comprise entre 7° et 12°C, 390 ml de POCl₃ dans 352.5 g de 4-chloro-1-(4-méthyl phényl) butanone, préparés précédemment selon l'exemple 12 a), en solution dans 450 ml de DMF. Porter la température, progressivement, d'abord à 50°C pendant 2 h puis à 75°C pendant 45 mn. Verser le mélange sur de la glace, extraire à l'éther trois fois, réunir les phases organiques, les laver à l'eau puis sécher et évaporer pour obtenir 387.8 g de 3-chloro-2-(2-chloroéthyl)-3-(4-méthyl phényl)-2-propen-1-al sous forme d'huile utilisée telle quelle à l'étape suivante.

### c) Préparation de 4,5-dihydro-3-formyl-2-(4-méthyl phényl) thiophène

Un mélange de 200 g de 3-chloro-2-(2-chloro éthyl)-3-(4-méthyl phényl)-2-propen-1-al préparé à l'exemple 12 b), 2.2 l de THF, 276.5 g de Na₂S, 9H₂O et 373 ml d'eau est porté au reflux pendant 6 h. Concentrer sous vide, reprendre à l'eau et extraire 3 fois à l'éther. Les phases organiques sont réunies et lavées à l'eau. Sécher et concentrer pour obtenir 170.3 g d'une huile qui cristallise. Point de fusion inférieur à 50°C.

### d) Préparation de 4,5-dihydro-3-formyl-2-(4-méthyl phényl)thiophène oxime

On ajoute, par portions, 132.1 g de chlorhydrate d'hydroxylamine dans 323.5 g d'aldéhyde préparé selon 12c), en solution dans 800 ml d'éthanol. Une solution de carbonate de sodium préparée à partir de 100.5 g de Na₂CO₃ et 700 ml d'eau est alors additionnée goutte à goutte. Porter le mélange à 40°C pendant 5 mn puis laisser la réaction à température ambiante pendant 1 h. Refroidir à 15°C, filtrer le solide, laver à l'eau puis avec un mélange d'éther isopropylique 50 % / éther de pétrole 50 % pour obtenir 252 g d'oxime. Une extraction du filtrat par du dichlorométhane permet d'obtenir un 2ème jet de 99 g d'oxime attendu.

### e) Préparation de 3-cyano-4,5-dihydro-2-(4-méthyl phényl) thiophène

On porte au reflux pendant 3 h 171.8 g d'oxime, préparé à l'exemple 12d), en solution dans 680 ml d'anhydride acétique. Concentrer pour enlever l'excès d'anhydride puis distiller pour obtenir 115.3 g de dérivé nitrile.
Point d'ébullition sous 0.05 mm de mercure : 140-150°C.

### f) Préparation de 3-cyano-2-(4-méthyl phényl) thiophène

Introduire goutte à goutte, 62 ml de brome, dans une solution, portée au préalable à 50°C, de 191.3 g de nitrile, préparés selon l'exemple 12e), dans 1.85 l de CCl₄. L'ensemble est porté au reflux jusqu'à ce que le dégagement de HBr s'arrête. Evaporer le CCl₄ et distiller pour obtenir 115.3 g de 3-cyano-2-(4-méthyl phényl) thiophène.
Point d'ébullition sous 0.05-0.1 mm de mercure : 130-150°C.

### g) 2-(4-bromo méthyl phényl)-3-cyano thiophène

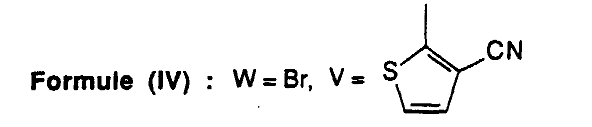

182.2 g du composé obtenu à l'exemple 12 f), sont bromés selon l'exemple 4 pour donner 133.7 g de 2-(4-bromo méthyl phényl)-3-cyano thiophène.

Point de fusion : 80-84°C.

### h) 2-[4-(3-cyano-2-thiényl) benzyl]-3-oxo hexanoate d'éthyle

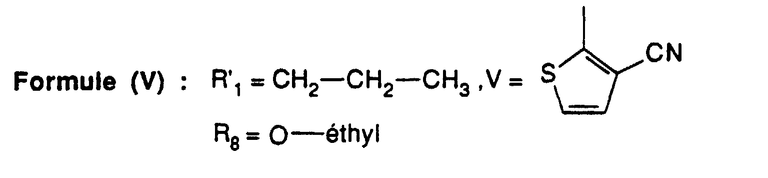

On porte au reflux pendant 15 h un mélange de 50 g de 2-(4-bromo méthyl phényl)-3-cyano thiophène, préparés précédemment, 40 g de 3-oxo hexanoate d'éthyle préparés à l'exemple 1, 300 ml de THF, 62 ml de diisopropyl éthylamine et 15.6 g de UBr. Concentrer sous vide, additionner une solution d'acide chlorhydrique diluée et extraire à l'acétate d'éthyle. Les phases organiques sont réunies, lavées à l'eau, séchées et évaporées pour conduire à 62.4 g de 2-[4-(3-cyano-2-thiényl) benzyl]-3-oxo hexanoate d'éthyle sous forme d'huile utilisée sans purification ultérieure.

### Exemple 13: 2-[4-(3-cyano-2-furyl) benzyl]-3-oxo hexanoate d'éthyle

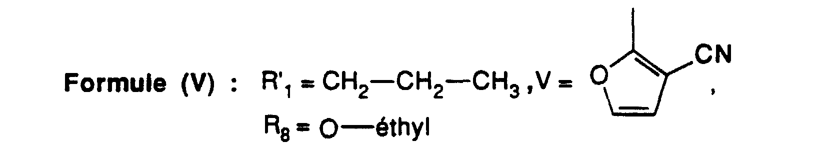

### a) Préparation d'acide 2-(4-méthyl phényl)-3-furanoïque

70.7 g de p-toluidine refroidi par un bain d'eau et de glace sont traités avec 205 ml de HCI à 36 %. Le mélange est ensuite agité à 55°-60°C pendant 30 mn avant d'être à nouveau refroidi à 0°C. Introduire alors une solution de 45 g de NaNO₂ dans 220 ml d'eau. Agiter 1 h à 0°C. Cette solution froide est introduite dans un mélange de 49.3 g d'acide 3-furanoique, 220 ml d'acétone, 23.4 g de CuCl₂ et 6.3 g d'eau refroidi à -5°C. L'ensemble est agité à 0°C pendant 2 h puis pendant 48 h à température ambiante. Extraire deux fois à l'éther, décanter, sécher la phase organique et concentrer pour obtenir une huile dont le traitement à l'eau conduit à des cristaux. Filtrer, laver avec 50 ml d'un mélange méthanol / eau à 50 % pour obtenir 13.4 g d'acide 2-(4-méthyl phényl)-3-furanoique.

Point de fusion : 166°C.

### b) Préparation de 2-(4-méthyl phényl)-3-furane carboxamide

On rajoute 20 ml de SOCl₂ à 13.4 g d'acide furanoïque préparés précédemment en solution dans 70 ml de toluène. Porter au reflux 3 h puis distiller l'excès de SOCl₂ et le toluène pour obtenir une huile que l'on fait réagir à 5°C avec une solution de 100 ml de diméthoxy-1,2-éthane saturé d'ammoniac. Le précipité est essoré, lavé à l'eau puis à l'alcool isopropylique pour donner 7 g de cristaux blancs d'amide.

Point de fusion : 174°C.

### c) Préparation de 3-cyano-2-(4-méthyl phényl) furane

On porte au reflux pendant 3 h, un mélange de 12.2 g d'amide préparés ci-dessus et 65 ml de SOCl₂. Concentrer sous vide. Reprendre au chloroforme l'huile obtenue puis rajouter de l'eau et de la glace. Décanter, extraire la phase aqueuse avec du chloroforme, réunir les phases organiques, sécher, évaporer pour obtenir une huile. La purification par chromatographie sur gel de silice (éluant toluène) conduit à 7.5 g d'une huile qui cristallise.

Point de fusion 66°C.

### d) 2-(4-bromo méthyl phényl)-3-cyano furane

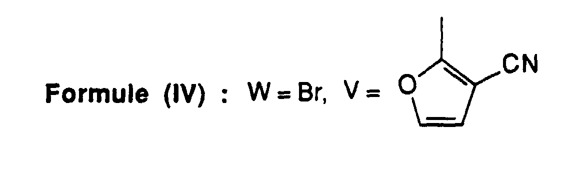

Les 7.5 g du composé obtenu à l'exemple 13c) sont bromés selon l'exemple 4 pour donner après purification par chromatographie sur gel de silice (éluant pentane 50 % / toluène 50 %) 4.6 g de 5-bromo-3-cyano-2-(4-méthyl phényl) furane, (point de fusion 88°C), 2.2 g de 5-bromo-3-cyano-2-(4-bromo méthyl phényl) furane (point de fusion 114°C) et 2 g de 2-(4-bromo méthyl phényl)-3-cyano furane.

Point de fusion 108°C.

Le composé 5-bromo-3-cyano-2-(4-méthyl phényl) furane est remis en réaction de bromation selon l'exemple 4 pour donner le 5-bromo-2-(4-bromo méthyl phényl)-3-cyano furane qui constitue le composé de **l'exemple 13 d)bis.**

### e) 2-[4-(3-cyano-2-furyl) benzyl]-3-oxo hexanoate d'éthyle

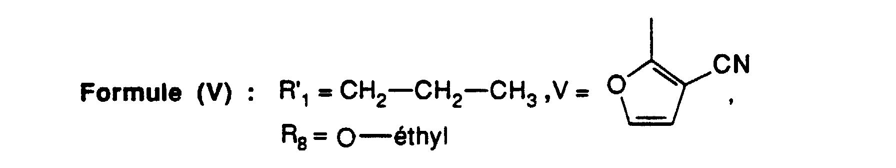

Le dérivé 2-(4-bromo méthyl phényl)-3-cyano furane ainsi obtenu est traité selon l'exemple 5 pour donner le 2-[4-(3-cyano-2-furyl) benzyl]-3-oxo hexanoate d'éthyle sous forme d'huile utilisée à l'état brut à l'étape suivante.

De même, le dérivé 5-bromo-2-(4-bromo méthyl phényl)-3-cyano furane de l'exemple 13d)bis est traité selon l'exemple 5 pour donner le 2-[4-(5-bromo-3-cyano-2-furyl)benzyl]-3-oxo hexanoate d'éthyle sous forme d'huile qui constitue le dérivé de **l'exemple 13 bis.**

### Exemple 14 : 3-[(2'-cyanobiphényl-4-yl) méthyl]-2,4-dioxo pentane

**Formule (V) :** R'₁ = CH₃, R₈ = CH₃,

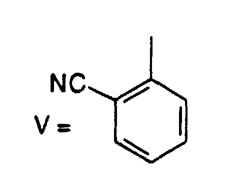

On chauffe au reflux pendant 27 h, 32.8 g de 2,4-dioxo pentane , 68 g de 4'-bromo méthyl 2-cyano biphényl préparés à l'exemple 4, 88 ml de diisopropylamine et 10.6 g de chlorure de lithium anhydre dans 300 ml de tétrahydrofurane. Refroidir, essorer le précipité. Concentrer à sec la phase organique pour obtenir 88.5 g de cristaux. Reprendre avec de l'isopropanol, filtrer pour isoler 38.8 g de 4'-bromo méthyl 2-cyano biphényl qui n'ont pas réagi. Les eaux mères concentrées conduisent à 26.5 g d'une huile dont la purification sur gel de silice (éluant chloroforme) conduit à 5.3 g de 4'-bromométhyl 2-cyano biphényl supplémentaire et 12.2 g de 3-[(2'-cyanobiphényl-4-yl) méthyl]-2,4-dioxo pentane attendu sous forme d'huile jaune.

### Exemple 15 : 5-[(2'-cyano biphényl-4-yl) méthyl]-4,6-dioxo nonane

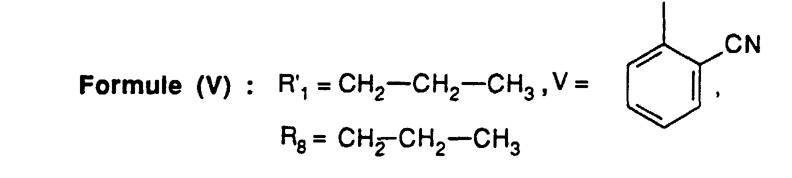

Dissoudre 15.6 g de 4,6 dioxo nonane, préparés à partir de méthyl propyl cétone et de butyrate d'éthyle en présence d'amidure de lithium (selon CA 42 : 4129 f) dans 160 ml de DMF anhydre. Ajouter par portions 4 g de NaH à 60 %. Après l'effet exothermique, ramener la température à l'ambiante et introduire goutte à goutte une solution de 27.2 g de 4'-bromo méthyl-2-cyano biphényl préparés à l'exemple 4 dans 90 ml de DMF. Agiter 30 min à température ambiante puis chauffer à 60°C pendant 2 h. Concentrer sous vide, reprendre dans un mélange eau / dichrométhane, acidifier par une solution diluée de HCI. Décanter et extraire deux fois au dichlorométhane. Les phases organiques sont lavées à l'eau, séchées puis concentrées pour obtenir 36.3 g d'une huile que l'on purifie par deux chromatographies successives. (éluant : chloroforme puis cyclohexane 90 % / acétate d'éthyle 10 % respectivement) pour obtenir un solide, identifié par RMN comme étant le tautomère énolique, point de fusion 105°C et une huile correspondant à la forme tautomère dicétone.

### Exemple 16 : 2,4-dioxo-3-[(2'(1-H-tétrazol-5-yl) biphényl-4-yl) méthyl] pentane

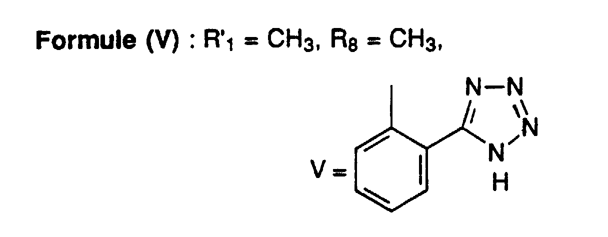

Un mélange de 11.8 g de 3-[(2'-cyano biphényl-4-yl) méthyl]-2,4 dioxo pentane préparés à l'exemple 14, 200 ml de xylène et 9.3 g d'azoture de triméthyl étain est porté au reflux pendant 50 h. Au bout de 24 h on rajoute un deuxième équivalent d'azoture de triméthyl étain.

Refroidir. Concentrer pour obtenir une huile visqueuse dont la chromatographie sur gel de silice (éluant chloroforme 90 % méthanol 10 %) conduit à 9.3 g de cristaux.

Un traitement supplémentaire à l'acétonitrile permet d'obtenir 6.2 g de 2,4-dioxo-3-[(2'(1-H-tétrazol-5-yl) biphényl-4-yl) méthyl] pentane analytiquement pur.

Formule brute : C₁₉ H₁₈ N₄ O₂

Point de fusion : 166°C.

### Exemple 17 :2-[[2'-(1H-tétrazol-5-yl) biphényl-4-yl] méthyl]-3-oxo hexanoate d'éthyle

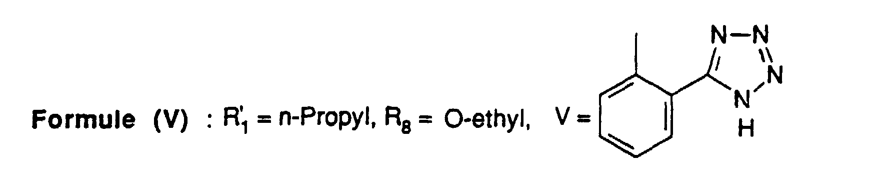

Un mélange de 69.9 g de 2-[(2'-cyano biphényl-4-yl) méthyl] 3-oxo hexanoate d'éthyle préparés selon l'exemple 5, 700 ml de toluène anhydre et 47.5 g d'azoture de triméthyl étain, préparés à partir d'azoture de sodium et de chlorure de triméthyl étain, est porté au reflux pendant 24 h. Rajouter 47.5 g d'azoture de triméthyl étain supplémentaire et continuer le reflux pendant 16 h. Concentrer à 50 %. La solution orangée obtenue est purifiée par deux chromatographies successives. (Eluant : chloroforme 90 % méthanol 10 % puis chloroforme 95 % méthanol 5 %) pour donner 58 g d'une huile orangée qui cristallise.

Point de fusion 65°C.

### Exemple 18: (Méthode A)

### 6-[(2'-cyano biphényl-4-yl) méthyl]-7-hydroxy-5-propyl-1,2,4-triazolo [1,5-a] pyrimidine

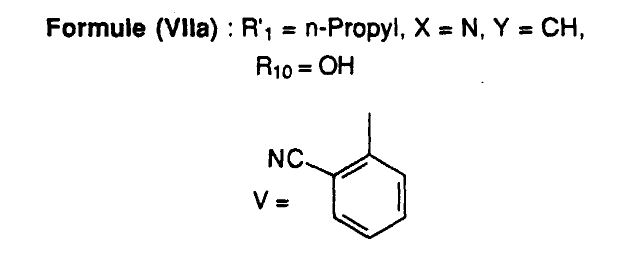

On chauffe au reflux pendant 6 h, 1.7 g de 3-amino 1,2,4-triazole, 7 g de β céto ester préparés à l'exemple 5, et 30 ml d'acide acétique. Evaporer l'acide acétique. L'huile obtenue est purifiée par chromatographie sur gel de silice (éluant CHCl₃ 90 % / MeOH 10 %) pour conduire à 5.2 g de β céto ester de départ et 1.2 g de 6-[(2'-cyano biphényl-4-yl) méthyl]-7-hydroxy-5-propyl-1,2,4-triazolo [1,5-a] pyrimidine.

Point de fusion 200-205°C.
- ¹H RMN (200 MHz ; DMSO-d₆) :: 2.65 (t, 2H, CH₂ Propyle) ;
8.2 (s, 1H, H₂)
- UV (10 µg/ml MeOH) :: λₐ = 209.1 nM
λ_{b} = 257.7 nM
λ_{c} = 286.8 nM

### Exemple 19 : (Méthode B)

### 6-[(2'-cyano biphényl-4-yl) méthyl]-5-hydroxy-7-propyl-1,2,4-triazolo [1,5-a] pyrimidine

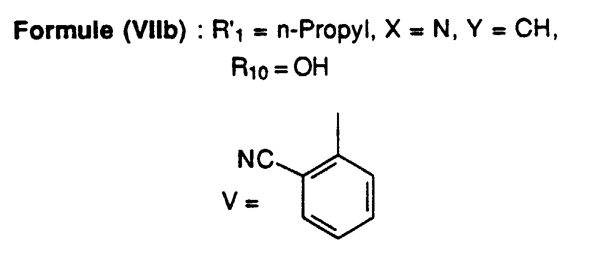

On chauffe au reflux pendant 7 h, 7.1 g de β-ceto ester préparés à l'exemple 5, 1,7 g de 3-amino-1,2,4 triazole et 70 ml de 1,2,4-trichloro benzène.Concentrer sous vide. L'huile épaisse obtenue est chromatographiée sur gel de silice (éluant : CHCl₃ 95 % / MeOH 5 %) pour conduire à 0.8 g d'isomère obtenu à l'exemple 18 (point de fusion 200°C) et 2.2 g de 6-[(2'-cyano biphényl-4-yl) méthyl]-5-hydroxy-7 propyl-1,2,4-triazolo [1,5-a] pyrimidine.

Point de fusion 212°C.
- ¹H RMN (DMSO-d₆) :: 2.98 (t, 2H, CH₂ propyl) ; 8.1 (s, 1H, H₂)
- UV (10 µg/ml MeOH) :: λₐ = 207.5 nM
λ_{b} = 258.2 nM

### Exemple 20: (Méthode C)

### 6-[(2'-cyano biphényl-4-yl) méthyl]-5-hydroxy-7-propyl-1,2,4-triazolo [4,3-a] pyrimidine

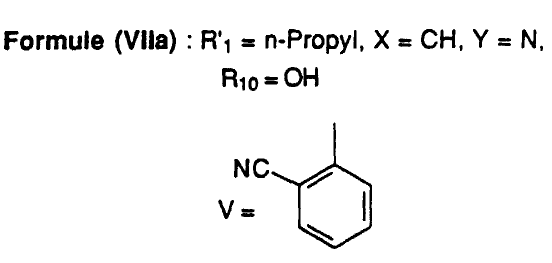

### a) 5-[(2'-cyano biphényl-4-yl) méthyl]-4-hydroxy-6-propyl-2-mercapto pyrimidine :

A une solution de méthanolate de sodium préparée à partir de 4.6 g de sodium et 150 ml de méthanol, ajouter par spatule 11 g de thiourée. Puis introduire goutte à goutte 34.9 g de β céto ester, préparés à l'exemple 5, en solution dans 50 ml de méthanol. Laisser reposer une nuit puis chauffer au reflux pendant 7 h. Concentrer sous vide, reprendre avec 500 ml d'eau puis acidifier avec HCI concentré pour ramener le pH à 1. Le précipité gommeux est isolé, repris au méthanol pour donner 17.3 g de cristaux blancs de 5-[(2'cyano biphényl-4-yl) méthyl]-4-hydroxy-6-propyl-2-mercapto pyrimidine.

Point de fusion 196°C.

### b) 5-[(2'-cyano biphényl-4-yl) méthyl]-4-hydroxy-2 méthylmercapto 6-propyl pyrimidine

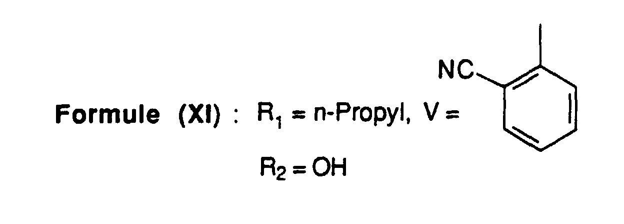

17.3 g de composé obtenus précédemment sont introduits par portions dans un mélange de 340 ml de méthanol et 2.9 g de KOH. Après obtention d'une solution limpide, refroidir puis introduire goutte à goutte 3.4 ml de ICH₃.

Laisser réagir pendant 2 h à température ambiante.

Filtrer le précipité pour obtenir 17.2 g de 5-[(2'-cyano biphényl-4-yl) méthyl]-4-hydroxy-2-méthylmercapto 6-propyl pyrimidine.

Point de fusion 220°C.

### c) 5-[(2'-cyano biphényl-4-yl) méthyl]-2-hydrazino-4-hydroxy 6-propyl pyrimidine

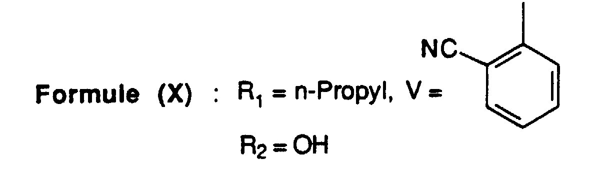

12.4 g de 5-[(2'-cyano biphényl-4-yl) méthyl]-4-hydroxy-2-méthyl mercapto 6-propyl pyrimidine préparés précédemment sont dissous dans 370 ml de 2-méthoxy éthanol. Introduire 33 ml d'hydrate d'hydrazine puis porter au reflux pendant 3 h. Concentrer sous vide. Reprendre dans l'acétonitrile, triturer. Le solide obtenu est essoré, lavé à l'éther et à l'éther isopropylique pour conduire à 9.9 g de 5-[(2'-cyano biphényl-4-yl) méthyl]-2-hydrazino-4-hydroxy 6-propyl pyrimidine.

Point de fusion 191°C.

### d) 6-[(2'-cyano biphényl-4-yl) méthyl]-5-hydroxy-7-propyl-1,2,4-triazolo [4,3-a] pyrimidine :

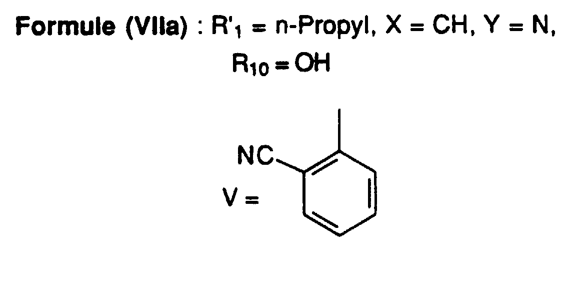

10 g de 5-[(2'-cyano biphényl-4-yl) méthyl]-2-hydrazino-4-hydroxy-6 propyl pyrimidine préparés comme ci-dessus sont placés dans 100 ml d'acide formique. Porter le mélange au reflux pendant 4 h. Concentrer sous pression réduite, reprendre à l'eau l'huile épaisse obtenue et triturer jusqu'à cristallisation.

Le composé est purifié par chromatographie sur gel de silice : éluant CHCl₃ 95 % / Méthanol 5 %.

Ainsi on obtient 8.3 g de 6-[(2'-cyano biphényl-4-yl) méthyl]-5-hydroxy-7-propyl-1,2,4-triazolo [4,3-a] pyrimidine.

Point de fusion 217°C.

¹H RMN (DMSO-d₆) : 2.6 (t, 2H, CH₂ propyl) ; 9 (s, 1H, H₃)
- UV (10 µg/ml MeOH) :: λₐ = 210.2 nM
λ_{b} = 257.5 nM
λ_{c} = 303.4 nM

### Exemple 21 : 6-[(2'-cyano biphényl-4-yl) méthyl]-7-hydroxy-5-propyl-1,2,4-triazolo [4,3-a] pyrimidine

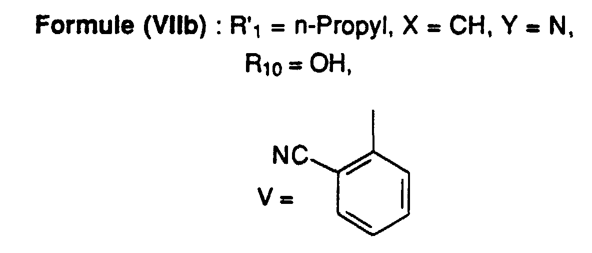

Selon le mode opératoire de l'exemple 20, à l'étape d), on obtient en même temps que le composé décrit précédemment 1.1 g de 6-[(2'-cyano biphényl-4-yl) méthyl]-7-hydroxy-5-propyl-1,2,4-triazolo [4,3-a] pyrimidine.

Point de fusion 204-206°C.

RMN (DMSO-d₆) : 2.9 (t, 2H, CH₂ propyl) ; 9 (s, 1H, H₃)
- UV (10 µg/ml, MeOH) :: λₐ = 211.5 nM
λ_{b} = 260 nM

Les composés des exemples 20 et 21 peuvent également être obtenus par réaction du composé 20 c) avec le triéthyl ortho formiate au reflux pendant 5 h. Dans ce cas la proportion en composé de l'exemple 21 se trouve légèrement améliorée.

### Exemple 22 : (Méthode D)

### 6-[(2'-cyano biphényl-4-yl) méthyl]-7-hydroxy-5-propyl-1,2,4-triazolo [1,5-a] pyrimidine

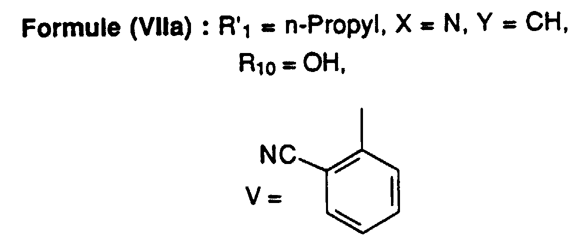

500 mg de 6-[(2'-cyano biphényl-4-yl) méthyl]-5 hydroxy-7-propyl-1,2,4-triazolo [4,3-a] pyrimidine préparés à l'exemple 20d ; sont chauffés dans un bain métallique à 225°C pendant 2 h 30 mn. Laisser refroidir. Reprendre avec du méthanol puis avec de l'acétate d'isopropyle pour obtenir 300 mg de cristaux crème identiques au composé de l'exemple 18.

Point de fusion 200°C.

### Exemple 23 : (Méthode E)

### 6-[(2'-cyano biphényl-4-yl) méthyl]-5-hydroxy-7-propyl-1,2,4-triazolo [1,5-a] pyrimidine

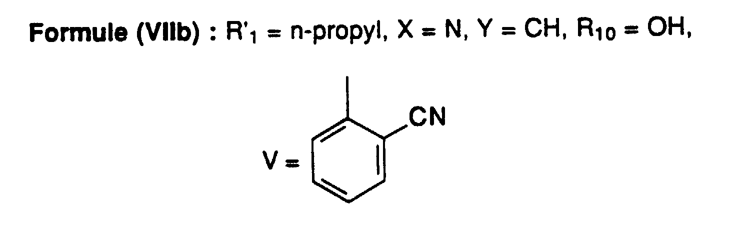

Un mélange de 24 g de 3-amino-1,2,4-triazole et 200 g de 5-éthyl-2-méthyl pyridine est porté à 175°C. Introduire goutte à goutte 100 g de β céto ester préparés à l'exemple 5 en solution dans 100 ml de 5-éthyl-2-méthyl pyridine. Laisser la réaction 6 h à 175°C. Distiller l'éthyl méthyl pyridine sous vide, reprendre le résidu avec un mélange d'eau et de chloroforme. Décanter, extraire la phase aqueuse au chloroforme. Les phases organiques réunies sont lavées avec une solution diluée de HCI puis avec de l'eau. Sécher, concentrer pour obtenir une huile qui cristallise lorsque triturée dans le méthanol. Une recristallisation dans le n-butanol donne 35.2 g de cristaux crème de 6-[(2'-cyano biphényl-4-yl) méthyl]-5-hydroxy-7-propyl-1,2,4-triazolo [1,5-a] pyrimidine.

Point de fusion 210°C.

Une purification des eaux-mères par chromatographie sur gel de silice conduit à un deuxième jet de 6.9 g de composé attendu ainsi qu'à 13.9 g de dérivé 6-[(2'-cyano biphényl-4-yl) méthyl]-7-hydroxy-5-propyl-1,2,4-triazolo [1,5-a] pyrimidine que l'on a obtenu à l'exemple 18.

Point de fusion 196°C.

L'addition de 10.5 g de 4-diméthylamino pyridine dans le mélange initial permet d'améliorer le rendement de la réaction d'environ 10 %.

### Exemple 24 : 6-[(2'-cyano biphényl-4-yl) méthyl]-7-hydroxy-2-méthyl-5-propyl-1,2,4 triazolo [1,5-a] pyrimidine, chlorhydrate, hemihydrate

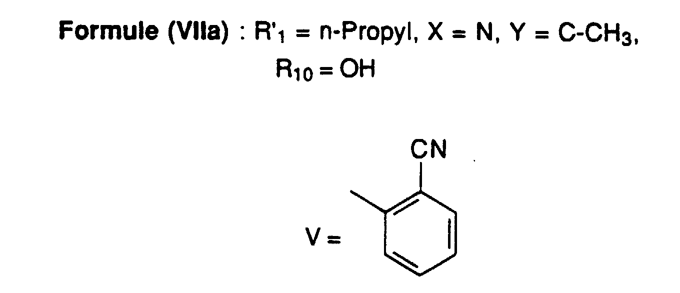

10 g de composé 20c mis en suspension dans 100 ml de phényl acétate sont portés au reflux pendant 4 h. Concentrer sous pression réduite. Reprendre à l'eau, extraire au chloroforme, sécher, évaporer pour obtenir 9.8 g de cristaux blancs de point de fusion 205°C. Ces cristaux repris dans 50 ml d'acétonitrile et 40 ml d'éther chlorhydrique à 10 % conduisent à 7.5 g de chlorhydrate de 6-[(2'-cyano biphényl-4-yl) méthyl]-7-hydroxy-2-méthyl-5-propy-1,2,4 triazolo [1,5-a] pyrimidine.

Formule brute : C₂₃H₂₁N₅O, HCl, 1/2 H₂O

Point de fusion 190°C.

¹H RMN (DMSO-d₆) : 2.65 (t, 2H, CH₂-propyl)
- UV (MeOH) :: λₐ = 213.7 nM
λ_{b} = 257.7 nM
λ_{c} = 285 nM

### Exemple 25 : 6-[(2'-cyano biphényl-4-yl) méthyl]-5-hydroxy 7-propyl-3-mercapto-1,2,4-triazolo [4,3-a] pyrimidine

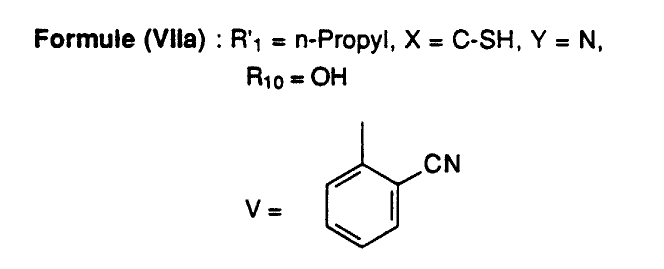

A une suspension de 10 g de composé préparé à l'exemple 20c dans 300 ml de butanol on ajoute 5.3 ml de disulfure de carbone. Porter au reflux 2 h. Rajouter 5.3 ml de CS₂ supplémentaire puis laisser au reflux 5 h. Concentrer sous vide. Reprendre avec de l'eau et extraire 3 fois au chloroforme. Evaporer le solvant pour obtenir 10.8 g de cristaux amorphes que l'on purifie par chromatographie sur gel de silice (éluant CHCl₃ 90 % MeOH 10 %).

Un premier composé de 1.9 g est isolé et identifié comme le 6-[(2'-cyano biphényl-4-yl) méthyl]-7-hydroxy-5-propyl-3-mercapto-1,2,4-triazolo [4,3-a] pyrimidine. Il constitue le produit de **l'exemple 25bis**.

Point de fusion 240°C

¹H RMN (DMSO-d₆) : 3.5 (t, 2H, CH₂ propyl)

Un deuxième composé de 1 g est le produit attendu 6-[(2'-cyano biphényl-4-yl) méthyl]-5-hydroxy-7-propyl-3-mercapto-1,2,4-triazolo [4,3-a] pyridine.

Point de fusion 180°C

¹H RMN (DMSO-d₆) : 2.5 (m, CH₂ propyl + DMSO-d₆)

Le troisième produit de 3.2 g est l'hydrazino 20c de départ.

### Exemple 26: 6-[(2'-cyano biphényl-4-yl) méthyl]-3,5 dihydroxy-7-propyl-1,2,4-triazolo [4,3-a] pyrimidine

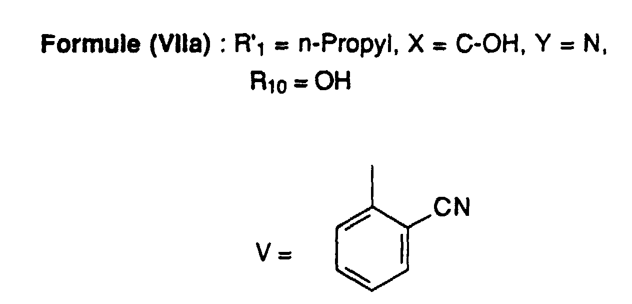

Dans un mélange de 10 g de composé préparé à l'exemple 20c et 500 ml de THF, chauffé à 50°C, ajouter 4.6 g de carbonyl diimidazole. Porter l'ensemble au reflux pendant 7 h. Concentrer sous vide. Reprendre avec de l'eau et extraire trois fois au chloroforme. L'évaporation du solvant conduit à 12.4 g de cristaux amorphes que l'on purifie par chromatographie sur gel de silice (éluant CHCl₃ 95 % MeOH 5 %).

Un premier composé de 3.1 g est isolé et identifié comme le 6-[(2'-cyano biphényl-4-yl) méthyl]-3,7 dihydroxy-5-propyl-1,2,4-triazolo [4,3-a] pyrimidine. Il constitue le produit de **l'exemple 26bis.**

Point de fusion 228°C.

¹H RMN (DMSO-d₆) : 3 (t, 2H, CH₂ propyl)

Le deuxième composé de 3.8 g est le produit attendu 6-[(2'-cyano biphényl-4-yl) méthyl]-3,5 dihydroxy-7-propyl-1,2,4-triazolo [4,3-a] pyrimidine.

Point de fusion 210°C

¹H RMN (DMSO-d₆) : 2.4 (t, 2H, CH₂ propyl) En utilisant l'une des méthodes décrites dans les exemples 19 ou 23 (méthode B ou méthode E), on fait réagir les amino triazoles appropriés sur les β céto esters préparés aux exemples 5 à 15 pour obtenir les composés suivants des exemples 27 à 43.

### Exemple 27 : 6-[(2'-cyano biphényl-4-yl) méthyl]-5-hydroxy-2-méthyl-7-propyl-1,2,4-triazolo [1,5-a] pyrimidine

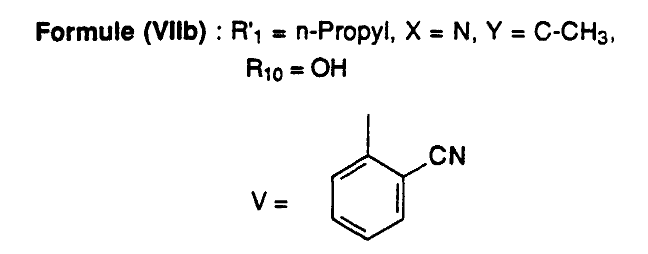

Cristallisation dans le méthanol. Les eaux mères sont purifiées par chromatographie sur gel de silice (éluant CHCl₃ 95 % / MeOH 5 %).

Point de fusion 218-220°C.

Un deuxième composé est isolé et identifié comme le 6-[(2'-cyano biphényl-4-yl) méthyl]-7-hydroxy-2-méthyl-5-propyl-1,2,4-triazolo [1,5-a] pyrimidine. Il constitue le produit de **l'exemple 27bis.**

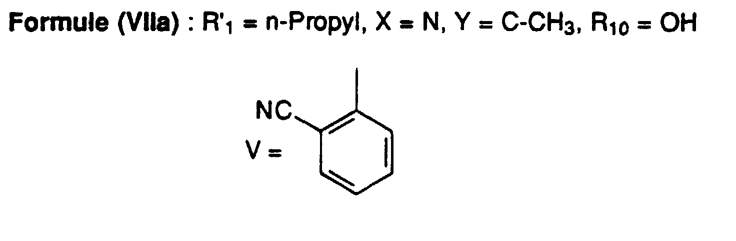

Point de fusion 204-206°C.

### Exemple 28 : 6-[(2'-cyano biphényl-4-yl)méthyl]-2-éthyl-5-hydroxy-7-propyl-1,2,4-triazolo [1,5-a] pyrimidine

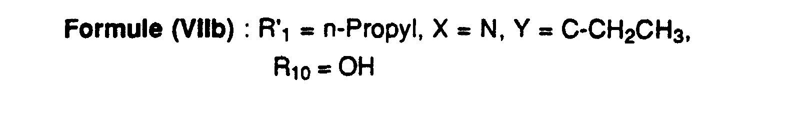

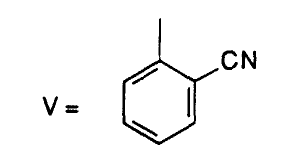

Cristallisation dans le méthanol. Les eaux mères sont purifiées par chromatographie sur gel de silice (éluant CHCl₃ 95 % / MeOH 5 %).

Point de fusion 216°C.

Un deuxième composé est isolé et identifié comme le 6-[(2'-cyano biphényl-4-yl) méthyl]-2-éthyl-7-hydroxy-5-propyl-1,2,4-triazolo [1,5-a] pyrimidine. Il constitue le produit de **l'exemple 28bis.**

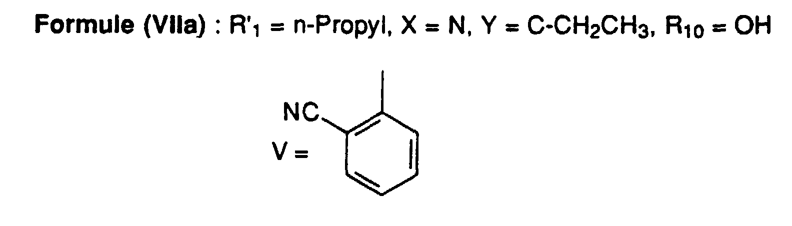

Point de fusion 186°C.

### Exemple 29 : 6-[(2'-cyano biphényl-4-yl)méthyl]-7-n-butyl-5-hydroxy-1,2,4-triazolo [1,5-a] pyrimidine

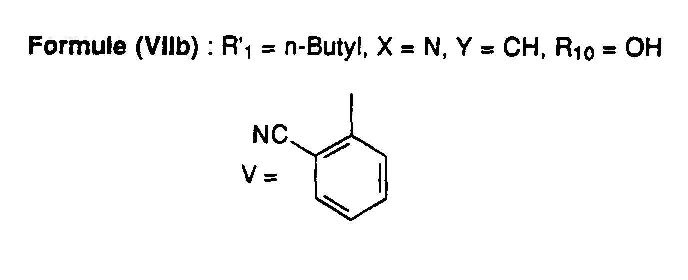

Purifié par recristallisation dans le n-butanol.

Point de fusion 210°C.

### Exemple 30 : 2-amine-6-[(2'-cyano biphényl-4-yl) méthyl]-5 hydroxy-7-propyl-1,2,4-triazolo [1,5-a] pyrimidine

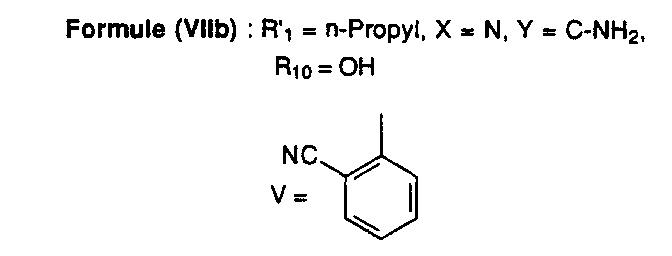

Cristallisation dans un mélange méthanol-chloroforme.

Purification des eaux-mères par chromatographie sur gel de silice (éluant CHCl₃ 90 % / MeOH 10 %).

Point de fusion 260°C.

Un deuxième composé est isolé et identifié comme le 2-amino-6-[(2'-cyano biphényl-4-yl) méthyl]-7-hydroxy-5-propyl-1,2,4-triazolo [1,5-a] pyrimidine. Il constitue le produit de **l'exemple 30bis.**

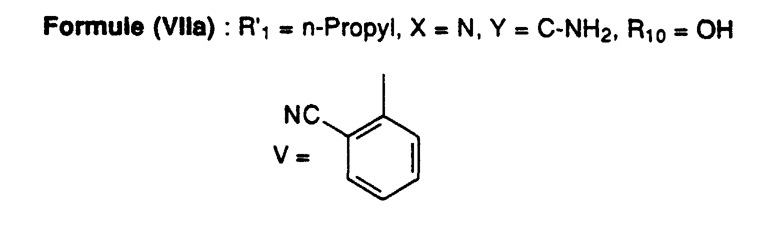

Point de fusion : 325-330°C.

### Exemple 31: 6-[(2'-cyano biphényl-4-yl) méthyl]-5-hydroxy-2-méthylthio-7-propyl-1,2,4-triazolo [1,5-a] pyrimidine

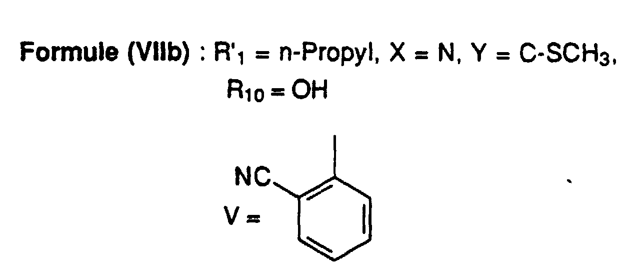

Purification par chromatographie sur gel de silice (éluant CHCl₃ 95 %/ MeOH 5 %).

Cristallisation dans l'acétate d'isopropyle.

Point de fusion 182°C.

### Exemple 32: 6-[4-(3-cyano-2-thiényl) benzyl]-5-hydroxy-7-propyl-1,2,4-triazolo [1,5-a] pyrimidine

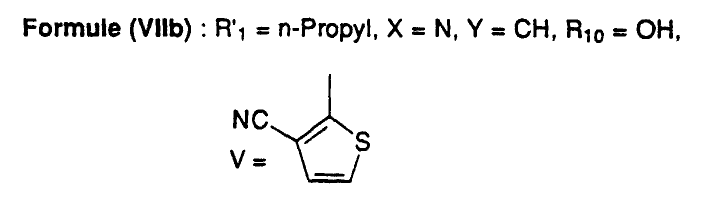

Cristallisation dans chloroforme / eau. Purification par recristallisation dans le méthoxy-2-éthanol.

Point de fusion 246°C.

### Exemple 33: 6-[4-(3-cyano-2-pyridyl) benzyl]-5-hydroxy-7-propyl-1,2,4-triazolo [1,5-a] pyrimidine

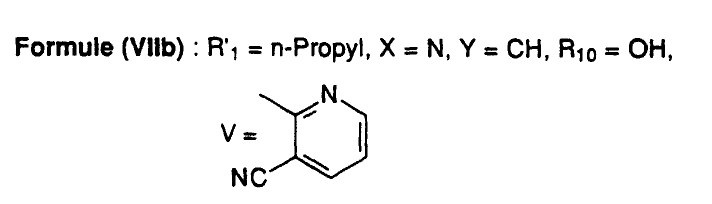

Cristallisation dans le méthanol. Les eaux-mères sont purifiées, par chromatographie sur gel de silice (éluant : CH₂Cl₂ 97.5 % / MeOH 2.5 %). L'ensemble est purifié par recristallisation dans le méthanol.

Point de fusion 212°C.

### Exemple 34: 6-[4-(3-cyano-2-thiényl) benzyl]-5-hydroxy-2-méthyl-7-propyl-1,2,4-triazolo [1,5-a] pyrimidine

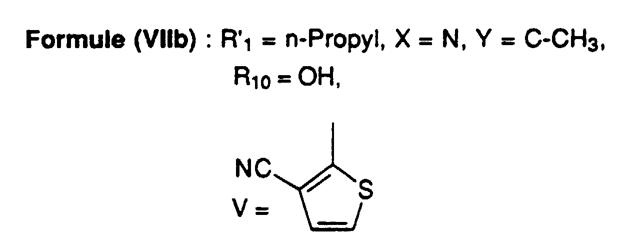

Cristallisation dans un mélange eau / chloroforme. Purification par recristallisation dans le méthoxy-2-éthanol.

Point de fusion 277°C.

### Exemple 35 : 6-[4-(3-cyano-2-furyl) benzyl]-5-hydroxy -7-propyl-1,2,4-trlazolo [1,5-a] pyrimidine

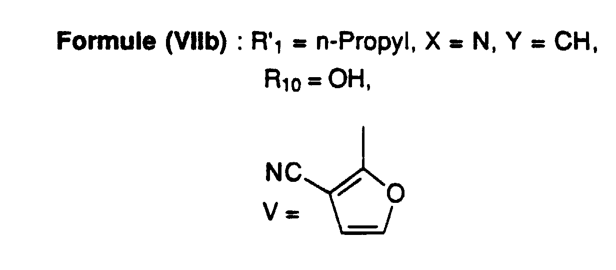

Point de fusion : 256°C.

### Exemple 36 : 7-butyl-6-[(2'-cyano biphényl-4-yl) méthyl]-5-hydroxy-2-méthyl-1,2,4-triazolo [1,5-a] pyrimidine

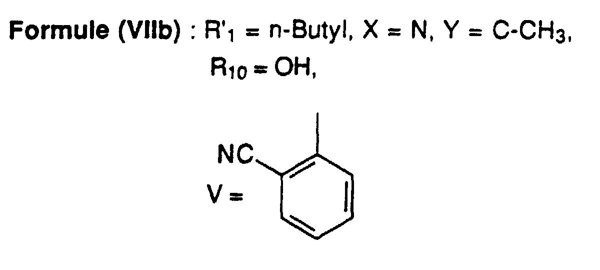

Purifié par recristallisation dans le n-butanol.

Point de fusion 230°C.

### Exemple 37 : 6-[(2'-cyano biphényl-4-yl) méthyl]-5-hydroxy-2-hydroxyméthyl-1,2,4-triazolo [1,5-a] pyrimidine

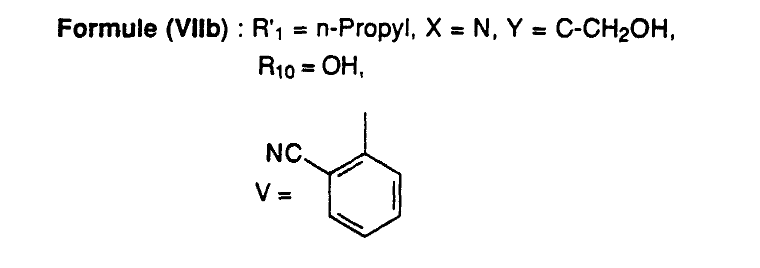

Purification par chromatographie sur gel de silice (éluant CHCl₃ 95 % / MeOH 5 %).

Point de fusion 214°C.

### Exemple 38 : 6-[(2'-cyano biphényl-4-yl) méthyl]-5-hydroxy-7-méthoxyméthyl-1,2,4-triazolo [1,5-a]pyrimidine

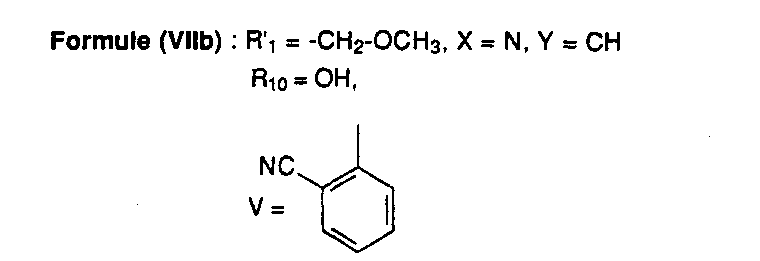

Purifié par chromatographie sur gel de silice (éluant chloroforme 95 % / méthanol 5 %).

Point de fusion 188°C.

Un deuxième composé est isolé et identifié comme le 6-[(2'-cyano biphényl-4-yl) méthyl]-7-hydroxy-5-méthoxyméthyl-1 ,2,4-triazolo [1,5-a] pyrimidine. Il constitue le produit de **l'exemple 38bis.**

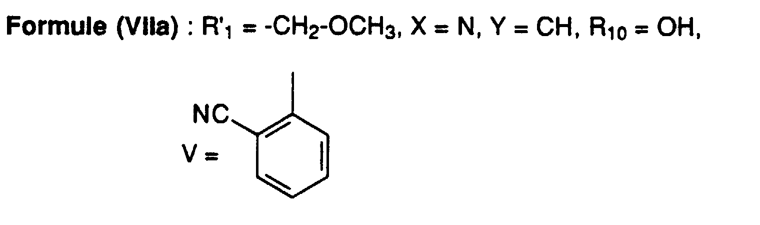

Point de fusion 240°C.

### Exemple 39 : 6-[(2'-cyano biphényl-4-yl) méthyl]-5-hydroxy-7-méthyl-1,2,4-triazolo [1,5-a] pyrimidine

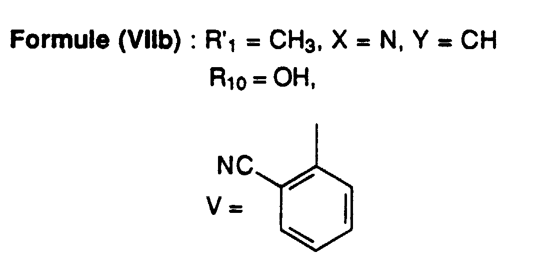

Le produit purifié par chromatographie sur gel de silice (éluant CHCl₃ 95 % / MeOH 5 %) est cristallisé dans le méthanol.

Point de fusion 212°C.

Un deuxième composé est isolé et identifié comme le 6-[(2'-cyano biphényl-4-yl) méthyl]-7-hydroxy-5-méthyl-1,2,4-triazolo [1,5-a] pyrimidine. Il constitue le produit de **l'exemple 39bis**.

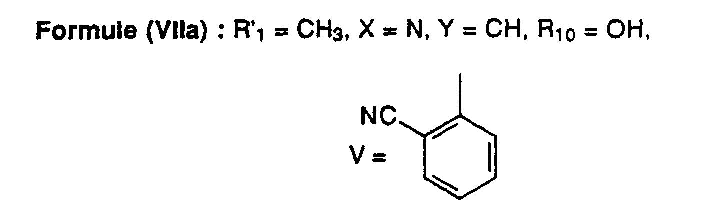

Point de fusion 252°C.

### Exemple 40 : 6-[(2'-cyano biphényl-4-yl) méthyl]-7-éthyl-5-hydroxy-1,2,4-triazolo [1,5-a] pyrimidine

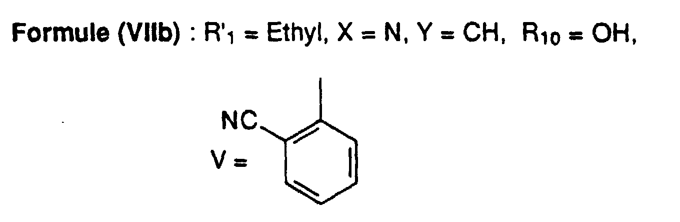

Isolé par chromatographie sur gel de silice (éluant CHCl₃ 95 % / MeOH 5 %) et purifié par recristallisation dans le n-butanol.

Point de fusion 224°C.

Un deuxième composé est isolé et identifié comme le 6-[(2'-cyano biphényl-4-yl) méthyl]-5-éthyl-7-hydroxy-1,2,4-triazolo [1,5-a] pyrimidine. Il constitue le produit de **l'exemple 40bis.**

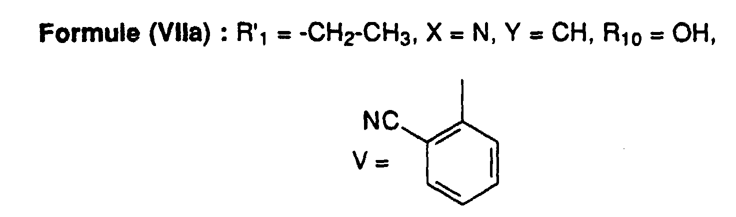

Point de fusion 234°C.

### Exemple 41: 6-[(2'-cyano biphényl-4-yl) méthyl]-2-N,N-diéthyl amino-5-hydroxy-7-propyl-1,2,4-triazolo [1,5-a] pyrimidine

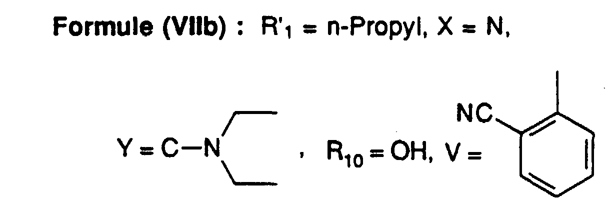

Le produit est cristallisé dans le méthanol.

Point de fusion 220°C.

Un deuxième composé est isolé sous forme de cristaux amorphes, après chromatographie des eaux mères sur gel de silice (éluant CHCl₃ 80 % / isopropylamine 20 %). Il est identifié comme étant le 6-[(2'-cyano biphényl-4-yl) méthyl]-2-N,N-diéthyl amino-7-hydroxy-5-propyl-1,2,4-triazolo [1,5-a] pyrimidine. Il constitue le composé de **l'exemple 41bis.**

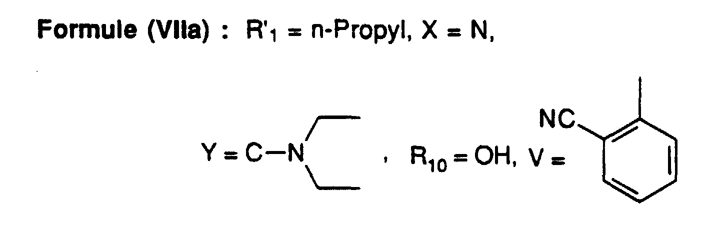

### Exemple 42 : 6-[(2'-cyano biphényl-4-yl) méthyl)-5,7-dipropyl -1,2,4-triazolo [1,5-a] pyrimidine

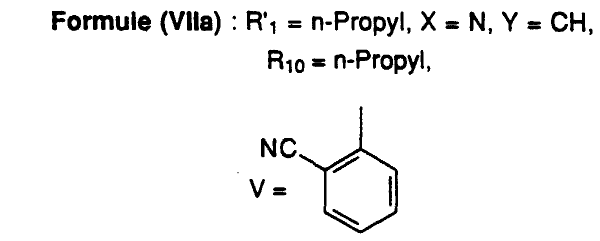

Produit purifié par chromatographie sur gel de silice (éluant chloroforme 95 % / méthanol 5 %).

Point de fusion 160°C.

### Exemple 43 : 7-benzyloxy méthyl-6-[(2'-cyano biphényl-4-yl) méthyl]-5-hydroxy-1,2,4-triazolo [1,5-a] pyrimidine

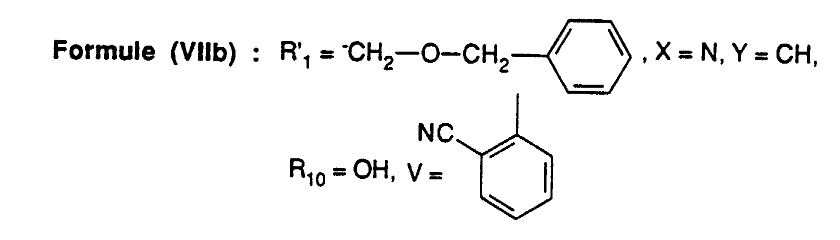

Purifié par recristallisation dans le butanol suivi d'une chromatographie sur gel de silice (éluant chloroforme 95 % / méthanol 5 %).

Point de fusion 218°C.

Un deuxième composé est isolé et identifié comme étant le 5-benzyloxy méthyl-6-[(2'-cyano biphényl-4-yl) méthyl]-7-hydroxy-1,2,4-triazolo [1,5-a] pyrimidine. Il constitue le produit de **l'exemple 43bis.**

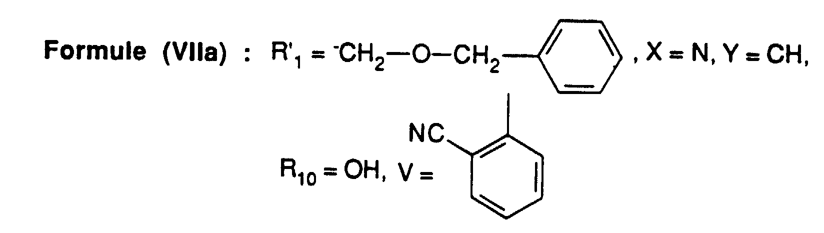

Point de fusion 260°C.

### Exemple 44 : 5-chloro-6-[(2'-cyano biphényl-4-yl)]-7-propyl 1,2,4-triazolo [1,5-a] pyrimidine

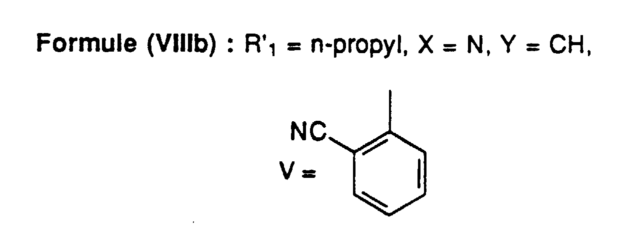

On ajoute par portion, 25.9 g de composé préparé à l'exemple 19 ou 23 dans 260 ml de POCl₃. Le mélange est porté au reflux pendant 4 h. Concentrer sous vide et reprendre avec 200 ml de chloroforme stabilisé à l'amylène puis ajouter une solution d'eau et de glace. Décanter, extraire au chloroforme, réunir les phases organiques. Après lavage à l'eau et séchage, on concentre sous vide pour obtenir une huile épaisse. Le produit est cristallisé dans l'acétate d'isopropyle pour donner 21 g de 5-chloro-6-[(2'-cyano biphényl-4-yl)]-7-propyl -1,2,4-triazolo [1,5-a] pyrimidine.

Point de fusion 138°C. Selon le mode opératoire de l'exemple 44 mais en utilisant le dérivé préparé à l'exemple 18, on obtient le dérivé de l'exemple 45.

### Exemple 45 : 7-chloro-6-[(2'-cyano biphényl-4-yl) méthyl]-5-propyl-1,2,4-triazolo [1,5-a] pyrimidine

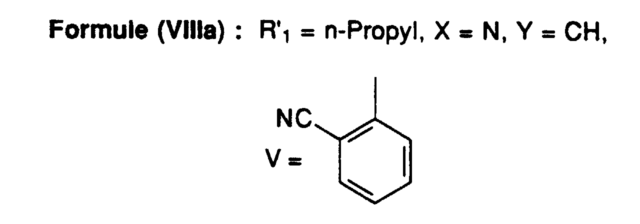

Point de fusion 132°C.

### Exemple 46: 6-[(2'-cyano biphényl-4-yl) méthyl]-7-mercapto-5-propyl-1,2,4-triazolo [1,5-a] pyrimidine

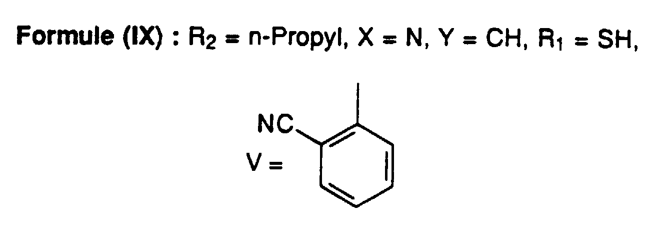

On porte au reflux pendant 7 h, un mélange de 5 g du composé chloré obtenu à l'exemple 45, 2 g de thiourée et 150 ml d'éthanol. Concentrer sous vide. Le solide jaune est repris avec 60 ml d'une solution de NaOH 0.5 N. Filtrer le léger insoluble. Acidifier le filtrat avec de l'acide acétique. Le précipité jaune est filtré, purifié par chromatographie sur gel de silice (éluant chloroforme 90 % / méthanol 10 %) pour donner 3.4 g de 6-[(2'-cyano biphényl-4-yl) méthyl]-7-mercapto-5-propyl-1,2,4-triazolo [1,5-a] pyrimidine.

Point de fusion 200-205°C.

### Exemple 47 : 6-[(2'-cyano biphényl-4-yl) méthyl]-5-mercapto-7-propyl-1,2,4-triazolo [1,5-a] pyrimidine

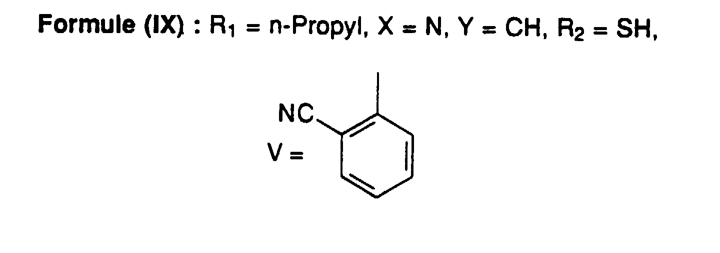

On porte au reflux, pendant 2 h, un mélange de 11.1 g de dérivé préparé à l'exemple 19 ou à l'exemple 23, 350 ml de toluène et 13.4 g de réactif de Lawesson. Filtrer le solide jaune obtenu. Une purification par chromatographie sur gel de silice (éluant CH₂Cl₂ 90 % / acétone 10 %) conduit à 10 g de 6-[(2'-cyano biphényl-4-yl) méthyl]-5-mercapto-7- propyl-1,2,4-triazolo [1,5-a] pyrimidine.

Point de fusion 226°C.

### Exemple 48 : 6-[(2'-cyano biphényl-4-yl) méthyl]-7-propyl-1,2,4-triazolo [1,5-a] pyrimidine

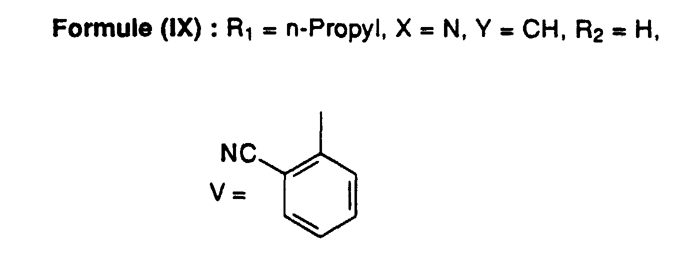

Hydrogéner, à pression et à température ambiante, en présence de 1.4 g de Pd sur charbon à 5 %, 5.4 g de composé préparé à l'exemple 44, en solution dans 110 ml de méthoxy-2-éthanol contenant 1.2 g d'acétate de sodium anhydre. Purger avec de l'azote. Essorer sur célite 545. Laver avec du méthoxy-2-éthanol chaud. Le filtrat est concentré, les cristaux obtenus repris à l'éther pour donner 3.7 g de produit brut. Une purification par chromatographie sur gel de silice (éluant dichlorométhane 90 % / acétone 10 %) conduit à 2.5 g de cristaux blancs de 6-[(2'-cyano biphényl-4-yl) méthyl]-7-propyl-1,2,4- triazolo [1,5-a] pyrimidine.

Point de fusion 180°C.

### Exemple 49 : 6-[(2'-cyano biphényl-4-yl) méthyl]-5-méthoxy 7-propyl-1,2,4-triazolo [1,5-a] pyrimidine

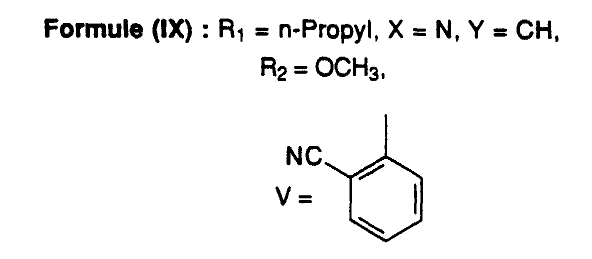

Une solution de méthylate de sodium, préparée à partir de 0.8 g de sodium et 25 ml de méthanol, est additionnée à 11.6 g de composé de l'exemple 44 en solution dans 120 ml de diméthoxy-1,2,-éthane. Le mélange est agité à température ambiante pendant 3 h. Essorer l'insoluble, concentrer le filtrat. Les cristaux obtenus sont repris à l'eau, filtrés et lavés d'abord à l'eau puis à l'alcool isopropylique et à l'éther pour donner 9.5 g de 6-[(2'-cyano biphényl-4-yl) méthyl]-5-méthoxy-7-propyl-1,2,4-triazolo [1,5-a] pyrimidine.

Point de fusion 166°C.

### Exemple 50 : Ethyl [6-[(2'-cyano biphényl-4-yl) méthyl]-7-propyl-1,2,4-triazolo [1,5a] pyrimidin-5-yl]mercapto acétate

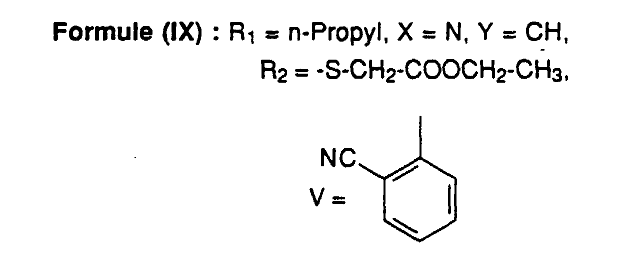

Ajouter, par portions, 0.6 g de NaH à 60 % à 1.8 g de mercapto acétate d'éthyle en solution dans 50 ml de toluène. La température est maintenue à 40° pendant 1/2 h puis ramenée à l'ambiante. Introduire alors 5 g de composé préparé à l'exemple 44, en solution dans 50 ml de toluène anhydre. Laisser réagir à température ambiante pendant 3 h puis 4 h à 50°C. Un deuxième équivalent de sel de sodium de mercapto acétate d'éthyle préparé comme ci-dessus est rajouté pour compléter la réaction. Après hydrolyse, décanter, laver la phase organique avec de l'eau puis une solution d'acide acétique diluée, sécher et concentrer. L'huile obtenue est purifiée par chromatographie sur gel de silice (éluant dichlorométhane 90 % / acétone 10 %) pour conduire à 5.4 g d'éthyl [6-[(2'cyano biphényl-4-yl) méthyl]-7-propyl-1,2,4-triazolo [1,5-a] pyrimidin-5-yl]mercapto acétate.

Point de fusion 76°C.

Selon le mode opératoire de l'exemple 50, mais en utilisant le méthoxy-2 éthanol au lieu du mercapto acétate d'éthyle, on obtient le composé de l'exemple 51.

### Exemple 51 : [6-[(2'-cyano biphényl-4-yl) méthyl]-7-propyl-1,2,4-triazolo [1,5-a] pyrimidin-5-yl]-2-méthoxy éthyl éther

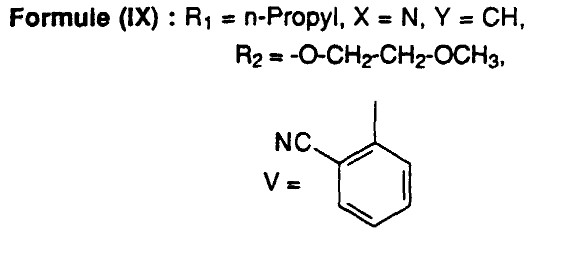

Produit cristallisé dans l'éther isopropylique.

Point de fusion 102°C.

### Exemple 52 : 5-amino-6-[(2'-cyano biphényl-4-yl) méthyl]-7-propyl-1,2,4-triazolo [1,5-a] pyrimidine

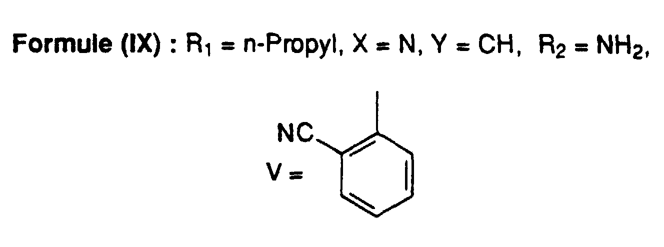

Dans un autoclave, on place, un mélange de 10 g de dérivé préparé à l'exemple 44 et 200 ml d'une solution de diméthoxy-1,2-éthane, saturée avec de l'ammoniac. Chauffer à 125°C pendant 24 h. Reprendre avec un mélange chloroforme-eau. Décanter, extraire la phase aqueuse. Les phases organiques sont réunies, séchées et concentrées pour donner 8.1 g de 5-amino-6-[(2'-cyano biphényl-4-yl) méthyl]-7-propyl-1,2,4-triazolo [1,5a] pyrimidine.

Point de fusion 206°C.

### Exemple 53 6-[(2'-cyano biphényl-4-yl) méthyl]-7-N,N-diéthyl amino-5-propyl-1,2,4-triazolo [1,5-a] pyrimidine

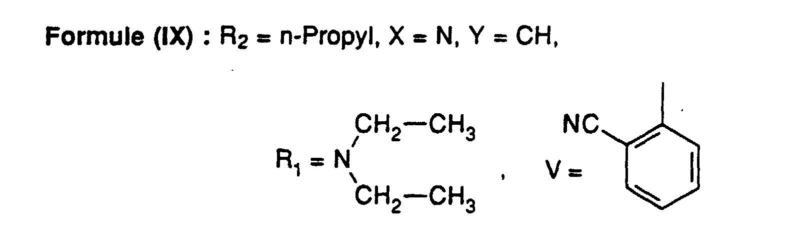

On porte au reflux pendant 4 h un mélange de 5 g du dérivé chloré de l'exemple 45, 100 ml d'éthanol, 16 ml de diéthylamine et 1.5 g de carbonate de sodium. Concentrer sous vide et reprendre l'huile épaisse à l'eau. Extraire trois fois avec du dichlorométhane, sécher, concentrer. Le composé obtenu est purifié par chromatographie sur gel de silice (éluant chloroforme 95 % / méthanol 5 %) pour donner 5 g de 6-[(2'-cyano biphényl-4-yl) méthyl]-7-N,N-diéthyl amino-5-propyl-1,2,4-triazolo [1,5-a] pyrimidine sous forme d'huile orangée.
En faisant réagir avec des amines appropriées et selon l'une quelconque des deux méthodes, décrites dans les exemples 52 et 53, l'un des dérivés décrits aux exemples 44 ou 45, on obtient les composés suivants des exemples 54 à 58.

### Exemple 54 6-[(2'-cyano biphényl-4-yl) méthyl]-5-N,N-diéthyl amino-7-propyl-1,2,4-triazolo [1,5-a] pyrimidine

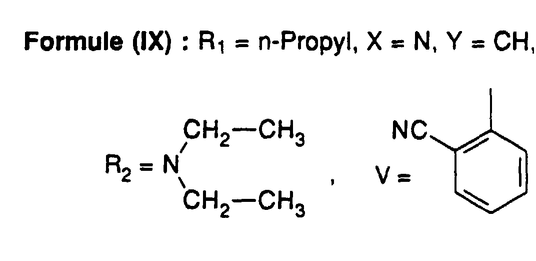

Produit cristallisé dans l'éther isopropylique à chaud.

Point de fusion 133°C.

### Exemple 55 6-[(2'-cyano biphényl-4-yl) méthyl]-7-propyl-5-(pyrrolidin-1-yl)-1,2,4-trlazolo [1,5-a] pyrimidine

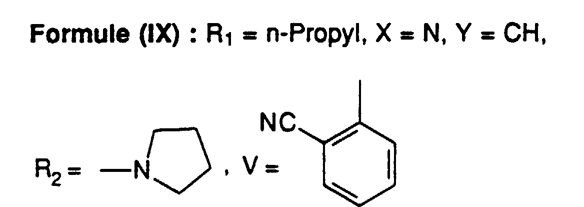

Produit cristallisé dans l'éther isopropylique à chaud.

Point de fusion 166°C.

### Exemple 56 6-[(2'-cyano biphényl-4-yl) méthyl-7-propyl-5-(morpholin-4-yl éthyl amino)-1,2,4-triazolo [1,5-a] pyrimidine

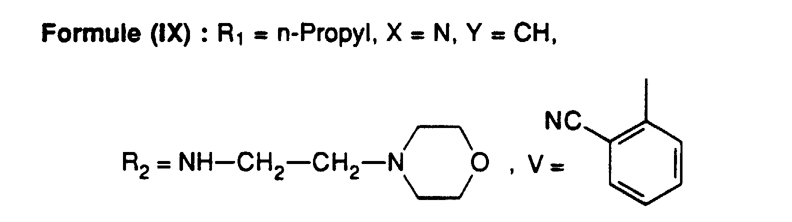

Produit huileux, purifié par chromatographie sur gel de silice (éluant chloroforme 95 % / méthanol 5 %).

### Exemple 57 6-[(2'-cyano biphényl-4-yl) méthyl]-5-(piperidin-1-yl)-7-propyl-1,2,4-triazolo [1,5-a] pyrimidine

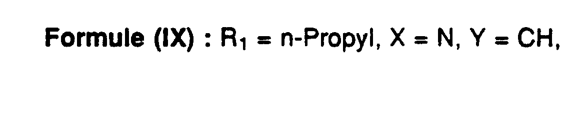

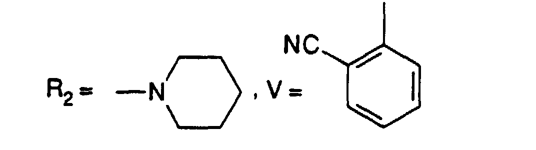

Composé purifié par recristallisation dans le méthoxy-2 éthanol. Point de fusion 266°C.

### Exemple 58 6-[(2'-cyano biphényl-4-yl) méthyl]-5-hydrazino-7-propyl-1,2,4-triazolo [1,5-a] pyrimidine

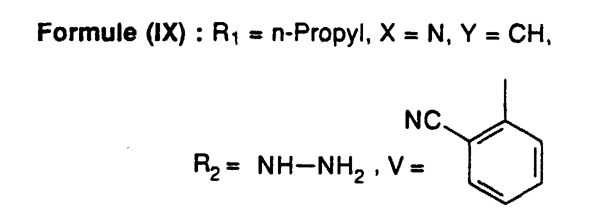

Produit cristallisé dans l'éther.

Point de fusion 161°C.

### Exemple 59 5-azido-6-[(2'-cyano biphényl-4-yl) méthyl]-7-propyl-1,2,4-triazolo [1,5-a] pyrimidine

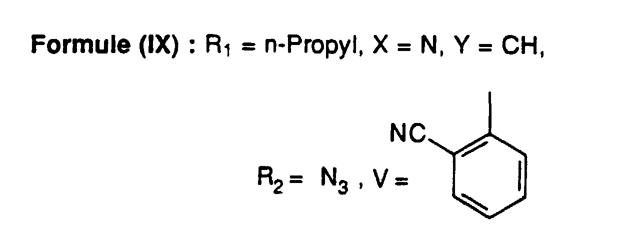

On mélange 10.3 g de composé préparé à l'exemple 58, 2.3 ml de HCI concentré et 300 ml d'acide acétique. Rajouter 1.9 g de NaNO₂ en solution dans 20 ml d'eau. Laisser une nuit à température ambiante. Rajouter de l'eau, décanter et extraire à l'acétate d'éthyle. Les phases organiques sont réunies, lavées à l'eau, séchées et évaporées. Une purification par deux chromatographies successives (éluant dichlorométhane 95 % / méthanol 5 %) et dichlorométhane 90 % / méthanol 10 %) permet d'obtenir 4.3 g de 5-azido-6-[(2'-cyano biphényl-4-yl) méthyl]-7- propyl-1,2,4-triazolo [1,5-a] pyrimidine.

Point de fusion 134°C.
Ce même composé peut être considéré comme un dérivé tricyclique selon l'équilibre connu des azides en position 2 des cycles azotés (cf. Temple and Montgomery, J. Org. Chem. 30, 826 (1965).

### Exemple 60 : 3-amino-5-hydroxyméthyl-1,2,4-triazole

**Formule (II) :** R₇ = -CH₂OH

Un mélange de 136 g de bicarbonate d'aminoguanidine et 80 g d'acide glycolique est porté progressivement à 120°C. La réaction est maintenue 5 h à cette température. Reprendre avec 100 ml d'éthanol, filtrer le solide pour obtenir 45.7 g de 3-amino-5-hydroxyméthyl-1,2,4-triazole.

Point de fusion 192-194°C.

### Exemple 61: 3-amino-5-N,N-diethylamino-1,2,4-triazole

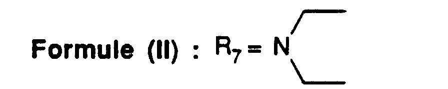

10.3 ml de diéthylamine sont additionnés à 16.1 g de diméthyl N-cyano dithioimino carbonate en solution dans 160 ml d'acétonitrile. Agiter une heure à température ambiante puis porter au reflux jusqu'à ce qu'il n'y ait plus de dégagement de méthylmercaptan. Refroidir la solution à l'aide d'un bain de glace, introduire 5 ml d'hydrate d'hydrazine. Porter le mélange au reflux pendant 4 h. Après distillation du solvant, le produit est repris avec de l'acétonitrile pour conduire à 8.9 g de cristaux blancs de 3-amino-5-N,N-diéthylamino-1,2,4-triazole.

Point de fusion 134°C.

### Exemple 62 : 7-hydroxy-5-propyl-6-[(2'-(1-H-tétrazol-5-yl) biphényl-4-yl) méthyl]-1,2,4-triazolo [1,5-a]pyrimidine

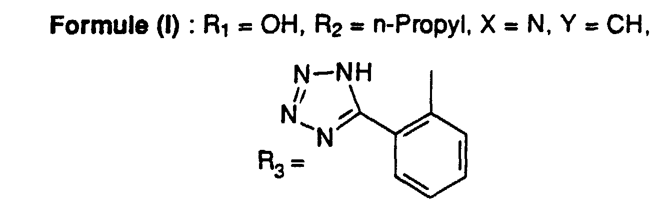

Un mélange de 7.8 g de β céto ester de l'exemple 17, 1.7 g de 3-amino 1,2,4-triazole et 70 ml de 1,2,4-trichlorobenzène est chauffé à 120°C pendant 7 h. Le précipité obtenu est purifié par chromatographie sur gel de silice (éluant CH₂Cl₂ 80 % / Méthanol 20 %). Le composé obtenu est dissout dans une solution de NaOH 1N, l'insoluble filtré et la solution limpide acidifiée par barbottage de SO₂ pour donner 2.4 g d'un précipité blanc de 7-hydroxy-5-propyl-6-[(2'-(1-H-tétrazol-5-yl) biphényl-4-yl) méthyl]-1,2,4-triazolo [1,5-a] pyrimidine.

Formule brute : C₂₂H₂₀N₈O, 0.5 H₂O

Point de fusion : 260-265°C avec décomposition

¹H RMN (DMSO-d₆) : 2.6 (t, 2H, CH₂ Propyl) ; 8.2 (s, 1H, H₂)
- UV (MeOH) :: λₐ = 210 nM
λ_{β} = 250 nM

### Exemple 63 : 5-hydroxy-7-propyl-6-[(2'-(1-H-tétrazol-5-yl) biphényl-4-yl) méthyl]-1,2,4-triazolo [1,5-a] pyrimidine

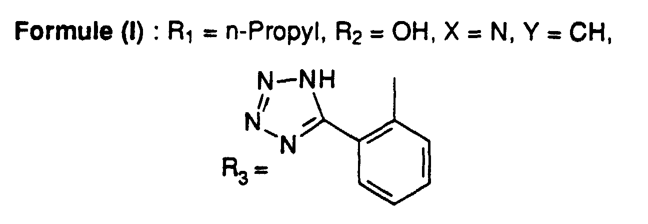

On porte au reflux pendant 50 h, un mélange de 25 g du composé obtenu selon l'exemple 19 ou 23, 750 ml de xylène et 34.5 g d'azoture de triméthyl étain. Le précipité blanc obtenu est filtré. Point de fusion 290°C avec décomposition. Ce composé est mis en suspension dans 500 ml de THF. On fait barbotter du gaz chlorhydrique pendant 30 min pour obtenir une dissolution complète. Concentrer alors sous vide. Reprendre à l'eau, triturer. La gomme obtenue est cristallisée dans de l'acétonitrile. Une recristallisation dans l'isopropanol conduit à 15.2 g du dérivé attendu.

Point de fusion 242°C.
Concentrer les eaux-mères. Basifier avec une solution 1N de KOH, extraire au chloroforme, puis neutraliser avec de l'acide acétique. Le précipité obtenu est recristallisé deux fois dans l'isopropanol pour donner 4.5 g d'un deuxième jet du composé 5-hydroxy-7-propyl-6-[(2'-(1-H-tétrazol-5-yl) biphényl-4-yl) méthyl]-1,2,4-triazolo [1,5-a] pyrimidine.

Formule brute : C₂₂H₂₀N₈O

Point de fusion : 242-244°C

¹H RMN (DMSO-d₆) : 2.91 (t, 2H, CH₂ Propyl) ; 8.11 (s, 1H, H₂) Selon l'une ou l'autre des modes opératoires décrits aux exemples 62 et 63, les composés suivants des exemples 64 à 95 ont été préparés.

### Exemple 64 : 7-hydroxy-2-méthyl-5-propyl-6-[(2'-(1-H-tétrazol-5-yl) biphényl-4-yl) méthyl] -1,2,4-triazolo [1,5-a] pyrimidine, hémisulfate

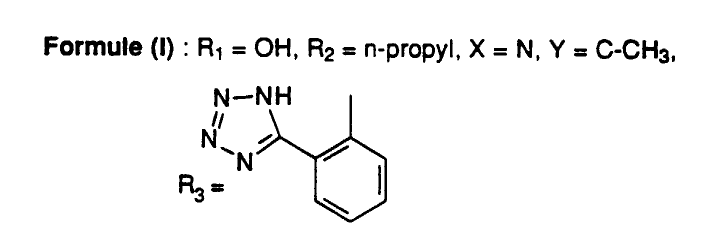

Formule brute : C₂₃ H₂₂ N₈O, 0.5 H₂SO₄

Point de fusion : 236°-238°C

¹H RMN (DMSO-d₆) : 2.6 (t,2H, CH₂ propyl)
- UV (MeOH) :: λₐ = 212.1 nM
λ_{b} = 250 nM

### Exemple 65 : 5-hydroxy-7-propyl-6-[(2'-(1-H-tétrazol-5-yl) biphényl-4-yl) méthyl]-1,2,4 triazolo [4,3-a] pyrimidine

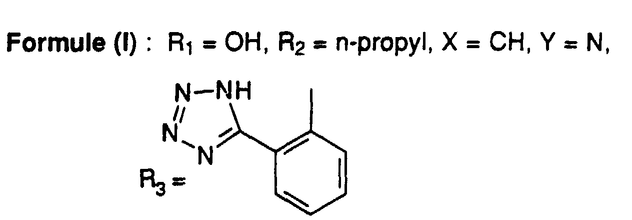

Formule brute : C₂₂ H₂₀ N₈O

Point de fusion 251°C

¹H RMN (DMSO-d₆) : 2.55 (t, 2H, CH₂ propyl) ; 9 (s, 1H, H₃)

### Exemple 66 : 5-propyl-7-mercapto 6-[(2'-(1-H-tétrazol-5-yl) biphényl-4-yl) méthyl]-1,2,4-triazolo [1,5-a] pyrimidine

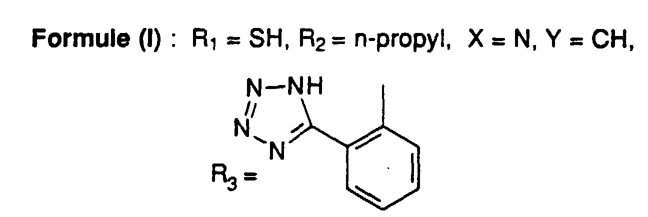

Formule brute : C₂₂ H₂₀ N₈ S

Point de fusion : 288°C

¹H RMN (DMSO-d₆) : 2.59 (t, 2H, CH₂ n-propyl) ; 8.6 (s, 1H, H₂)

### Exemple 67 : 5,7-diméthyl-6-[(2'-(1-H-tétrazol-5-yl) biphényl -4-yl) méthyl]-1,2,4-triazolo [1,5-a] pyrimidine

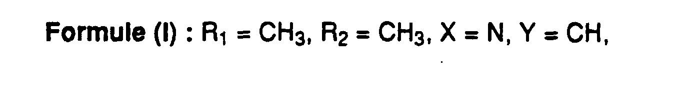

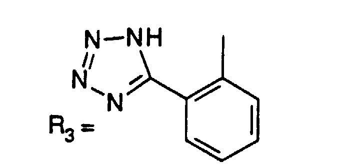

Ce composé a été obtenu selon le mode opératoire de l'exemple 62 mais en utilisant le 2,4-dioxo-3-[(2'-(1-H-tétrazol-5-yl) biphényl-4-yl) méthyl] pentane préparé à l'exemple 16.

Formule brute : C₂₁ H₁₈ N₈

Point de fusion : 264°C

¹H RMN (DMSO-d₆) : 2.48 (s, 3H, CH₃) ; 2.81 (s, 3H, CH₃) ; 8.56 (s, 1H, H₂)

### Exemple 68 : 2-éthyl-7-hydroxy-5-propyl-6-[(2'-(1-H-tétrazol-5-yl) biphényl-4-yl) méthyl]-1,2,4-triazolo [1,5-a] pyrimidine

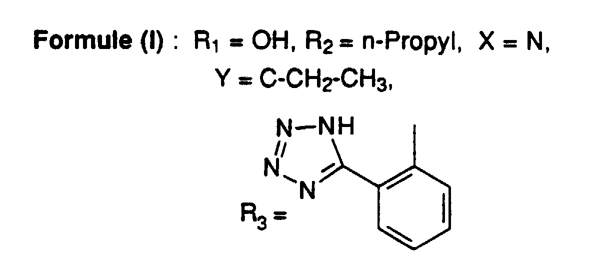

Formule brute : C₂₄ H₂₄ N₈ O

Point de fusion : 246°C

¹H RMN (DMSO-d₆) : 2.57 (m, 2H, CH₂ propyl + DMSO-d₆)

### Exemple 69 : 7-N,N-diethylamino-5-propyl-6-[(2'-(1-H-tétrazol-5-yl) biphényl-4-yl) méthyl]-1,2,4-triazolo [1,5-a] pyrimidine

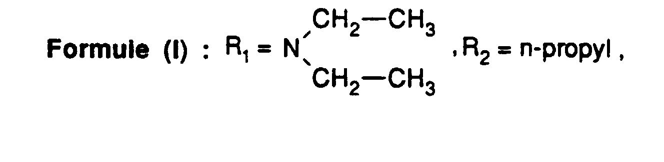

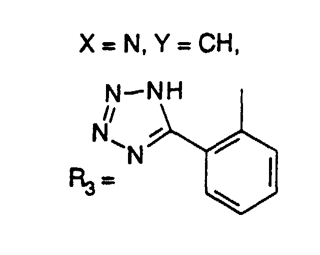

Formule brute : C₂₅ H₂₉ N₅

Point de fusion : 192°C

¹H RMN (DMSO-d₆) : 2.65 (t, 2H, CH₂ n-propyl) ; 8.5 (s, 1H, H₂)

### Exemple 70 : 5-azido-7-propyl-6-[(2'-(1-H-tétrazol-5-yl) biphényl-4-yl) méthyl]-1,2,4-triazolo [1,5-a] pyrimidine

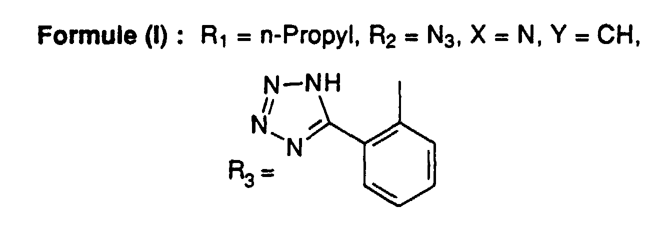

Formule brute : C₂₂ H₁₉ N₁₁

Point de fusion : 212-213°C

¹H RMN (DMSO-d₆) : 3.17 (t, 2H, CH₂ n-propyl) ; 4.06 (s, 2H, CH₂ benzyl équilibre azido - tétrazole ~ 10 %) ; 4.47 (s, 2H, CH₂ benzyl) ; 8.56 (s, 1H, H₂ équilibre azido - tétrazole ~ 10 %) ; 8.7 (s, 1H, H₂)

### Exemple 71: 3,5-dihydroxy-5-propyl-6-[(2'-(1-H-tétrazol-5-yl) biphényl-4-yl) méthyl]-1,2,4-triazolo [4,3-a] pyrimidine

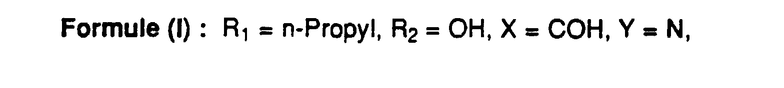

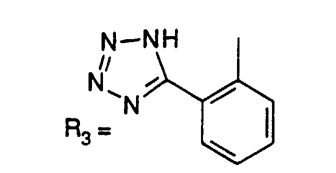

Formule brute : C₂₂ H₂₀ N₈ O₂

Point de fusion : 252°C

¹H RMN (DMSO-d₆) : 2.93 (t, 2H, CH₂ n-propyl) ; 3.7 (s, 2H, CH₂ benzyl)

### Exemple 72: 5-hydroxy-2-méthyl-7-propyl-6-[(2'-(1-H-tétrazol-5-yl) biphényl-4-yl) méthyl]-1,2,4-triazolo [4,3-a] pyrimidine

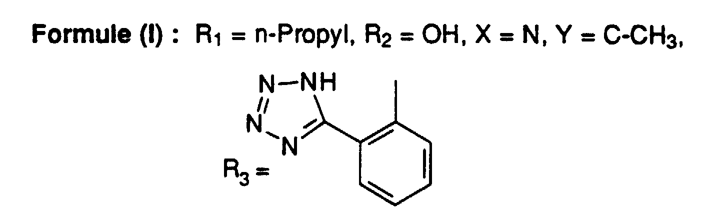

Formule brute : C₂₃ H₂₂ N₈ O

Point de fusion : 286°C

¹H RMN (DMSO-d₆) : 2.85 (t, 2H, CH₂ n-propyl) ; 3.84 (s, 2H, CH₂ benzyl)

### Exemple 73 : 2-éthyl-5-hydroxy-7-propyl-6-[(2'-(1-H-tétrazol-5-yl) biphényl-4-yl) méthyl]-1,2,4-triazolo [4,3-a] pyrimidine

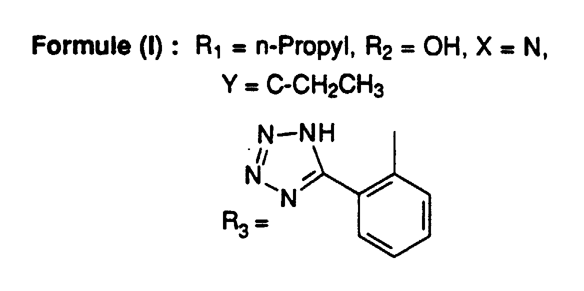

Formule brute : C₂₄ H₂₄ N₈ O

Point de fusion : 260°C

¹H RMN (DMSO-d₆) : 2.86 (t, 2H, CH₂ n-propyl) ; 3.85 (s, 2H, CH₂ benzyl)

### Exemple 74 : 7-butyl-5-hydroxy-6-[(2'-(1 -H-tétrazol-5-yl) biphényl-4-yl) méthyl]-1,2,4-triazolo [1,5-a] pyrimidine

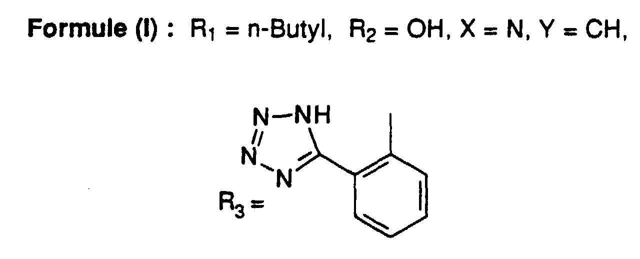

Formule brute : C₂₃ H₂₂ N₈ O

Point de fusion : 255°C

¹H RMN (DMSO-d₆) 2.92 (t, 2H, CH₂ n-propyl) ; 3.86 (s, 2H, CH₂ benzyl) ; 8.11 (s, 1H, H₂)

### Exemple 75 : 2-amino-5-hydroxy-7-propyl-6-[(2'-(1-H-tétrazol-5-yl) biphényl-4-yl) méthyl]-1,2,4-triazolo [1,5-a] pyrimidine

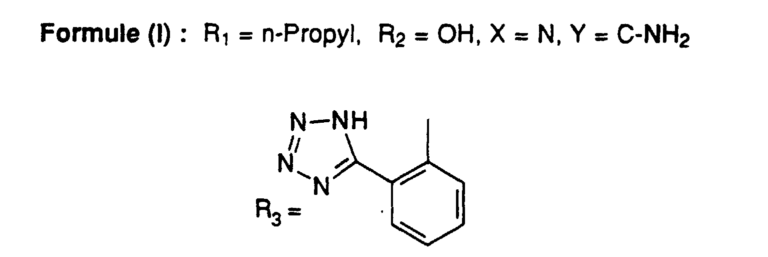

Formule brute : C₂₂ H₂₁ N₉ O

Point de fusion : 282°C

¹H RMN (DMSO-d₆) : 2.76 (t, 2H, CH₂ n-propyl) 3.8 (s, 2H, CH₂ benzyl)

### Exemple 76 : 5-N,N-diéthylamino-7-propyl-6-[(2'-(1-H-tétrazol-5-yl) biphényl-4-yl) méthyl]-1,2,4-triazolo [1,5-a] pyrimidine

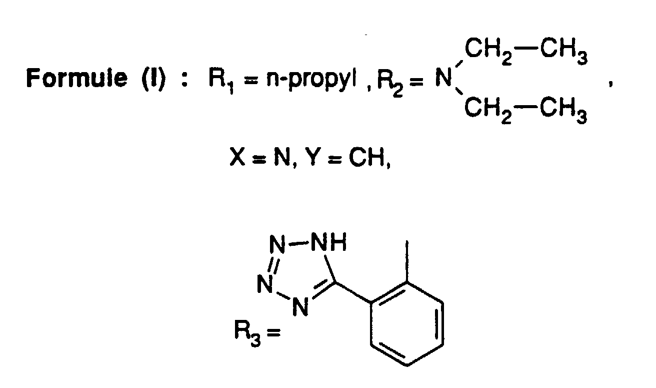

Formule brute : C₂₆ H₂₉ N₉

Point de fusion : 140°C puis 205°C

¹H RMN (DMSO-d₆) : 2.91 (t, 2H, CH₂ propyl) ; 4.07 (s, 2H, CH₂ benzyl) ; 8.32 (s, 1H, H₂)

### Exemple 77 : 5-amino-7-propyl-6-[(2'-(1-H-tétrazol-5-yl) biphényl-4-yl) méthyl]-1,2,4-triazolo [1,5-a] pyrimidine

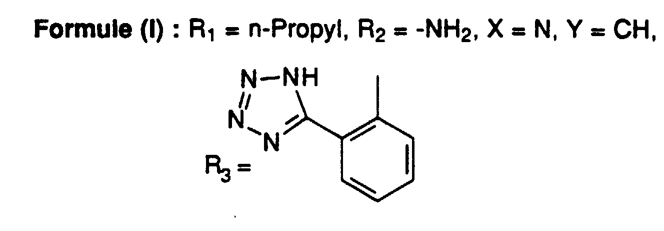

Formule brute : C₂₂ H₂₁ N₉

Point de fusion : 276°C

¹H RMN (DMSO-d₆) : 2.98 (t, 2H, CH₂ propyl) ; 4.03 (s, 2H, CH₂ benzyl) ; 8.1 (s, 1H, H₂)

### Exemple 78: 5-hydroxy-2-mercaptométhyl-7-propyl-6-[(2'-(1-H-tétrazol-5- yl) biphényl-4-yl) méthyl]-1,2,4-triazolo [1,5-a] pyrimidine

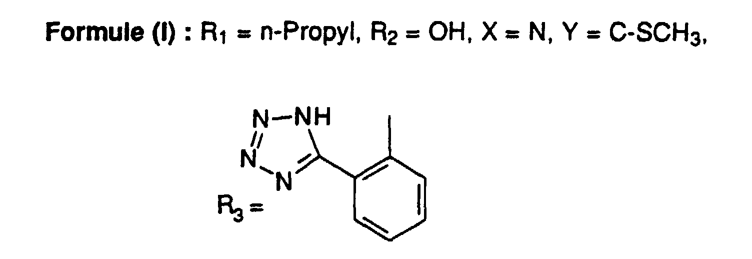

Formule brute : C₂₃ H₂₂ N₈OS

Point de fusion : 260°C

¹H RMN (DMSO-d₆) : 2.85 (t, 2H, CH₂ n-propyl) ; 3.84 (s, 2H, CH₂ benzyl)

### Exemple 79 : 5-hydroxy-7-propyl-6-[4-[3-(1-H-tétrazol-5-yl)-2-thiényl] benzyl]-1,2,4-triazolo [1,5-a] pyrimidine

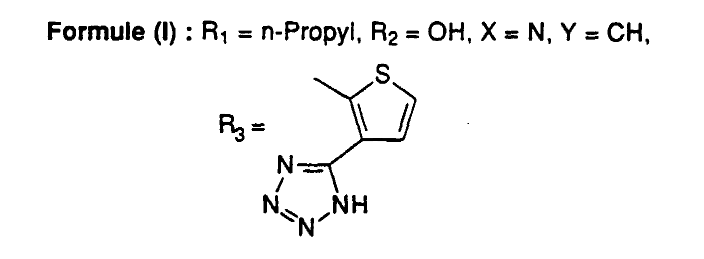

Formule brute : C₂₀ H₁₈ N₈OS

Point de fusion : 275°C

¹H RMN (DMSO-d₆) : 2.95 (t, 2H, CH₂ n-propyl) 3.91 (s, 2H, CH₂ benzyl) ; 8.12 (s, 1H, H₂)

### Exemple 80 : 5-hydroxy-7-propyl-6-[4-[3-(1-H-tétrazol-5- yl)-2-pyridyl] benzyl]-1,2,4-triazolo [1,5-a] pyrimidine

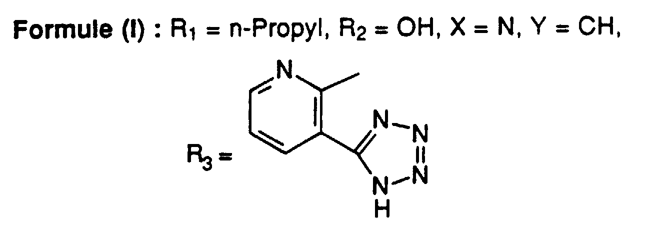

Formule brute : C₂₁ H₁₉ N₉O

Point de fusion : 244°C

¹H RMN (DMSO-d₆) : 2.91 (t, 2H, CH₂ n-propyl) ; 3.89 (s, 2H, CH₂ benzyl) ; 8.11 (s, 1H, H₂ avec H₄ pyridinique)

### Exemple 81 : 5-hydroxy-2-méthyl-7-propyl-6-[4-[3-(1-H-tétrazol-5-yl)-2-thiényl] benzyl]-1,2,4-triazolo [1,5-a] pyrimidine

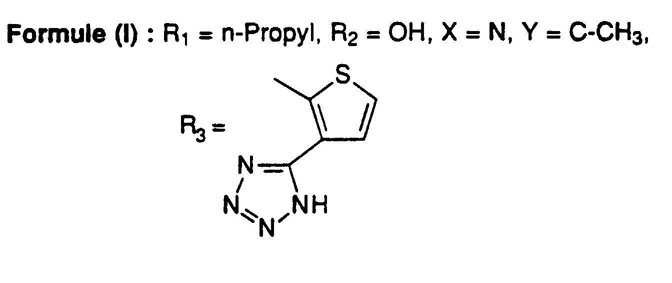

Formule brute : C₂₁ H₂₀ N₈OS

Point de fusion : 287°C

¹H RMN (DMSO-d₆) : 2.9 (t, 2H, CH₂ n-propyl) 3.89 (s, 2H, CH₂ benzyl)

### Exemple 82: 7-butyl-5-hydroxy-2-méthyl-6-[(2'(1-H-tétrazol-5- yl) biphényl-4-yl) méthyl]-1,2,4-triazolo [1,5-a] pyrimidine

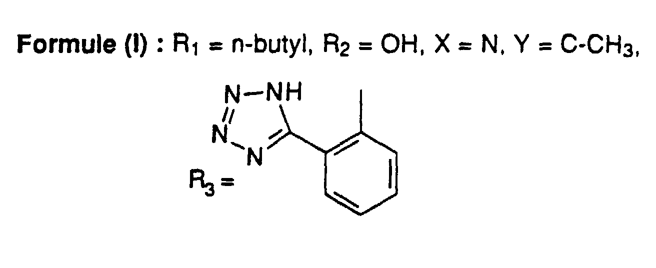

Formule brute : C₂₄ H₂₄ N₈O

Point de fusion : 275°C

¹H RMN (DMSO-d₆) 2.87 (t, 2H, CH₂n-butyl) ; 3.84 (s, 2H, CH₂ benzyl)

### Exemple 83: 5-hydroxy-2-hydroxyméthyl-7-propyl-6-[(2'(1-H-tétrazol-5-yl) biphényl 4-yl) méthyl)-1,2,4-trlazolo [1,5-a] pyrimidine

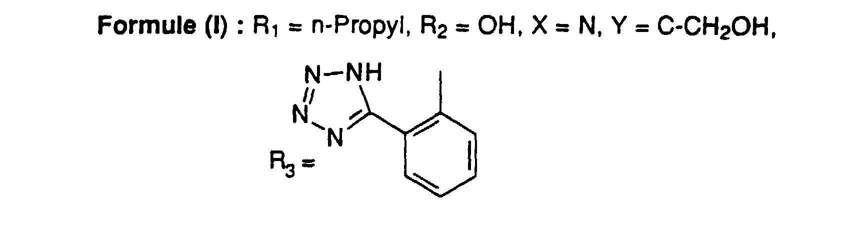

Formule brute : C₂₃ H₂₂ N₈O₂

Point de fusion : 274°C

¹H RMN (DMSO-d₆) : 2.88 (t, 2H, CH₂ n-propyl) ; 3.86 (s, 2H, CH₂ benzyl)

### Exemple 84 : 5-mercapto-7-propyl-6-[(2'(1-H-tétrazol-5- yl) biphényl-4-yl) méthyl]-1,2,4-triazolo [1,5-a] pyrimidine

**Formule (I) :** R₁ = n-Propyl, R₂ = SH, X = N, Y = CH,

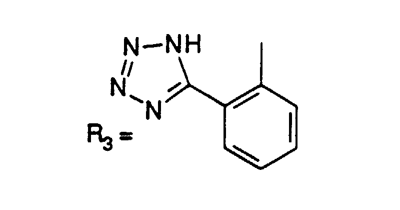

Formule brute : C₂₂ H₂₀ N₈S

Point de fusion : 278°C

¹H RMN (DMSO-d₆) : 2.87 (t, 2H, CH₂ n-propyl) ; 3.37 (s, 2H, CH₂ benzyl) ; 8.29 (s, 1H, H₂)

### Exemple 85 : 5-hydroxy-7-méthoxyméthyl-6-[(2'(1-H-tétrazol-5-yl) biphényl-4-yl) méthyl]-1,2,4-triazolo [1,5-a] pyrimidine

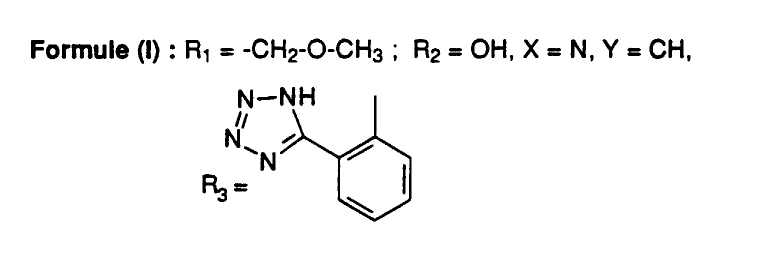

Formule brute : C₂₁ H₁₈ N₈O₂

Point de fusion : 264°C

¹H RMN (DMSO-d₆) : 3.91 (t, 2H, CH₂ benzyl) ; 4.79 (s, 2H, O-CH₂) ; 8.12 (s, 1H, H₂)

### Exemple 86 : 7-propyl-5-(pyrrolidin-1-yl )-6-[(2'(1-H-tétrazol-5-yl) biphényl-4-yl) méthyl]-1,2,4-triazolo [1,5-a] pyrimidine

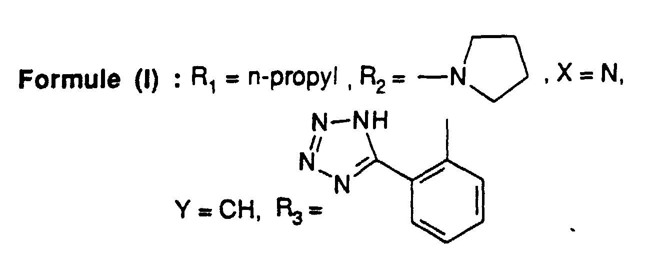

Formule brute : C₂₆ H₂₇ N₉

Point de fusion : 280°C

¹H RMN (DMSO-d₆) : 2.94 (t, 2H, CH₂ propyl) ; 4.22 (s, 2H, CH₂ benzyl) ; 8.18 (s, 1H, H₂)

### Exemple 87 : 5-hydroxy-7-méthyl-6-[(2'(1-H-tétrazol-5-yl) biphényl-4-yl) méthyl]-1,2,4-triazolo [1,5-a] pyrimidine

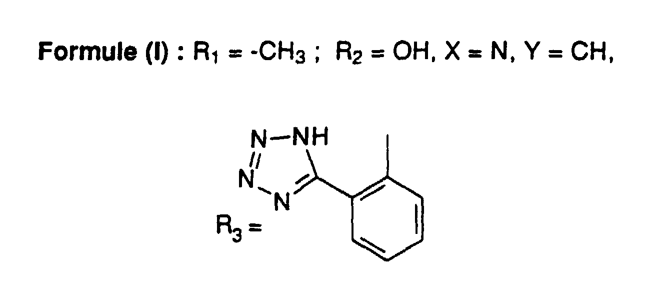

Formule brute : C₂₀ H₁₆ N₈O

Point de fusion : 248°C

¹H RMN (DMSO-d₆) : 2.56 (s, 3H, CH₃) ; 3.86 (s, 2H,CH₂ benzyl) ; 8.11 (s, 1H, H₂)

### Exemple 88 : 7-éthyl-5-hydroxy-6-[(2'(1-H-tétrazol-5-yl) biphényl 4-yl) méthyl]-1,2,4-triazolo [1,5-a] pyrimidine

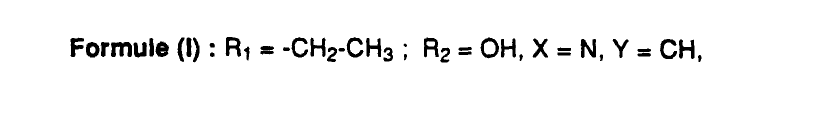

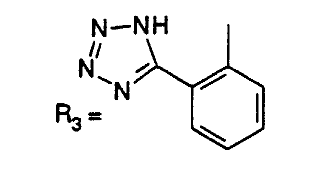

Formule brute : C₂₁ H₁₈ N₈O

Point de fusion : 245°C

¹H RMN (DMSO-d₆) : 2.94 (q, 2H, CH₂ éthyl) ; 3.87 (s, 2H,CH₂ benzyl) ; 8.12 (s, 1H, H₂)

### Exemple 89 : 2-N,N-diéthyl amino-5-hydroxy-6-[(2'(1-H-tétrazol-5-yl) biphényl-4-yl) méthyl]-1,2,4-triazolo [1,5-a] pyrimidine

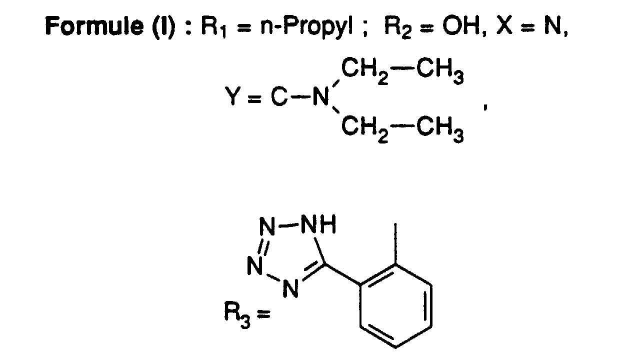

Formule brute : C₂₆ H₂₉ N₉O

Point de fusion : 207°C

¹H RMN (DMSO-d₆) : 2.79 (t, 2H, CH₂ n-propyl) ; 3.80 (s, 2H,CH₂ benzyl)

### Exemple 90 : 5-(morpholin-4-yl éthylamino)-7-propyl-6-[(2'(1-H-tétrazol-5-yl) biphényl-4-yl) méthyl]-1,2,4-triazolo [1,5-a] pyrimidine

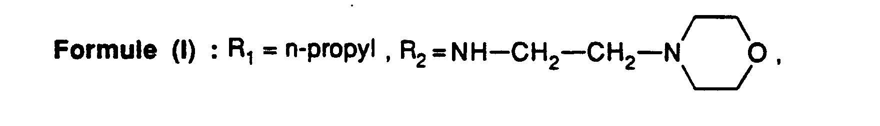

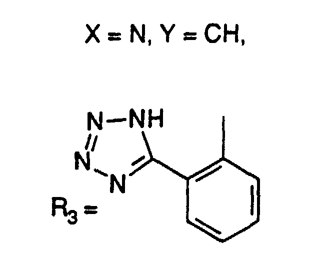

Formule brute : C₂₈ H₃₂ N₁₀O

Point de fusion : 236°C

¹H RMN (DMSO-d₆) : 2.99 (t, 2H, CH₂n- propyl) ; 4.02 (s, 2H,CH₂ benzyl) ; 8.13 (s, 1H, H₂)

### Exemple 91 : 5,7-dipropyl-6-[(2'(1-H-tétrazol-5-yl) biphényl-4-yl) méthyl]-1,2,4-triazolo [1,5-a] pyrimidine

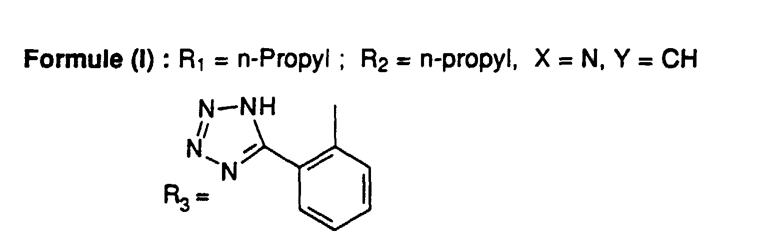

Formule brute : C₂₅ H₂₆ N₈

Point de fusion : 226°C

¹H RMN (DMSO-d₆) : 2.66 (t, 2H, CH₂n- propyl) ; 3.15 (t, 2H,CH₂ n-propyl) ; 4.14 (s, 2H, CH₂ benzyl) ; 8.26 (s, 1H, H₂)

### Exemple 92 : 7-propyl-6-[(2'(1 -H-tétrazol-5-yl) biphényl-4-yl) méthyl]-1,2,4-triazolo [1,5-a] pyrimidine

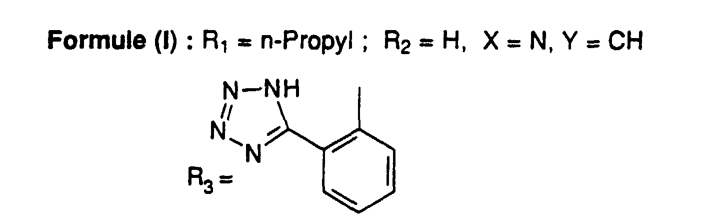

Formule brute : C₂₂ H₂₀ N₈

Point de fusion : 238°C

¹H RMN (DMSO-d₆) : 3.16 (t, 2H, CH₂n- propyl) ; 4.21 (s, 2H, CH₂ benzyl) ; 8.65 (s, 1H) ; 8.82 (s, 1H)

### Exemple 93 : 7-benzyloxyméthyl-5-hydroxy-6-[(2'(1-H-tétrazol-5-yl) biphényl-4-yl) méthyl]-1,2,4-triazolo [1,5-a] pyrimidine

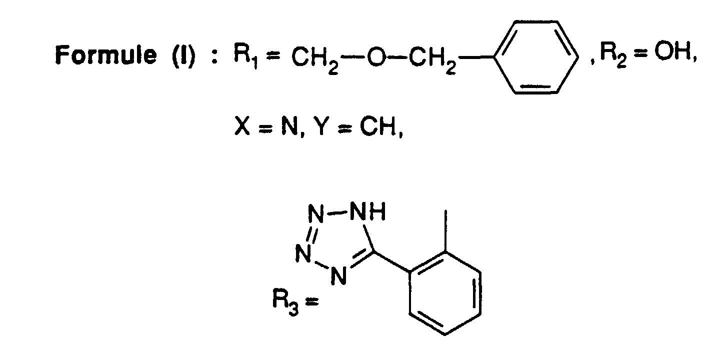

Formule brute : C₂₇ H₂₂ N₈ O₂

Point de fusion : 270-5°C (décomposition)

¹H RMN (DMSO-d₆) : 3.86 (s, 2H, CH₂ benzyl) ; 4.62 (s, 2H, O-CH₂) ; 4.88 (s, 2H, O-CH₂) ; 8.11 (S, 1H, H₂)

### Exemple 94 : 5-(piperidin-1-yl)-7-propyl-6-[(2'(1-H-tétrazol-5-yl) biphényl-4-yl) méthyl]-1,2,4-triazolo [1,5-a] pyrimidine

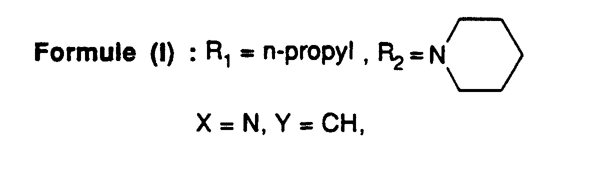

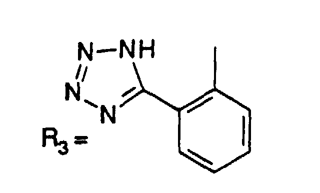

Formule brute : C₂₇ H₂₉ N₉

Point de fusion : 266°C

¹H RMN (DMSO-d₆) : 2.88 (t, 2H, CH₂n- propyl) ; 4.09 (s, 2H, CH₂ benzyl) ; 8.35 (s, 1H, H₂)

### Exemple 95 : [7-propyl-6-[(2'(1-H-tétrazol-5-yl) biphényl-4-yl) méthyl]-1,2,4-triazolo [1,5-a] pyrimidin-5-yl]-2-méthoxy éthyl éther

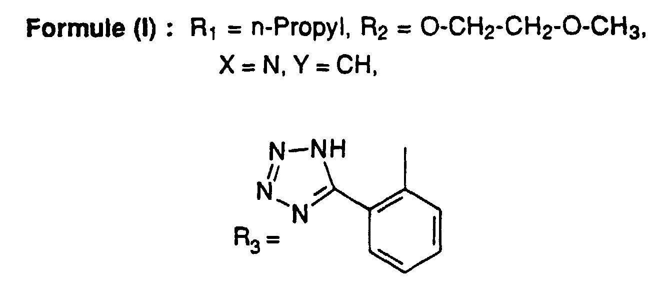

Formule brute : C₂₅ H₂₆ N₈ O₂

Point de fusion : 224°C

¹H RMN (DMSO-d₆) : 3.14 (t, 2H, CH₂n- propyl) ; 4.04 (s, 2H, CH₂ benzyl) ; 8.41 (s, 1H, H₂)

### Exemple 96 : 5-chloro-7-propyl-6-[(2'(1H-tétrazol-5-yl) biphényl-4-yl) méthyl]-1,2,4-triazolo [1,5-a] pyrimidine

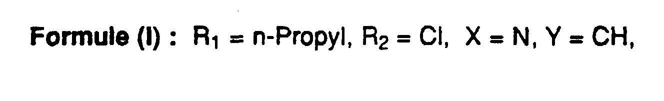

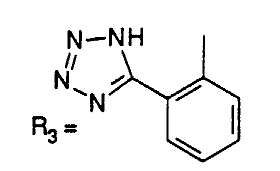

Obtenu par diazotation du dérivé de l'exemple 77 et traitement du sel de diazonium par le chlorure cuivreux selon réaction classique de Sandmeyer.

### Exemple 97 : 6-[(2'-aminocarbonyl biphényl-4-yl) méthyl]-5-hydroxy-7-propyl-1,2,4-triazolo [4,3-a] pyrimidine

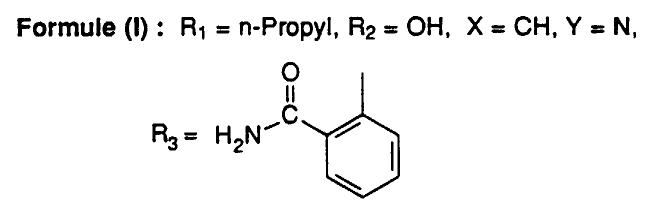

On porte au reflux pendant 4 h, 2 g du composé de l'exemple 20d dans 200 ml de NaOH 1N. Concentrer, sous vide, acidifier avec 200 ml de HCI 1N. Les cristaux obtenus sont purifiés par recristallisation dans le méthoxy-2 éthanol pour donner 1.6 g de 6-[(2'-aminocarbonyl biphényl-4-yl) méthyl]-5-hydroxy-7-propyl-1,2,4-triazolo [4,3-a] pyrimidine.

Formule brute : C₂₂ H₂₁ N₅ O₂

Point de fusion : 258°C

¹H RMN (DMSO-d₆) : 2.61 (t, 2H, CH₂n- propyl) ; 3.9 (s, 2H, CH₂ benzyl) ; 9 (s, 1H, H₃)

### Exemple 98 : 6-[(2'-carboxybiphényl-4-yl) méthyl]-5-hydroxy-7-propyl-1,2,4-triazolo [1,5-a] pyrimidine

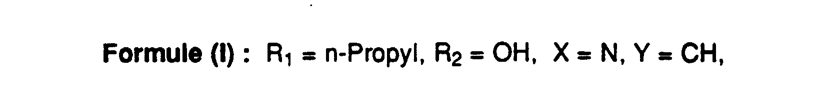

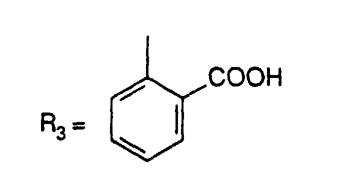

On porte au reflux pendant 10 h un mélange de 9.4 g de produit obtenu à l'exemple 97, 200 ml d'éthylène glycol et 20 ml de NaOH concentré. Distiller l'éthylène glycol, ajouter 200 ml d'eau et acidifier avec une solution de HCI. Les cristaux obtenus sont purifiés par recristallisation dans le méthoxy-2 éthanol pour donner 5.8 g de 6-[(2'-carboxybiphényl-4-yl) méthyl]-5-hydroxy-7-propyl-1,2,4-triazolo [1,5-a] pyrimidine.

Formule brute : C₂₂ H₂₀ N₄ O₃

Point de fusion : 265°C

¹H RMN (DMSO-d₆) : 2.96 (t, 2H, CH₂n- propyl) ; 3.92 (s, 2H, CH₂ benzyl) ; 8.12 (s, 1H, H₂)
Selon le mode opératoire de l'exemple 23, le composé de l'exemple 99 a été préparé.

### Exemple 99: 6-[4-(5-bromo-3-cyano-2-furyl) benzyl]-5-hydroxy-7-propyl-1,2,4-triazolo [1,5-a] pyrimidine

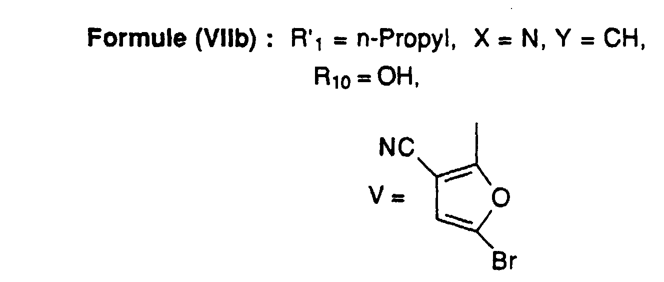

Point de fusion : 262°C.

### Exemple 100 : 6-[(2'-cyano biphényl-4-yl) méthyl]-5-hydroxy-7-hydroxyméthyl-1,2,4-triazolo [1,5-a] pyrimidine

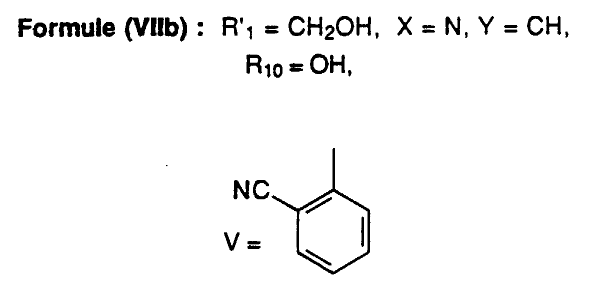

Réduire par hydrogénation catalytique en présence de 1.8 g de palladium sur charbon à 5 %, 9 g de composés préparés à l'exemple 43 en solution dans 360 ml d'acide acétique. La réaction est effectuée à pression atmosphérique et à 50°C. Filtrer sur célite 545. Laver à l'acide acétique, concentrer puis purifier par chromatographie sur gel de silice (éluant : chloroforme 95 % / méthanol 5 %) pour obtenir 4.5 g de produit de départ et 2.2 g de 6-[(2'-cyano biphényl-4-yl) méthyl]-5-hydroxy-7-hydroxyméthyl-1,2,4-triazolo [1,5-a] pyrimidine.

Point de fusion 262°C.

Ce même composé peut être également obtenu par réaction avec le BBr₃ dans du chloroforme.

Selon le mode opératoire de l'exemple 63, les dérivés suivants des exemples 101 et 102 ont été préparés.

### Exemple 101 : 5-hydroxy-7-hydroxyméthyl-6-[(2'-(1H-tétrazolo-5-yl) biphényl-4-yl) méthyl]-1,2,4-triazolo [1,5-a] pyrimidine

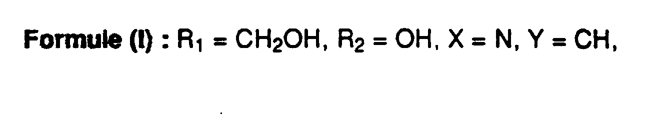

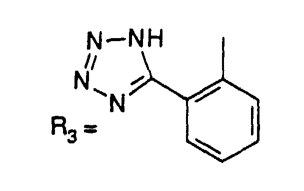

Formule brute : C₂₀ H₁₅ N₅ O₂

Point de fusion : > 360°C (décomposition)

¹H RMN (DMSO-d₆) : 3.93 (s, 2H, CH₂ benzyl) ; 4.82 (s, 2H, CH₂-O) ; 8.06 (s, 1H, H₂)

### Exemple 102 : 6-[4-[5-bromo-3-(1H-tetrazol-5-yl)-2-furyl] benzy]-5-hydroxy-7-propyl-1,2,4-triazolo [1,5-a] pyrimidine

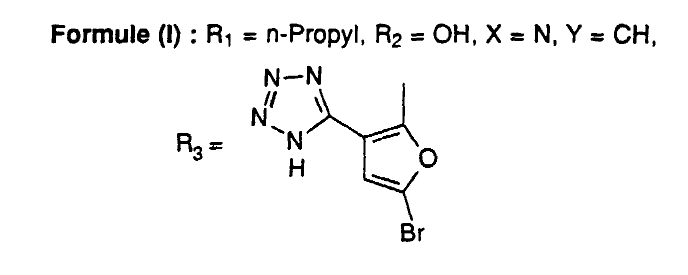

Formule brute : C₂₀ H₁₇ Br N₈ O₂

Point de fusion : > 360°C

¹H RMN (DMSO-d₆) : 2.93 (t, 2H, CH₂ n-propyl) ; 3.92 (s, 2H, CH₂ benzyl) ; 6.9 (s, 1H, proton furanique) ; 8.03 (s, 1H, H₂)

### PHARMACOLOGIE

### A • Etude des récepteurs surrénaliens à l'angiotensine Il

### I. Principe

L'affinité des produits des exemples pour les récepteurs de l'angiotensine Il est évaluée par technique de déplacement d'un radioligand spécifiquement fixé sur les récepteurs surrénaliens de l'angiotensine Il, chez le rat.

### II. Mode opératoire

Une aliquote d'un homogénat de surrénales de rat incube en présence d'une concentration unique de [1251] - SIAII (Sar1, Tyr4, Ile8 - angiotensine il), antagoniste des récepteurs de l'angiotensine Il, et de deux concentrations d'agents compétiteurs (10-5 M, 10-7 M) durant 60 min à 25°C.

La réaction est achevée par ajout de tampon, puis rapide filtration à travers des filtres de papier de verre. La liaison non spécifique est déterminée en présence d'angiotensine II.

### III. Expression des résultats

Les résultats sont exprimés, pour les concentrations testées, en pourcentage de déplacement du radioligand spécifiquement fixé sur les récepteurs surrénaliens de l'angiotensine il.

### IV. Résultats

| Produit de l'exemple | % de déplacement du ligand marqué | |
|---|---|---|
| | 1E-5M | 1E-7M |
| Exemple 62 | 65 | 52 |
| Exemple 63 | 61 | 52 |
| Exemple 65 | 63 | 47 |
| Exemple 68 | 69 | 59 |
| Exemple 69 | 69 | 19 |
| Exemple 75 | 61 | 60 |
| Exemple 76 | 63 | 28 |
| Exemple 77 | 63 | 31 |
| Exemple 79 | 58 | 26 |
| Exemple 82 | 58 | 11 |

**B. Mesure de l'inhibition de la prolifération cellulaire induite par les facteurs de croissance (exemple : le Platelet-Derived Growth Factor ou PDGF) dans les cellules musculaires lisses d'aortes de rat**

### I. Principe

L'inhibition de la prolifération cellulaire induite par un facteur de croissance (exemple : le PDGF) est évaluée par mesure de l'incorporation de 3H-thymidine dans les cellules musculaires lisses d'aortes de rat (CMLV).

### II. Mode opératoire

Les CMLV sont cultivées à 37°C, en 5 % C02, jusqu'à la sous-confluence puis placées pendant 24 heures en quiescence dans un milieu pauvre en sérum. Elles sont ensuite prétraitées pendant une heure par la molécule à tester (10-4M) puis stimulées pendant 22 heures par un facteur de croissance (exemple : le PDGF). L'incorporation de 3H-thymidine est effectuée pendant les 4 dernières heures. Toutes ces étapes sont effectuées à 37°C, en 5 % CO2.
La réaction est terminée par aspiration du milieu réactionnel, détachement des cellules, puis filtration des cellules lysées à travers des filtres en fibres de verre.

### III. Expression des résultats

Les résultats sont exprimés en pourcentage d'inhibition de la stimulation d'incorporation de 3H-thymidine due à l'action du facteur de croissance.

### IV. Résultats

| Produit de l'exemple | % inhibition de l'incorporation de 3H-thymidine induite par le PDGF **1E - 4M** |
|---|---|
| Exemple 69 | 100 |

### TOXICOLOGIE

Les produits des exemples décrits présentent, après administration par voie orale une excellente tolérance.

Leur dose létale 50 chez le rat a été évaluée supérieure à 300 mg/kg.

### CONCLUSION

Les produits des exemples décrits présentent une bonne affinité pour les récepteurs à l'angiotensine Il. A ce titre, ils pourront être utilisés avec bénéfice dans les diverses pathologies où l'angiotensine Il est impliquée, en particulier dans les traitements de l'hypertension artérielle de l'insuffisance cardiaque, à des posologies de 1 à 400 mg par voie orale et 0.01 à 50 mg par voie intraveineuse, en une ou plusieurs prises par jour. De plus, certains composés présentent également une activité antiproliférative, et à ce titre, sont potentiellement intéressants dans le traitement de maladies prolifératives telles que l'athérosclérose.

## Revendications

1. Dérivés de triazolopyrimidines caractérisés en ce qu'ils répondent à la formule générale (I) : 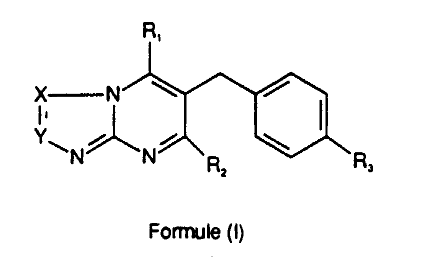 Dans laquelle :
- l'un des radicaux R₁ et R₂ représente
- un radical alkyle inférieur de 1 à 6 atomes de carbone ;
- un radical éther de formule -(CH₂)ₚ OR dans laquelle p est un nombre entier de 1 à 6 et R représente un radical alkyle inférieur de 1 à 6 atomes de carbone ou un radical benzyle ;
- un radical alcool de formule -(CH₂)ₚ OH dans laquelle p est tel que défini précédemment ;
- l'autre des radicaux R₁ et R₂ représente
- l'atome d'hydrogène,
- un atome d'halogène,
- un radical alkyle inférieur de 1 à 6 atomes de carbone,
- un radical choisi dans le groupe comprenant les radicaux N₃, OR₄, SR₄,NR₅R₆, NH(CH₂)ₙ-NR₅R₆ ;
dans lesquels :
R₄ représente :
- l'atome d'hydrogène,
- un radical alkyle inférieur de 1 à 6 atomes de carbone ou un radical cycloalkyle en C₃-C₇,
- un radical (CH₂)ₘ-COOR', m étant un nombre entier de 1 à 4, R' l'atome d'hydrogène ou un radical alkyle inférieur de 1 à 6 atomes de carbone,
- un radical (CH₂)ₘ-O-R', m et R' étant défini comme ci-dessus ;
R₅ et R₆, identiques ou différents, représentent :
- l'atome d'hydrogène,
- un radical alkyle inférieur de 1 à 6 atomes de carbone ou un radical cycloalkyle en C₃-C₇,
ou
R₅ et R₆, pris ensemble avec l'atome d'azote auquel ils sont rattachés, forment un hétérocycle choisi parmi la morpholine, la pyrrolidine ou la pipéridine ;
- n est un nombre entier de 1 à 4,
- X et Y différents représentent :
- l'un, l'atome d'azote,
- l'autre un groupement C-R₇ dans lequel R₇ représente
- l'atome d'hydrogène,
- un radical alkyle inférieur de 1 à 6 atomes de carbone ou un radical cycloalkyle en C₃-C₇,
- un radical (CH₂)_{n'} OH dans lequel n' est un nombre entier de 0 à 4,
- un radical SR',R' étant tel que défini précédemment,
- un radical NR₅R₆ dans lequel R₅ et R₆ identiques ou différents représentent l'atome d'hydrogène ou un radical alkyle inférieur de 1 à 6 atomes de carbone ou un radical cycloalkyle en C₃-C₇ ;
- R₃ représente un radical de formule : 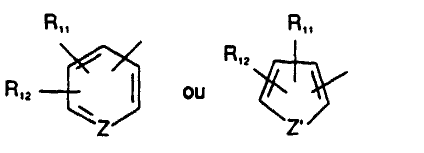 dans lesquelles
- Z représente CH, N ou Z' représente S, O,
- R₁₁ représente l'atome d'hydrogène ou un atome d'halogène,
- R₁₂ représente un radical tétrazole, CN, COOH, CONH₂, ainsi que leurs formes tautomères et leurs sels d'addition pharmaceutiquement acceptables.

2. Dérivés selon la revendication 1, caractérisés en ce qu'ils répondent à la formule générale (I) précitée dans laquelle :
- l'un des radicaux R₁ et R₂ représente :
- un radical alkyle inférieur de 1 à 6 atomes de carbone ;
- un radical éther de formule -(CH₂)ₚ OR dans laquelle p est un nombre entier de 1 à 6 et R représente un radical alkyle inférieur de 1 à 6 atomes de carbone ou un radical benzyle ;
- un radical alcool de formule -(CH₂)ₚ OH dans laquelle p est tel que défini précédemment ;
- l'autre des radicaux R₁ et R₂ représente :
- l'atome d'hydrogène,
- un atome d'halogène,
- un radical alkyle inférieur de 1 à 6 atomes de carbone,
- un radical choisi dans le groupe comprenant les radicaux N₃, OR₄, SR₄, NR₅R₆, NH(CH₂)ₙ-NR₅R₆ ;
dans lesquels :
R₄ représente :
- l'atome d'hydrogène,
- un radical -(CH₂)ₘ-O-R' dans lequel, m est un nombre entier de 1 à 4 et R' représente un radical alkyle inférieur de 1 à 6 atomes de carbone,
R₅ et R₆, identiques ou différents, représentent :
- l'atome d'hydrogène,
- un radical alkyle inférieur de 1 à 6 atomes de carbone, ou
R₅ et R₆, pris ensemble avec l'atome d'azote auquel ils sont rattachés, forment un hétérocycle choisi parmi la morpholine, la pyrrolidine ou la pipéridine ;
- n est un nombre entier de 1 à 4
- X et Y différents représentent :
- l'un, l'atome d'azote,
- l'autre un groupement C-R₇ dans lequel R₇ représente
- l'atome d'hydrogène,
- un radical alkyle inférieur de 1 à 6 atomes de carbone,
- un radical (CH₂)ₙ, OH dans lequel n' est un nombre entier de 0 à 4,
- un radical SR',R' étant tel que défini précédemment,
- un radical NR₅R₆ dans lequel R₅ et R₆ identiques ou différents représentent l'atome d'hydrogène ou un radical alkyle inférieur de 1 à 6 atomes de carbone ;
- R₃ représente l'un des radicaux suivants :

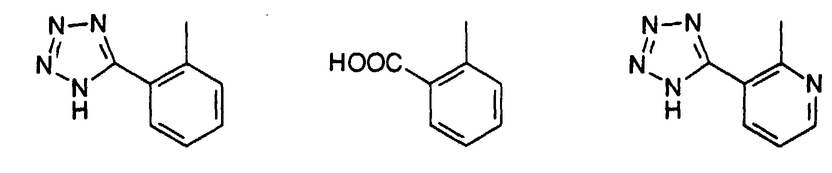

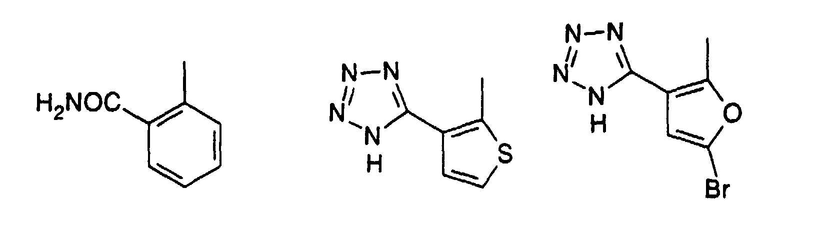

3. Dérivés selon la revendication 1 ou 2 caractérisés en ce que R₁ est un groupement n-propyl, n-butyl ou N-diéthylamino.

4. Dérivés selon l'une quelconque des revendications 1 à 3, caractérisés en ce que R₂ est un groupement hydroxy, n-propyl ou N-diéthylamino.

5. Dérivés selon l'une quelconque des revendications 1 à 4 caractérisés en ce que R₃ est un groupement 2-(1-H tétrazol-5-yl) phényl.

6. Dérivés selon l'une quelconque des revendications 1 à 5 caractérisés en ce que X est l'atome d'azote.

7. Dérivés selon l'une quelconque des revendications 1 à 6 caractérisés en ce que Y représente le groupement CH, C-CH₃ ou C-NH₂.

8. Dérivés selon la revendication 1 ou 2 caractérisés en ce que R₁ est un radical alkyle inférieur de 1 à 6 atomes de carbone, R₂ est un groupement hydroxy, R₃ est un groupement 2-(1-H tétrazol-5-yl) phényl, X représente l'atome d'azote et Y représente le groupement CH ou C-CH₃.

9. Dérivé selon la revendication 1 ou 2 caractérisé en ce qu'il s'agit du dérivé :
-5-hydroxy-7-propyl-6-[(2'-(1-H-tétrazol-5-yl) biphényl-4-yl) méthyl]-1,2,4-triazolo [1,5-a] pyrimidine

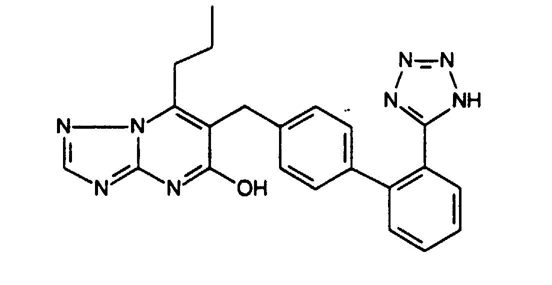

10. Dérivés selon la revendication 1 ou 2 caractérisés en ce qu'ils sont choisis parmi les dérivés de formule :

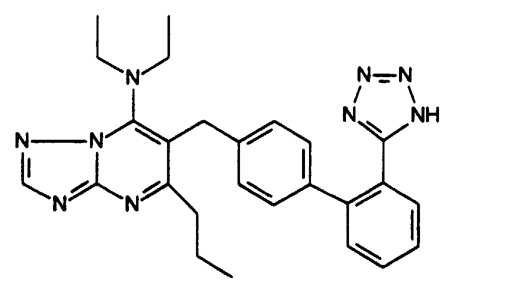

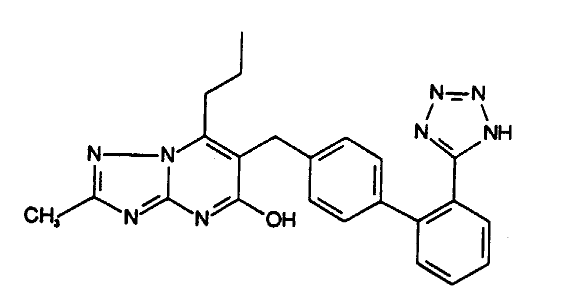

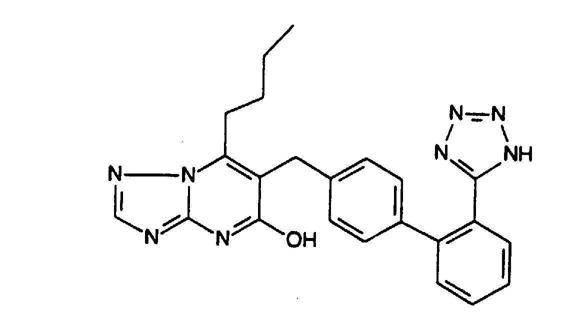

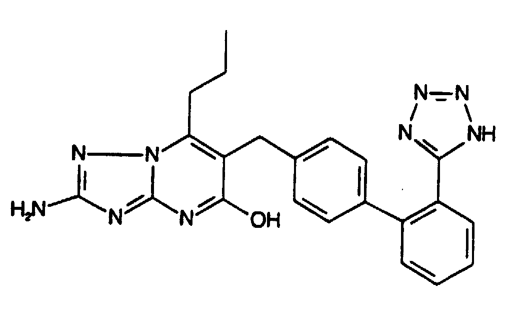

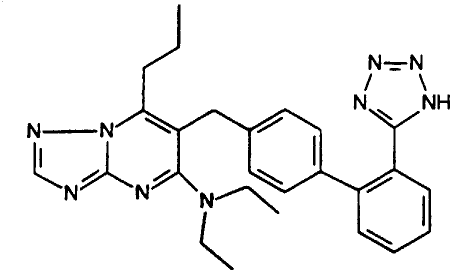

11. Procédé de préparation des composés de formule (I) selon l'une quelconque des revendications 1 à 10, caractérisé en ce qu'il comprend :
a) la préparation d'un composé de formule (α) 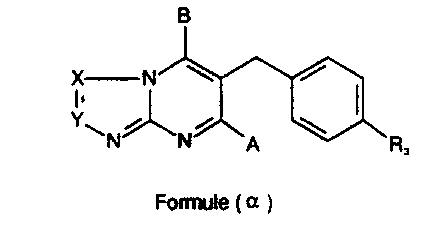 dans laquelle :
- X, Y et R₃ sont tels que définis à la revendication 1 ;
- A et B représentent l'un un groupe hydroxy ou un radical alkyle inférieur de 1 à 6 atomes de carbone, l'autre un radical alkyle inférieur de 1 à 6 atomes de carbone ou un radical éther de formule -(CH₂)pOR dans laquelle p est un nombre entier de 1 à 6 et R représente un radical alkyle inférieur de 1 à 6 atomes de carbone ou un radical benzyle,
par condensation d'un 3-amino-1,2,4-triazole de formule (II) : 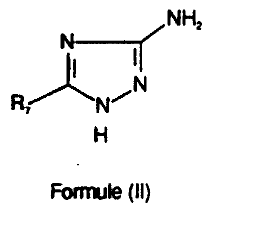 dans laquelle R7 est tel que défini à la revendication 1 sur un dérivé de formule β : 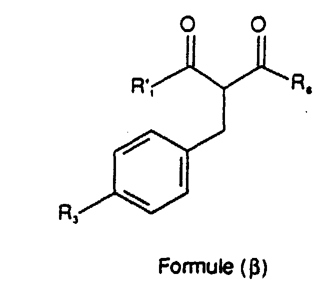 dans laquelle R'1 est un radical alkyle inférieur de 1 à 6 atomes de carbone ou un radical éther de formule -(CH₂)ₚ OR dans laquelle p est un nombre entier de 1 à 6 et R représente un radical alkyle inférieur de 1 à 6 atomes de carbone ou un radical benzyle, R₈ représente un radical alkyle inférieur de 1 à 6 atomes de carbone ou un groupement O-alkyle inférieur de 1 à 6 atomes de carbone de préférence méthyle ou éthyle et R₃ est tel que défini à la revendication 1,
dans un solvant aprotique ou dans un solvant acide ou encore dans un alcool en présence de l'alcoolate de sodium ou de potassium correspondant ou encore dans la pyridine ou la 2-méthyl-5-éthyl pyridine en présence ou non de 4-diméthylamino pyridine à une température comprise entre 50 et 200°C ;
b) éventuellement, la protection de la fonction portée par R₃ selon une méthode connue en soi,
c) le chauffage du dérivé ainsi obtenu à partir du dérivé de la formule (β) lorsque celui-ci est un céto ester dans un réactif approprié pour transformer le groupe hydroxy représenté par A ou B en un atome de chlore ;
d) le chauffage de ce dérivé chloré en présence d'un nucléophile azoté, oxygéné ou soufré dans un alcool au reflux ou à l'autoclave à 100°C, en présence ou non d'une base pour obtenir un dérivé de formule (α) dans laquelle A et B ont la même signification que R₁ et R₂ respectivement;
e) éventuellement, la déprotection de la fonction portée par R₃;
e₁) la transformation de cette fonction en une fonction acide ; ou
e₂) la transformation de cette fonction en une fonction tétrazole ; ou
e₃) la transformation de cette fonction en fonction amide ; et
f) éventuellement, la transformation du dérivé ainsi obtenu en un sel d'addition.

12. Composition pharmaceutique à activité antaqoniste des récepteurs à l'angiotensine Il permettant de traiter et de prémunir favorablement les maladies cardiovasculaires notamment l'hypertension, l'insuffisance cardiaque, les maladies de la paroi artérielle, caractérisée en ce qu'elle renferme une quantité pharmaceutiquement efficace d'au moins un composé de formule (I) tel que défini dans l'une quelconque des revendications 1 à 10, ou un de ses sels d'addition pharmaceutiquement acceptables, éventuellement incorporé dans un excipient, véhicule ou support pharmaceutiquement acceptable.

13. Composition pharmaceutique à activité antiproliférative permettant de traiter et de prémunir favorablement les maladies de la paroi artérielle notamment l'athérosclérose caractérisée en ce qu'elle renferme une quantité pharmaceutiquement efficace d'au moins un composé de formule (I) tel que défini dans l'une quelconque des revendications 1 à 10, ou un de ses sels d'addition pharmaceutiquement acceptables, éventuellement incorporé dans un excipient, véhicule ou support pharmaceutiquement acceptable.

## Patentansprüche

1. Triazolopyrimidin-Derivate, dadurch gekennzeichnet, daß sie der allgemeinen Formel (I) entsprechen: 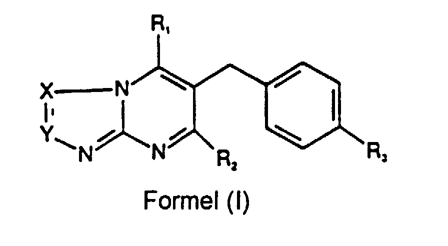 worin bedeuten:
einer der Reste R₁ und R₂
- einen niederen Alkylrest mit 1 bis 6 Kohlenstoffatomen;
- einen Etherrest der Formel -(CH₂)ₚOR, worin p für eine ganze Zahl von 1 bis 6 und R für einen niederen Alkylrest mit 1 bis 6 Kohlenstoffatomen oder einen Benzylrest stehen;
- einen Alkohol-Rest der Formel -(CH₂)ₚOH, worin p wie oben definiert ist;
der andere der Reste R₁ und R₂
- ein Wasserstoffatom,
- ein Halogenatom,
- einen niederen Alkylrest mit 1 bis 6 Kohlenstoffatomen,
- einen Rest, ausgewählt aus der Gruppe, die N₃, OR₄, SR₄, NR₅R₆ und NH(CH₂)ₙ-NR₅R₆ umfaßt, worin R₄ für ein Wasserstoffatom; einen niederen Alkylrest mit 1 bis 6 Kohlenstoffatomen oder einen C₃-C₇-Cycloalkyirest, für einen Rest (CH₂)ₘ-COOR', worin m eine ganze Zahl von 1 bis 4 und R' ein Wasserstoffatom oder einen niederen Alkylrest mit 1 bis 6 Kohlenstoffatomen darstellen; für einen Rest (CH₂)ₘ-O-R', worin m und R' wie oben definiert sind; und R₅ und R₆, die identisch oder verschieden sind, für ein Wasserstoffatom, für einen niederen Alkylrest mit 1 bis 6 Kohlenstoffatomen oder einen C₃-C₇-Cycloalkylrest stehen, oder R₅ und R₆ gemeinsam zusammen mit dem Stickstoffatom, an das sie gebunden sind, einen Heterocyclus, ausgewählt aus der Gruppe Morpholin, Pyrrolidin und Piperidin, bilden, und n für eine ganze Zahl von 1 bis 4 steht,
X und Y, die verschieden sind, jeweils
- der eine davon ein Stickstoffatom und
- der andere davon eine C-R₇-Gruppe, in der R₇ für ein Wasserstoffatom; für einen niederen Alkylrest mit 1 bis 6 Kohlenstoffatomen oder einen C₃-C₇-Cycloalkylrest; für einen Rest (CH₂)_{n,}-OH, worin n' eine ganze Zahl von 0 bis 4 darstellt; für einen Rest SR', worin R' wie oben definiert ist; oder für einen Rest NR₅R₆ steht, worin R₅ und R₆, die gleich oder verschieden sind, ein Wasserstoffatom oder einen niederen Alkylrest mit 1 bis 6 Kohlenstoffatomen oder einen C₃-C₇-Cycloalkylrest darstellen;
R₃ einen Rest der Formel 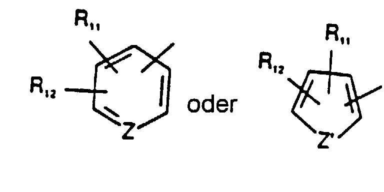 worin bedeuten:
- Z CH, N oder Z' S, O,
- R₁₁ ein Wasserstoffatom oder ein Halogenatom,
- R₁₂ einen Rest Tetrazol, CN, COOH oder CONH₂,
sowie ihre tautomeren Formen und ihre pharmazeutisch akzeptablen Additionssalze.

2. Derivate nach Anspruch 1, dadurch gekennzeichnet, daß sie der obengenannten allgemeinen Formel (I) entsprechen, worin bedeuten: einer der Reste R₁ und R₂
- einen niederen Alkylrest mit 1 bis 6 Kohlenstoffatomen;
- einen Etherrest der Formel -(CH₂)ₚOR, in der p für eine ganze Zahl von 1 bis 6 und R für einen niederen Alkylrest mit 1 bis 6 Kohlenstoffatomen oder einen Benzylrest stehen;
- einen Alkohol-Rest der Formel -(CH₂)ₚOH, in der p wie oben definiert ist;
der andere der Reste R₁ und R₂
- ein Wasserstoffatom,
- ein Halogenatom,
- einen niederen Alkylrest mit 1 bis 6 Kohlenstoffatomen,
- einen Rest, ausgewählt aus der Gruppe, welche die Reste N₃, OR₄, SR₄, NR₅R₆ und NH(CH₂)ₙ-NR₅R₆ umfaßt, worin R₄ steht für ein Wasserstoffatom oder einen Rest -(CH₂)ₘ-O-R', in dem m eine ganze Zahl von 1 bis 4 und R' einen niederen Alkylrest mit 1 bis 6 Kohlenstoffatomen darstellen, R₅ und R₆, die gleich oder verschieden sind, stehen für ein Wasserstoffatom oder einen niederen Alkylrest mit 1 bis 6 Kohlenstoffatomen oder worin R₅ und R₆ gemeinsam zusammen mit dem Stickstoffatom, an das sie gebunden sind, einen Heterocyclus bilden, der ausgewählt wird aus der Gruppe Morpholin, Pyrrolidin und Piperidin, und n steht für eine ganze Zahl von 1 bis 4;
X und Y, die verschieden sind, jeweils
- der eine davon ein Stickstoffatom und
- der andere davon eine C-R₇-Gruppe, in der R₇ für ein Wasserstoffatom, für einen niederen Alkylrest mit 1 bis 6 Kohlenstoffatomen, für einen Rest (CH₂)_{n'}-OH, in dem n' eine ganze Zahl von 0 bis 4 darstellt, für einen Rest SR', worin R' wie oben definiert ist, oder für einen Rest NR₅R₆ steht, in dem R₅ und R₆ gleich sind oder verschieden sind und jeweils ein Wasserstoffatom oder einen niederen Alkylrest mit 1 bis 6 Kohlenstoffatomen darstellen; und
R₃ einen der folgenden Reste:

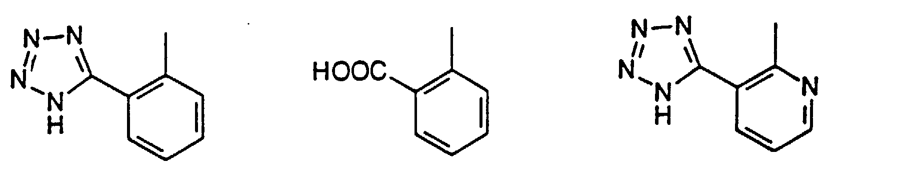

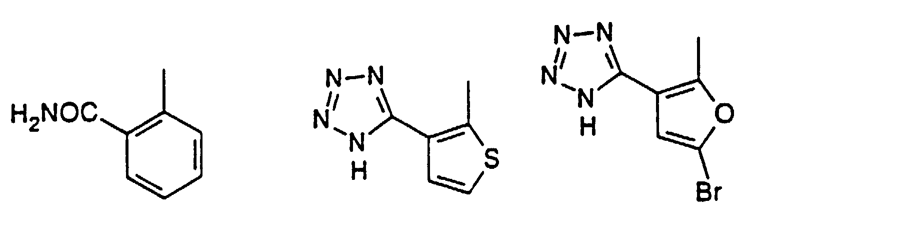

3. Derivate nach Anspruch 1 oder 2, dadurch gekennzeichnet, daß R₁ für eine n-Propyl, n-Butyl- oder N-Diethylamino-Gruppe steht.

4. Derivate nach einem der Ansprüche 1 bis 3, dadurch gekennzeichnet, daß R₂ für eine Hydroxy-, n-Propyl- oder N-Diethylamino-Gruppe steht.

5. Derivate nach einem der Ansprüche 1 bis 4, dadurch gekennzeichnet, daß R₃ für eine 2-(1-H-Terazol-5-yl)phenyl-Gruppe steht.

6. Derivate nach einem der Ansprüche 1 bis 5, dadurch gekennzeichnet, daß X für ein Stickstoffatom steht.

7. Derivate nach einem der Ansprüche 1 bis 6, dadurch gekennzeichnet, daß Y für eine CH-, C-CH₃- oder C-NH₂-Gruppe steht.

8. Derivate nach Anspruch 1 oder 2, dadurch gekennzeichnet, daß R₁ für einen niederen Alkylrest mit 1 bis 6 Kohlenstoffatomen, R₂ für eine Hydroxygruppe, R₃ für eine 2-(1-H-Tetrazol-5-yl)-phenyl-Gruppe, X für ein Stickstoffatom und Y für eine CH- oder C-CH₃-Gruppe stehen.

9. Derivat nach Anspruch 1 oder 2, dadurch gekennzeichnet, daß es sich dabei handelt um das Derivat:
5-Hydroxy-7-propyl-6-[(2'-(1-H-tetrazol-5-yl)biphenyl-4-yl)methyl]-1,2,4-triazolo[1,5-a]pyrimidin

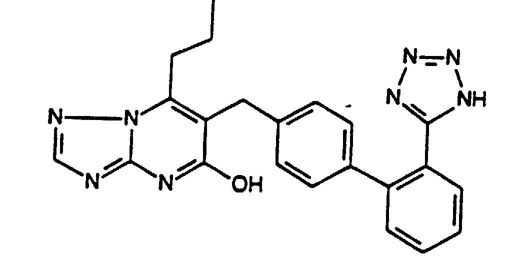

10. Derivate nach Anspruch 1 oder 2, dadurch gekennzeichnet, daß sie ausgewählt werden aus den Derivaten der Formel:

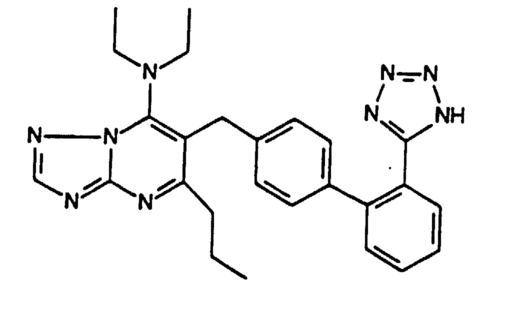

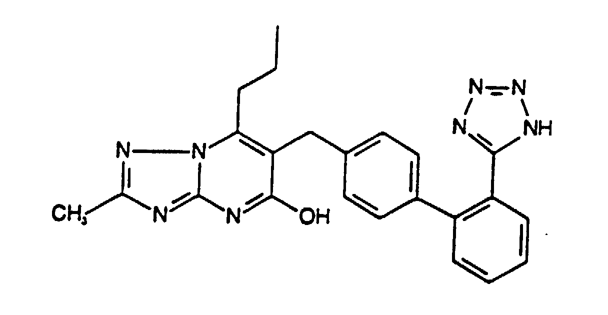

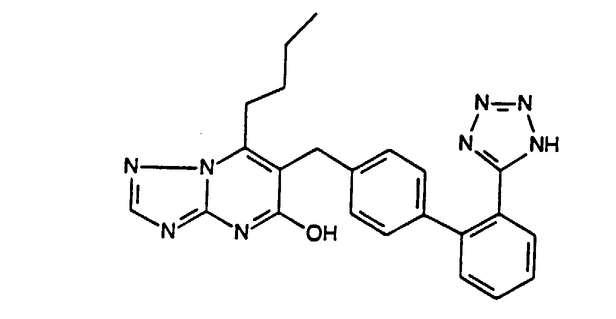

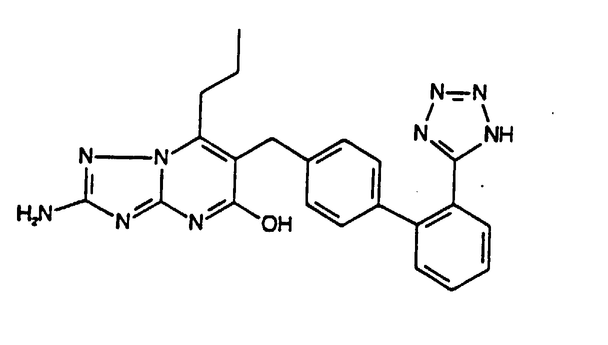

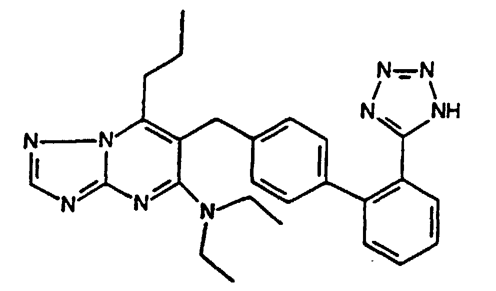

11. Verfahren zur Herstellung der Verbindungen der Formel (I) nach einem der Ansprüche 1 bis 10, dadurch gekennzeichnet, daß es umfaßt:
a) die Herstellung einer Verbindung der Formel (a) 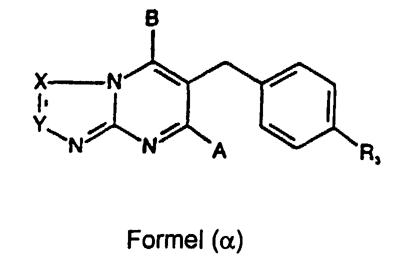 worin bedeuten:
- X, Y und R₃ wie in Anspruch 1 definiert sind;
- einer der Reste A und B eine Hydroxy-Gruppe oder einen niederen Alkylrest mit 1 bis 6 Kohlenstoffatomen und der andere Rest einen niederen Alkylrest mit 1 bis 6 Kohlenstoffatomen oder einen Etherrest der Formel -(CH₂)ₚOR, in der p für eine ganze Zahl von 1 bis 6 und R für einen niederen Alkylrest mit 1 bis 6 Kohlenstoffatomen oder einen Benzylrest stehen,
durch Kondensation eines 3-Amino-1,2,4-triazols der Formel (II): 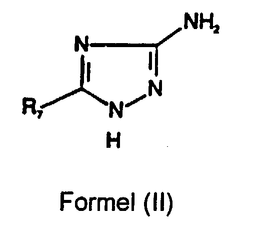 in der R₇ wie in Anspruch 1 definiert ist,
an ein Derivat der Formel (β): 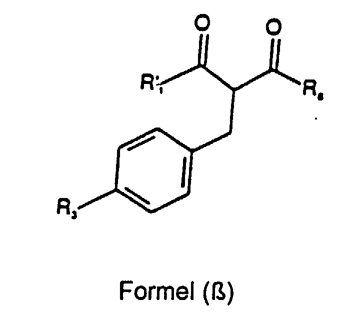 in der R'₁ steht für einen niederen Alkylrest mit 1 bis 6 Kohlenstoffatomen oder einen Etherrest der Formel -(CH₂)ₚOR, in der p eine ganze Zahl von 1 bis 6 und R einen niederen Alkylrest mit 1 bis 6 Kohlenstoffatomen oder einen Benzylrest darstellen, R₈ steht für einen niederen Alkylrest mit 1 bis 6 Kohlenstoffatomen oder eine niedere O-Alkyl-Gruppe mit 1 bis 6 Kohlenstoffatomen, vorzugsweise für O-Methyl oder O-Ethyl, und R₃ wie in Anspruch 1 definiert ist,
in einem aprotischen Lösungsmittel oder in einem sauren Lösungsmittel oder auch in einem Alkohol in Gegenwart des entsprechenden Natrium- oder Kaliumalkoholats oder auch in Pyridin oder in 2-Methyl-5-ethylpyridin in Gegenwart oder in Abwesenheit von 4-Dimethylaminopyridin bei einer Temperatur zwischen 50 und 200°C;
b) gegebenenfalls das Schützen der von R₃ getragenen Funktion auf an sich bekannte Weise,
c) die Erwärmung des auf diese Weise aus dem Derivat der Formel (β) erhaltenen Derivats, wenn es sich dabei um einen Ketoester handelt, in einem geeigneten Reagens, um die durch A oder B dargestellte Hydroxygruppe in ein Chloratom umzuwandeln;
d) die Erwärmung dieses chlorierten Derivats in Gegenwart eines Stickstoff-haltigen, Sauerstoff-haltigen oder Schwefel-haltigen Nucleophils in einem Alkohol unter Rückfluß oder in einem Autoklaven bei 100°C in Gegenwart oder Abwesenheit einer Base, wobei man ein Derivat der Formel (α) erhält, in der A und B jeweils die gleichen Bedeutungen wie R₁ und R₂ haben;
e) gegebenenfalls das Schützen der von R₃ getragenen Funktion;
e₁) die Umwandlung dieser Funktion in eine Säure-Funktion; oder
e₂) die Umwandlung dieser Funktion in eine Tetrazol-Funktion; oder
e₃) die Umwandlung dieser Funktion in eine Amid-Funktion; und
f) gegebenenfalls die Umwandlung des so erhaltenen Derivats in ein Additionssalz.

12. Pharmazeutische Zusammensetzung mit antagonistischer Aktivität gegenüber den Rezeptoren für Angiotensin II, die geeignet ist für die vorteilhafte Behandlung und Vorbeugung von cardiovasculären Erkrankungen, insbesondere Hypertension, Herzinsuffizienz und Erkrankungen der Arterienwand, dadurch gekennzeichnet, daß sie eine pharmazeutisch wirksame Menge mindestens einer Verbindung der Formel (I), wie sie in einem der Ansprüche 1 bis 10 definiert ist, oder eines ihrer pharmazeutisch akzeptablen Additionssalze, gegebenenfalls eingearbeitet in einen Exzipienten, ein Vehiculum oder einen pharmazeutisch akzeptablen Träger, enthält.

13. Pharmazeutische Zusammensetzung mit antiproliferativer Aktivität, die geeignet ist für die vorteilhafte Behandlung und Vorbeugung von Erkrankungen der Arterienwand, insbesondere der Arteriosklerose, dadurch gekennzeichnet, daß sie eine pharmazeutisch wirksame Menge mindestens einer Verbindung der Formel (I), wie sie in einem der Ansprüche 1 bis 10 definiert ist, oder eines ihrer pharmazeutisch akzeptablen Additionssalze, gegebenenfalls eingearbeitet in einen Exzipienten, ein Vehiculum oder einen pharmazeutisch akzeptablen Träger, enthält.

## Claims

1. A triazolopyrimidine derivative, characterised in that it is of general formula (I): 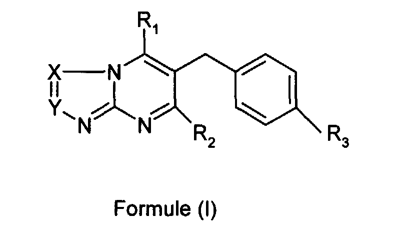 in which :
- one of the radicals R₁ and R₂ represents
- a lower alkyl radical having 1 to 6 carbon atoms ;
- an ether radical of the formula -(CH₂)ₚOR in which p is an integer from 1 to 6 and R represents a lower alkyl radical having 1 to 6 carbon atoms or a benzyl radical ; or
- an alcohol radical of the formula -(CH₂)ₚOH in which p is as defined above ; and
- the other radical R₁ or R₂ represents
- a hydrogen atom ;
- a halogen atom ;
- a lower alkyl radical having 1 to 6 carbon atoms ; or
- a radical selected from the group comprising the radicals N₃, OR₄, SR₄, NR₅R₆ and NH(CH₂)ₙ-NR₅R₆ ;
in which :
R₄ represents :
- a hydrogen atom ;
- a lower alkyl radical having 1 to 6 carbon atoms or a C₃-C₇ cycloalkyl radical;
- a (CH₂)ₘ-COOR' radical, m being an integer from 1 to 4, and R' being a hydrogen atom or a lower alkyl radical having 1 to 6 carbon atoms ; or
- a (CH₂)ₘ-O-R' radical, m and R' being as defined above ;
R₅ and R₆, which are identical or different, represent :
- a hydrogen atom ; or
- a lower alkyl radical having 1 to 6 carbon atoms or a C₃-C₇ cycloalkyl radical;
or
R₅ and R₆, taken together with the nitrogen atom to which they are attached, form a heterocycle selected from morpholine, pyrrolidine or piperidine ; and
- n is an integer from 1 to 4 ;
- X and Y, which are different, represent :
- in one case a nitrogen atom ; and
- in the other case a C-R₇ group, in which R₇ represents
- a hydrogen atom;
- a lower alkyl radical having 1 to 6 carbon atoms or a C₃-C₇ cycloalkyl radical;
- a (CH₂)_{n'}OH radical in which n' is an integer from 0 to 4 ;
- an SR' radical, R' being as defined above ; or
- an NR₅R₆ radical in which R₅ and R₆, which are identical or different, represent a hydrogen atom, a lower alkyl radical having 1 to 6 carbon atoms or a C₃-C₇ cycloalkyl radical ; and - R₃ represents a radical of the formula : 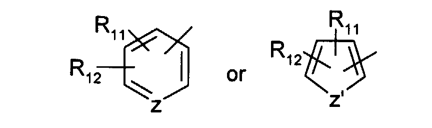 in which :
- Z represents CH or N or Z' represents S or O ;
- R₁₁ represents a hydrogen atom or a halogen atom ; and
- R₁₂ represents a tetrazole radical, CN, COOH or CONH₂ ;
and its tautomeric forms, and its pharmaceutically acceptable addition salts.

2. The derivative according to claim 1, characterised in that it is of general formula (I) given above in which :
- one of the radicals R₁ and R₂ represents :
- a lower alkyl radical having 1 to 6 carbon atoms ;
- an ether radical of the formula -(CH₂)ₚOR in which p is an integer from 1 to 6 and R represents a lower alkyl radical having 1 to 6 carbon atoms or a benzyl radical ; or
- an alcohol radical of the formula -(CH₂)ₚOH in which p is as defined above ; and
- the other radical R₁ or R₂ represents :
- a hydrogen atom ;
- a halogen atom;
- a lower alkyl radical having 1 to 6 carbon atoms ; or
- a radical selected from the group comprising the radicals N₃, OR₄, SR₄, NR₅R₆ and NH(CH₂)ₙ-NR₅R₆ ;
in which :
R₄ represents :
- a hydrogen atom ; or
- a (CH₂)ₘ-O-R' radical in which m is an integer from 1 to 4 and R' represents a lower alkyl radical having 1 to 6 carbon atoms ;
R₅ and R₆, which are identical or different, represent :
- a hydrogen atom; or
- a lower alkyl radical having 1 to 6 carbon atoms ;
or
R₅ and R₆, taken together with the nitrogen atom to which they are attached, form a heterocycle selected from morpholine, pyrrolidine or piperidine ; and
- n is an integer from 1 to 4 ;
- X and Y, which are different, represent :
- in one case a nitrogen atom ; and
- in the other case a C-R₇ group, in which R₇ represents
- a hydrogen atom;
- a lower alkyl radical having 1 to 6 carbon atoms ;
- a (CH₂)_{n'}OH radical in which n' is an integer from 0 to 4 ;
- an SR' radical, R' being as defined above ; or
- an NR₅R₆ radical in which R₅ and R₆, which are identical or different, represent a hydrogen atom, or a lower alkyl radical having 1 to 6 carbon atoms ; and
- R₃ represents one of the following radicals :

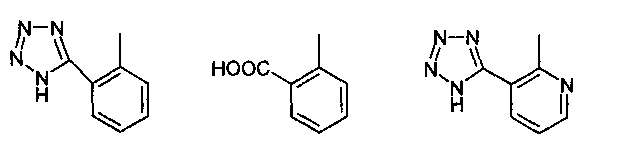

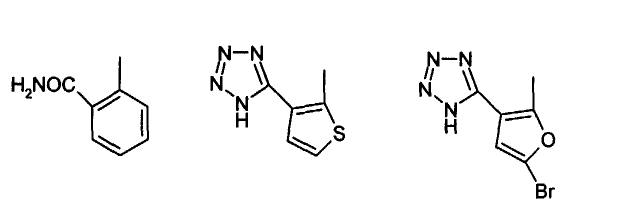

3. The derivative according to claim 1 or 2, characterised in that R₁ is an n-propyl, n-butyl or N-diethylamino group.

4. The derivative according to any one of claims 1 to 3, characterised in that R₂ is a hydroxyl, n-propyl or N-diethylamino group.

5. The derivative according to any one of claims 1 to 4, characterised in that R₃ is a 2-(1H-tetrazol-5-yl)phenyl group.

6. The derivative according to any one of claims 1 to 5, characterised in that X is a nitrogen atom.

7. The derivative according to any one of claims 1 to 6, characterised in that Y represents a CH, C-CH₃ or C-NH₂ group.

8. The derivative according to claim 1 or 2, characterised in that R₁ is a lower alkyl radical having 1 to 6 carbon atoms, R₂ is a hydroxyl group, R₃ is a 2-(1H-tetrazol-5-yl)phenyl group, X represents a nitrogen atom and Y represents a CH or C-CH₃ group.

9. The derivative according to claim 1 or 2, characterised in that it is the derivative :
5 -hydroxy-7-propyl-6-[(2'-(1H-tetrazol-5-yl)biphenyl-4-yl)methyl]-1,2,4-triazolo[1,5-a]pyrimidine :

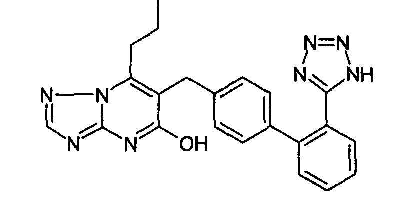

10. The derivative according to claim 1 or 2, characterised in that it is selected from the derivatives of the formulae :

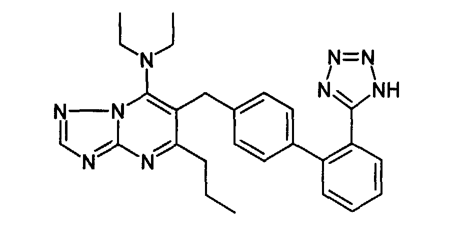

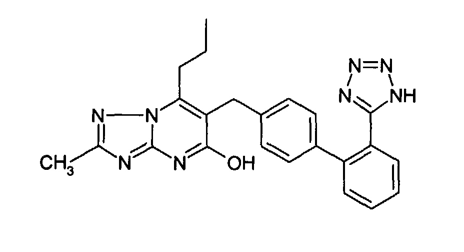

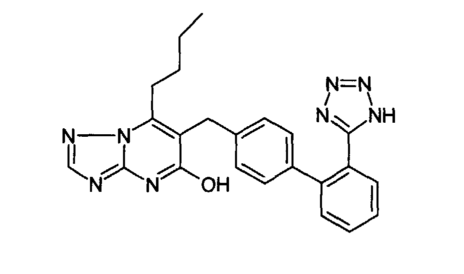

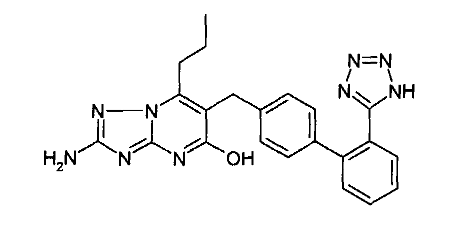

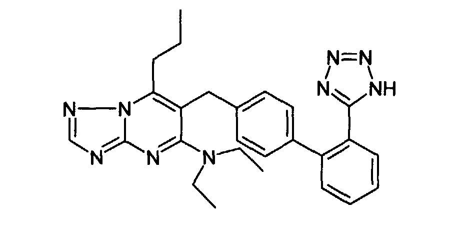

11. A method of preparing the compounds of formula (I) according to any one of claims 1 to 10, characterised in that it comprises :
a) preparing a compound of formula (α): 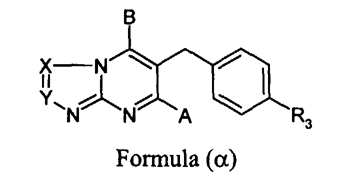 in which :
- X, Y and R₃ are as defined in claim 1; and
- A and B represent in one case a hydroxyl group or a lower alkyl radical having 1 to 6 carbon atoms, and in the other case a lower alkyl radical having 1 to 6 carbon atoms or an ether radical of the formula -(CH₂)ₚOR in which p is an integer from 1 to 6 and R represents a lower alkyl radical having 1 to 6 carbon atoms or a benzyl radical,
by condensing a 3-amino-1,2,4-triazole of formula (II): 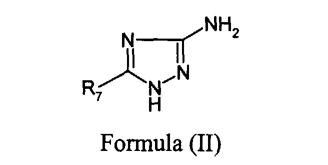 in which R₇ is as defined in claim 1, with a derivative of formula (β) : 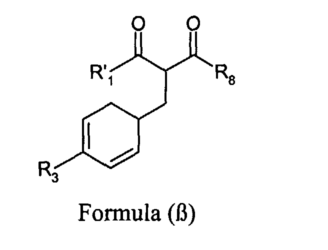 in which R'₁ is a lower alkyl radical having 1 to 6 carbon atoms or an ether radical of the formula -(CH₂)ₚOR, in which p is an integer from 1 to 6 and R represents a lower alkyl radical having 1 to 6 carbon atoms or a benzyl radical, R₈ represents a lower alkyl radical having 1 to 6 carbon atoms or a lower O-alkyl group having 1 to 6 carbon atoms, preferably methyl or ethyl, and R₃ is as defined in claim 1, in an aprotic solvent or in an acid solvent or even in an alcohol in the presence of the corresponding sodium or potassium alkylate, or even in pyridine or 2-methyl-5-ethylpyridine in the presence or absence of 4-dimethylaminopyridine, at a temperature between 50 and 200°C;
b) optionally, protecting the function carried by R₃ according to a method known *per se ;*
c) heating the derivative thus obtained from the derivative of formula (β), when the latter is a ketoester, in an appropriate reagent for converting the hydroxyl group represented by A or B into a chlorine atom ;
d) heating this chlorinated derivative in the presence of a nucleophile containing nitrogen, oxygen or sulphur, under reflux in an alcohol or in an autoclave at 100°C, in the presence or absence of a base, to give a derivative of formula (α) in which A and B have the same meanings as R₁ and R₂ respectively ;
e) optionally, deprotecting the function carried by R₃ ;
e₁) converting this function into an acid group ; or
e₂) converting this function into a tetrazole group ; or
e₃) converting this group to an amide group ; and
f) optionally, converting the resulting derivative into an addition salt.

12. A pharmaceutical composition with antagonistic activity towards angiotensin II receptors which permits a favourable treatment and prevention of cardiovascular diseases, especially hypertension, cardiac insufficiency and diseases of the arterial wall, said composition being characterised in that it contains a pharmaceutically effective amount of at least one compound of formula (I) as defined in any one of claims 1 to 10, or one of its pharmaceutically acceptable addition salts, which may or may not be incorporated in a pharmaceutically acceptable excipient, vehicle or carrier.

13. A pharmaceutical composition with antiproliferative activity which permits a favourable treatment and prevention of diseases of the arterial wall, especially atherosclerosis, said composition being characterised in that it contains a pharmaceutically effective amount of at least one compound of formula (I) as defined in any one of claims 1 to 10, or one of its pharmaceutically acceptable addition salts, which may or may not be incorporated in a pharmaceutically acceptable excipient, vehicle or carrier.
